# EUROPEAN PATENT APPLICATION

(11) **EP 2 636 755 A1**
(43) Date of publication of application: **11.09.2013**
(21) Application number: 13162222.7
(22) Date of filing: 29.05.2007
(51) Int. Cl.: C12Q 1/68

(54) **Biochemical analysis of partitioned cells**

(30) Priority: 26.05.2006 US 808762 P
(62) Divisional of application: 07809240.0
(71) Applicant: AltheaDx Incorporated, San Diego, CA 92121 (US)
(72) Inventor: Monforte, Joseph, Kensington, CA 94707 (US)
(74) Representative: Woolley, Lindsey Claire

(57) **Abstract**

The invention relates to compositions and methods for the analysis of biomolecules associated with cells, where the presence or absence of a particular biomolecule (e.g. an expressed protein or a nucleic acid gene expression product) associated with the cells is examined. The invention provides methods for single cell biochemical analysis, as well as instrumentation for the single-cell biochemical analysis. Most advantageously, the invention affords methods and instrumentation for high-throughput biochemical analysis of large numbers of single cells.

## Description

### CROSS-REFERENCE TO RELATED APPLICATIONS

This application claims priority to and benefit of United States Provisional Application Serial No. 60/808,762, filed on May 26, 2006, the specification of which is hereby incorporated by reference in its entirety for all purposes.

### FIELD OF THE INVENTION

The invention pertains to the fields of biochemistry and cell biology. The compositions and methods of the invention relate to the analysis of cells, where the presence or absence of a particular biomolecule (*e.g.*, an expressed protein or an expressed nucleic acid) associated with the cells is examined. The invention provides methods for high-throughput single cell biochemical analysis, as well as instrumentation for the biochemical analysis.

### BACKGROUND OF THE INVENTION

Understanding individual cell behavior in biological processes such as differentiation, development, and disease requires knowledge of gene and protein expression information for individual cells. Analysis of gene and protein expression at the population level (*i.e.,* in collections of cells or in tissues) are insufficient, because these analyses can only determine an average gene/protein expression level within the population of cells as a whole. This situation overlooks cell-to-cell heterogeneity that could lead to different cell behaviors or cell fates. It is likely that there exists considerable variation in gene-expression levels between individual cells in a population, implying that the arithmetic mean of expression analysis may not always adequately describe a typical cell, and furthermore, may not accurately reflect the gene expression profiles of the few cells in the population that follow developmental programs that yield rare cell types or cause disease (e.g., a tumor cell).

Continued efforts to improve the sensitivity of the polymerase chain reaction (PCR) now routinely allows PCR amplification of target sequences from isolated single cells and rare sequences (e.g., low copy number sequences). The generation of gene expression profiles or genomic DNA analysis using PCR on isolated single cells has impacted diverse fields in biological research and medicine. For example, this is especially true in the field of prenatal diagnostics, and has permitted preimplantation genetic analysis and the use of fetal cells enriched from the blood or placenta of pregnant women for the assessment of single-gene Mendelian disorders. Other applications that benefit from single-cell PCR include addressing diverse immunological, neurological and developmental questions, where messenger RNA expression patterns and genomic sequences are examined.

Various methods are known in the art for dispensing single cells. Cell sorters using traditional flow cytometry technology allow the selection and sorting of cells (including single cells) according to a range of criteria. Using cell sorting technology, single cells can be identified and sorted for microscopy, for culture and for genetic analysis by way of single cell PCR. The use of cell sorters to isolate individual cells (e.g., for use in PCR) requires reliable coupling between the detection event and the ability to deflect the one cell in the corresponding saline droplet to a unique addressable location (e.g., an eppendorf tube or a well in a microwell plate).

Traditional cell sorting, as well as other cell dispensing technologies, are constrained by various technical limitations in achieving high-throughput single cell biochemical analyses. First, the single cell sorting throughput capacity of devices such as traditional flow cytometry systems is limited. For example, flow cytometry cell sorting, at best, can only segregate individual cells using 96-well, 384-well or 1536-well multiwell plate formats. Second, traditional cell sorting (using, for example, a labeled monoclonal antibody) is limited to systems that do not permit target molecule amplification (e.g., PCR amplification of a target expressed gene) or signal amplification using non-homogenous and homogenous detection assays.

The present invention provides solutions to these and other problems. The invention provides compositions and methods for monitoring (e.g., detecting, quantitating, assaying) a plurality of biomolecules (e.g., proteins or nucleic acids) within or otherwise associated with individual cells or small groups of cells. These assays can use either homogenous or non-homogenous detection/signal systems. In particularly advantageous embodiments, these compositions and methods for monitoring biomolecules are used in extremely high-throughput methodologies for the rapid assessment of large numbers of single cells, for example, many thousands or millions of cells.

### SUMMARY OF THE INVENTION

The present invention provides compositions and methods for monitoring (e.g., detecting, quantitating, assaying) a plurality of biomolecules (e.g., proteins or nucleic acids) within or otherwise associated with individual cells or small groups of cells. In particularly advantageous embodiments, these compositions and methods for monitoring biomolecules associated with cells are used in high-throughput, highly parallel methodologies for the rapid assessment of large numbers of single cells. In various aspects, the invention provides methods for the highly parallel, high-throughput cell biochemical analysis. In other aspects, the invention provides compositions that are formed as a result of methods of the invention. In still other aspects, the invention provides devices that produce compositions of the invention, and further, facilitate the methods of the invention.

In some aspects, the invention provides devices for practicing the invention. For example, in some device of the invention, the devices are for generating single cell aqueous reaction volumes in a flow channel. Those devices comprise:
a) a flow channel (typically in a liquid flow system);
b) a first source of an aqueous solution that comprises a plurality of cells, where the source is fluidly coupled to the flow channel;
c) a second source of a partitioning solution that is immiscible in the aqueous solution, where the second source is also fluidly coupled to the flow channel;
d) a controller operably coupled to the first and second sources, where the controller directs the flow of the aqueous solution from the first and second sources into the flow channel, where the partitioning solution acts as barriers and partitions cells in the aqueous solution into separate aqueous reaction volumes;
e) an excitation light source capable of illuminating the aqueous reaction volumes in the flow channel at a desired wavelength;
f) a spectrophotometric detector capable of detecting a light emission signal from a single aqueous reaction volume in the flow channel; and,
g) an electronic module operably connected to the detector for collecting and storing said signals detected by the detector from a plurality of aqueous reaction volumes.

These devices can further optionally contain a thermal control element operably coupled to the flow channel for regulating the temperature of the aqueous reaction volumes in said flow channel, and further can optionally can contain an optical sensor for detecting cells in the aqueous solution, the sensor operably coupled to the controller. The electronic module associated with the device can optionally comprise one or more algorithm (e.g., a program) for displaying the plurality of signals collected from the detector (i.e., the detection results).

In other aspects, the invention provides methods for single cell biochemical analysis, where the analysis is contained in a reaction vesicle. These methods are for detecting the presence or absence of a biomolecule associated with a plurality of cells. These methods contains the steps of:
a) providing:
   i) a plurality of cells in an aqueous first solution that further contains at least one reagent for detecting the presence or absence of the biomolecule, where the reagent is capable of generating or participating in generating an amplified signal corresponding to the presence or absence of the biomolecule;
   ii) a second solution that is immiscible with said first solution and capable of forming vesicles when admixed with the first solution;
b) combining the first ad second solutions under conditions that result in the formation of a plurality of reaction vesicles, where each reaction vesicle formed contains, (i) one cell from the plurality of cells, and (ii) a reagent for detecting the presence or absence of the biomolecule;
c) subjecting the reaction vesicles to conditions suitable for detecting the biomolecule (e.g., thermal cycling in PCR), wherein the reagent generates or participates in generating an amplified signal corresponding to the presence or absence of the biomolecule associated with the one cell in each reaction vesicle;
d) detecting the amplified signal from the plurality of vesicles;
e) correlating the signal with the presence or absence of the biomolecule associated with said cells, thereby detecting the presence or absence of the biomolecule associated with said cells and generating a detection result; and
f) displaying the detection result for said plurality of cells.

Used advantageously, these methods are high throughput and highly parallel. In various high-throughput embodiments, at least 100 cells are analyzed, at least 1,000 cells are analyzed, at least 10,000 cells are analyzed, at least 100,000 cells are analyzed, or at least 1,000,000 cells are analyzed.

In various embodiments of these methods, the second solution is a non-aqueous solution, e.g., silicone oil. The amplified signal that is generated can be a colorimetric signal, a fluorescence signal, a phosphorescence signal, an isotopic signal, a radioisotopic signal, or an enzymatic reaction product. The detecting step can optionally include quantitating the amplified signal.

In some embodiments of these methods, the biomolecule detected can be a genomic nucleic acid of interest, where the detecting step includes amplifying at least one genomic nucleic acid to produce a genomic amplification product, and the detected signal corresponds to the genomic amplification product. Alternatively, the biomolecule can be a nucleic acid gene expression product of interest, where the detecting step includes amplifying the gene expression product to produce an amplification product, and where the detected signal corresponds to the amplification product. Either of these methods ca optionally use real-time PCR detection. Where RNA is detected, amplifying can use RT-PCR, e.g., incorporating a nucleic acid polymerase reagent comprising a reverse transcriptase activity. Where more than one nucleic acid is targeted, the PCR can be multiplex PCR.

The reagents used are not particularly limited, can utilize any of the following: at least one primer-pair specific for the nucleic acid of interest, a thermostable DNA-dependent DNA polymerase, free nucleotide triphosphates, and wherein said subjecting said vesicle to conditions suitable for detecting the biomolecule comprises subjecting the vesicle to thermal cycling. The PCR amplification can optimally incorporate universal priming sequence at the 5' end of a primer, and the reagents will further comprise at least one universal primer complimentary to the universal priming sequence.

In some embodiments, the reagents include a solid phase component, e.g., beads, where the solid phase comprises a nucleic acid molecule at least partially complementary to the amplification product.

In some aspects of these methods, a polypeptide (e.g., a cell-surface polypeptide) is the biomolecule of interest. In this case, the reagent in the detection reaction can be a probe moiety that is capable of specific binding to the polypeptide, and where the vesicles are exposed to conditions suitable for allowing specific interaction of the polypeptide with the moiety. In some aspects, the moiety is a second polypeptide, e.g., an antibody, a fragment of an antibody, or derived from an antibody. In some embodiments, the polypeptide is a cell surface receptor, and the probe moiety is a ligand specific for the receptor. The detection moiety can be attached to a solid phase component, e.g., a bead.

In still other aspects, the invention provides methods for single cell biochemical analysis, where the analysis is contained in a partitioned aqueous volume in a flow channel. These methods are for detecting the presence or absence of a biomolecule associated with a plurality of cells. These methods contains the steps of:
a) providing:
   i) a plurality of cells in an aqueous first solution, the first solution further comprising at least one reagent for detecting the presence or absence of the biomolecule, where the reagent is capable of generating or participating in generating an amplified signal corresponding to the presence or absence of the biomolecule;
   ii) a second solution that is immiscible with the first solution;
b) channeling the aqueous solution through a liquid flow system, thereby generating a channeled liquid flow, where the liquid flow system further contains a means for delivering the second solution into the channeled liquid flow at intervals, thereby generating a plurality of partitioned aqueous reaction volumes in the channeled liquid flow, where the partitioned aqueous reaction volumes are separated from each other by partitions comprising the injected second solution, wherein each partitioned aqueous reaction volume thus formed contains (i) at least one cell from the plurality of cells, and (ii) a reagent for detecting the presence or absence of the biomolecule;
c) subjecting the partitioned aqueous reaction volumes to conditions suitable for detecting the biomolecule, where the reagent generates or participates in generating an amplified signal corresponding to the presence or absence of the biomolecule associated with the one cell in each partitioned aqueous reaction volume, thereby generating a signal corresponding to the presence or absence of the biomolecule in the partitioned aqueous reaction volume;
d) detecting said amplified signal form the plurality of partitioned aqueous reaction volumes;
e) correlating the signal with the presence or absence of the biomolecule associated with the cells, thereby detecting the presence or absence of the biomolecule associated with said cells and generating a detection result; and
f) displaying the detection result for the plurality of cells.

Used advantageously, these methods are high throughput and highly parallel. In various high-throughput embodiments, at least 100 cells are analyzed, at least 1,000 cells are analyzed, at least 10,000 cells are analyzed, at least 100,000 cells are analyzed, or at least 1,000,000 cells are analyzed.

In various embodiments of these methods, the second solution is a non-aqueous solution, e.g., silicone oil. The amplified signal that is generated can be a colorimetric signal, a fluorescence signal, a phosphorescence signal, an isotopic signal, a radioisotopic signal, or an enzymatic reaction product. The detecting step can optionally include quantitating the amplified signal.

In some embodiments of these methods, the biomolecule detected can be a genomic nucleic acid of interest, where the detecting step includes amplifying at least one genomic nucleic acid to produce a genomic amplification product, and the detected signal corresponds to the genomic amplification product. Alternatively, the biomolecule can be a nucleic acid gene expression product of interest, where the detecting step includes amplifying the gene expression product to produce an amplification product, and where the detected signal corresponds to the amplification product. Either of these methods ca optionally use real-time PCR detection. Where RNA is detected, amplifying can use RT-PCR, e.g., incorporating a nucleic acid polymerase reagent comprising a reverse transcriptase activity. Where more than one nucleic acid is targeted, the PCR can be multiplex PCR.

The reagents used are not particularly limited, can utilize any of the following: at least one primer-pair specific for the nucleic acid of interest, a thermostable DNA-dependent DNA polymerase, free nucleotide triphosphates, and wherein said subjecting said vesicle to conditions suitable for detecting the biomolecule comprises subjecting the vesicle to thermal cycling. The PCR amplification can optimally incorporate universal priming sequence at the 5' end of a primer, and the reagents will further comprise at least one universal primer complimentary to the universal priming sequence.

In some embodiments, the reagents include a solid phase component, e.g., beads, where the solid phase comprises a nucleic acid molecule at least partially complementary to the amplification product.

In some aspects of these methods, a polypeptide (e.g., a cell-surface polypeptide) is the biomolecule of interest. In this case, the reagent in the detection reaction can be a probe moiety that is capable of specific binding to the polypeptide, and where the vesicles are exposed to conditions suitable for allowing specific interaction of the polypeptide with the moiety. In some aspects, the moiety is a second polypeptide, e.g., an antibody, a fragment of an antibody, or derived from an antibody. In some embodiments, the polypeptide is a cell surface receptor, and the probe moiety is a ligand specific for the receptor. The detection moiety can be attached to a solid phase component, e.g., a bead.

The invention also includes compositions. For example, in one aspect, a composition of the invention comprises (a) a plurality of aqueous reaction core solutions, each core solution containing (i) a cell, and (ii) at least one reagent for detecting the presence or absence of a biomolecule associated with the cell, where the reagent is capable of generating or participating in generating an amplified signal corresponding to the presence or absence of the biomolecule; and (b) an immiscible liquid shell that partitions the aqueous reaction core solution.

In some aspects, the shell surrounds the reaction core solutions, thereby forming a plurality of vesicles. In other aspects, the shell partitions one aqueous core solution from an adjacent aqueous core solution, thereby partitioning two or more aqueous cores within the partitioned fluid. The partitioning solution can optionally be a silicon oil.

In some embodiments, the biomolecule of interest is a genomic nucleic acid or a gene expression product, and where the detection reagents can be chosen from at least one primer-pair specific for the genomic nucleic acid or the gene expression product, a thermostable DNA-dependent DNA polymerase, and free nucleotide triphosphates, or a nucleic acid polymerase comprising a reverse transcriptase activity. Each member of the primer-pair can further comprise a universal priming sequence at the 5' end of the primer, and the reagents further comprise at least one universal primer complimentary to the universal priming sequence. A reagent can be a solid phase component, e.g., particles, beads, strands, precipitates, gels, sol-gels, sheets, tubing, spheres, containers, channels, capillaries, pads, slices, films, plates, dipsticks or slides.

In some aspects of these compositions, a polypeptide (e.g., a cell-surface polypeptide) is the biomolecule of interest. In this case, the reagent in the detection reaction can be a probe moiety that is capable of specific binding to the polypeptide, and where the vesicles are exposed to conditions suitable for allowing specific interaction of the polypeptide with the moiety. In some aspects, the moiety is a second polypeptide, e.g., an antibody, a fragment of an antibody, or derived from an antibody. In some embodiments, the polypeptide is a cell surface receptor, and the probe moiety is a ligand specific for the receptor. The detection moiety can be attached to a solid phase component, e.g., a bead.

### DEFINITIONS

Before describing the invention in detail, it is to be understood that this invention is not limited to any particular biological system (e.g., any particular type of cell or cells). It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a vesicle" includes a plurality of vesicles; reference to "a polynucleotide" (e.g., a primer) includes, as a practical matter, many molecules of that polynucleotide.

Unless defined herein and below in the remainder of the specification, all technical and scientific terms used herein have the same meaning as commonly understood by one of ordinary skill in the art to which the invention pertains. Although many methods and materials similar or equivalent to those described herein can be used in the practice for testing of the present invention, the preferred materials and methods are described herein. In describing and claiming the present invention, the following terminology will be used in accordance with the definitions set out below.

Biomolecule: As used herein, the term "biomolecule," "biochemical," "biochemical component," or any other similar or equivalent expression refers to any molecule that is produced by or associated with a cell. A biomolecule that is "associated with" a cell refers to a biomolecule that is produced by the cell or comes in contact with the cell, and is not limited to any particular subcellular compartment (e.g., the cell membrane, the cell nucleus, the cell mitochondria, etc.). The biomolecule can be of any structure or type, including but not limited to, nucleic acids, proteins (including peptides and polypeptides) carbohydrates, lipids and any other organic biomolecules (e.g., free amino acids, vitamins and hormones such as steroids). Methodologies and reagents for detecting and/or quantitating biomolecules such as those listed above are common in the art and are widely available.

Aqueous: As used herein, the term "aqueous," as in an "aqueous solution," refers to a solution in which the primary solvent is water. The term implies that the feature being referred to is situated in, dissolved in, taking place in, or otherwise associated with liquid water. Substances that do not dissolve in, or do not dissolve well in water are generally termed hydrophobic, whereas substances that readily enter into an aqueous phase are generally hydrophilic. Aqueous solutions can contain components in addition to water, including any compound that can dissolve in water, for example, salts. As used herein, the term "aqueous solution" includes solutions that are substantially aqueous that contain some fraction of a non-aqueous component.

Non-aqueous: As used herein, the term "non-aqueous," as in a "non-aqueous solution," refers to a solution in which the solvent is not water. Non-aqueous solutions are typically immiscible with aqueous solutions. In one aspect in describing non-aqueous solutions, the solvent can be organic or inorganic. An organic non-aqueous solvent is a solvent other than water, where the solvent is an organic compound. An inorganic non-aqueous solvent is a solvent other than water, where the solvent is not an organic compound. As used herein, the term "non-aqueous solution" includes solutions that are substantially non-aqueous that contain some small fraction of an aqueous component. In some embodiments, the non-aqueous solution can include gases, including air, nitrogen or inert gases.

Aqueous phase: As used herein, the term "aqueous phase" refers to that portion of a mixture of liquids that is aqueous. The term "aqueous phase" implies that there also exists in the system under study a second phase that is not aqueous.

Miscible: As used herein, the term "miscible" refers to the property of two or more substances, most typically liquids, that allows them to be mixed together and form a single homogeneous phase. For example, water and ethanol are miscible in all proportions.

Immiscible: As used herein, the term "immiscible" refers to the property of two or more substances, most typically liquids, that prevents them mixing together and forming a single homogeneous phase. For example, water and silicone oil are immiscible. In the case of organic compounds, the length of the carbon chain often determines miscibility with water. For example, in the alcohols, ethanol has two carbon atoms and is miscible with water, whereas octanol has eight carbon atoms and is not miscible with water. Similarly, lipids (which are typically characterized as having very long carbon chains) are almost always immiscible with water.

Emulsion: As used herein, the term "emulsion" refers to a mixture of two immiscible (unblendable) substances. As used herein, the term refers to liquid substances. Typically, one liquid of the two (termed "the dispersed phase") is dispersed in the second liquid (the "continuous phase"). Emulsification is the process by which emulsions are prepared. Emulsions typically have a cloudy appearance due to the many phase interfaces (the boundary between the phases is called the interface) that scatter light that passes through the emulsion. Emulsions are unstable and thus do not form spontaneously. Energy input through shaking, stirring, homogenizers, or spray processes are needed to form an emulsion. Over time, emulsions tend to revert to a stable separated state (e.g., oil separated from water). Surface active substances (surfactants) can increase the kinetic stability of emulsions greatly so that once formed, the dispersion of the dispersed phase in the emulsion does not change significantly over time. The term "emulsions" refers to part of a more broader class of two-phase systems of matter called colloids. Although the terms "colloid" and "emulsion" are sometimes used interchangeably, "emulsion" typically implies that both the dispersed and the continuous phases are liquid.

As used herein in some embodiments, an emulsion is generated in order to produce the reaction vesicles of the invention. In this case, the non-aqueous liquid (that is immiscible in the aqueous phase) is the dispersed phase, and the aqueous phase is the continuous phase.

Vesicle: As used herein, the term "vesicle" refers to an enclosed aqueous compartment that is separated from its surrounding local environment by a layer of a water-immiscible substance. In biological systems, a vesicle is a small, subcellular enclosed compartment separated from the cytosol by at least one lipid bilayer. However, as used herein, vesicles refer to a larger class of structures that includes artificial vesicles, and further where the artificial vesicles are optionally formed from non-biological, non-aqueous substances (that are immiscible in aqueous environments). For example, the vesicles of the invention can be formed from the combination of an aqueous solution and a silicone oil. The artificial vesicles of the invention can be produced in sizes large enough to capture cells within the interior aqueous volume of the vesicle.

Reaction vesicle: As used herein, the term "reaction vesicle" refers to a vesicle that comprises at least one reagent for detecting the presence or absence of at least one biomolecule associated with a cell.

Liposome: As used herein, the term "liposome" refers to an artificial spherical vesicle typically having an aqueous core and a confining membrane composed of a phospholipid and cholesterol bilayer. Liposomes can be composed of naturally-derived phospholipids with mixed lipid chains (like egg phosphatidylethanolamine), or of pure surfactant components like DOPE (dioleolylphosphatidylethanolamine). In some embodiments, the liposome vesicles find use with the invention. Lipid spheres that do not contain a partitioned aqueous space when dispersed in an aqueous environment are called micelles; these micelles do not find use with the invention.

Aqueous reaction core solution: As used herein, the term "aqueous reaction core solution" refers to an aqueous volume comprising at least one cell and at least one reagent for detecting the presence or absence of at least one biomolecule associated with the cell, where the aqueous reaction core solution is isolated from the local environment or any adjacent aqueous reaction core solution by an immiscible liquid shell.

Immiscible liquid shell: As used herein, the term "immiscible liquid shell" refers to a liquid that is immiscible in water that confines an aqueous reaction core solution. The immiscible liquid shell acts as a barrier to isolate any one aqueous reaction core solution from an adjacent aqueous reaction core solution or from the local environment. In some embodiments, the immiscible liquid shell completely surrounds the aqueous reaction core solution, thereby forming a reaction vesicle. In other embodiments, the immiscible liquid shell prevents mixing of one aqueous reaction core solution with an adjacent aqueous reaction core solution within a liquid flow channel, thus the shell as a partition between adjacent aqueous reaction core solutions in the flow channel. In some embodiments, the immiscible liquid shell is or comprises silicone oil.

Reagent: As used herein, the term "reagent" refers to any component (of sufficient purity) not endogenous to a cell that is involved in the assay of a biomolecule associated with a cell. It is not intended that the term "reagent" refer to any particular class of component involved in any one type of biochemical assay. For example, a reagent can refer to any component involved in assays for nucleic acids, proteins (including peptides and polypeptides) carbohydrates, lipids or any other organic biomolecules (e.g., free amino acids, vitamins and hormones such as steroids). For example, reagents can include, but are not limited to: nucleic acid primers, primer-pairs, nucleic acid polymerases of any type (e.g., including polymerases comprising a reverse transcriptase activity and a thermostable DNA-dependent DNA polymerases), free nucleotide triphosphates, molecular beacons, FRET pair reagents for nucleic acid detection, antibodies, fragments of antibodies, molecules derived from antibodies, including antibody-enzyme fusion proteins, secondary antibodies to detect a primary antibody, substrates for enzymes that generate colorimetric signals for detection, labels, and any manner of probes. In some aspects, reagents can also include solid phase components that are involved in the biochemical assay, for example a bead that has a conjugated antibody, or a bead that has a conjugated polynucleotide.

Liquid flow system: As used herein, the expression "liquid flow system" or "liquid flow device" or similar expressions refer to any system for the handling of liquids. A wide variety of such systems are well known in the art for the transport of one or more fluids between one or more reservoirs within or external to a system or device, and can be further specialized for the execution of a desired assay, chemical or biological reaction, measurement, monitoring, amplification, or signal detection, all within the device. Incorporation of a variety of pumps and valves within liquid flow devices to move the fluids within tubes, channels, capillaries, reservoirs, chambers, compartments, wells, or the like are thoroughly described in the art. Liquid flow systems have also been created for the handling of exceptionally small volumes of analyte and reagent liquids. Such systems can operate using microliter and nanoliter scale fluid volumes, and employ structures having dimensions as small as nanometer scale (typically 1-100 nm); such platforms are commonly termed "microfluidic" or "nanofluidic" devices.

Operably coupled: As used herein, the expressions "operably coupled," "operably connected" or any other equivalent expression refers to a arrangement of two or more parts such that the parts are functional together. For example, a computer that is operably coupled to a detector is able to receive and store signals from the detector. A controller that is operably coupled to a liquid source is able to regulate (e.g., control) the flow of that liquid into or through a flow channel in a liquid flow system.

Flow channel: As used herein, the expression "flow channel" or any equivalent expression refers to any type of structure that carries or transports liquid in a liquid flow system.

Fluidly coupled: As used herein, the expression "fluidly coupled" or any equivalent expression refers to an arrangement of two or more features such that the features are connected in such a way as to permit the flow of fluid (e.g., a fluid path) between the features and permits fluid transfer. For example, where a source or reservoir of a solution is fluidly coupled to a flow channel, that solution is capable of flowing from said source to said flow channel. The fluid coupling can be unregulated, or more typically, the fluid coupling can be regulated by the time of flow, the rate of flow, the volume of flow, or any other parameter.

Homogenous detection assay: As used herein, an homogenous detection assay is an assay for a biomolecule, wherein the detectable signal is a freely diffusible molecule, for example, an enzymatic assay that uses a chromogenic substrate. Examples of homogeneous assays include but are not limited to real-time PCR amplification assays wherein a fluorescent chromophore is converted from a quenched state to a fluorescent state as a consequence of a specific nucleic acid amplification event or an enzyme and enzyme substrate systems wherein the enzyme substrate converts to an optically detectable product as a function enzyme catalysis. Homogeneous assays, in general, do not require the addition of additional reagents once the detection system is set up. In the case of a reaction vesicle or aqueous reaction core, a homogenous assay does not require the disruption of the reaction vesicle or core as a part of the biochemical detection event.

Nucleic acid: As used herein, the terms "polynucleotide," "nucleic acid," "oligonucleotide," "oligomer," "oligo" or equivalent terms, refer to a polymeric arrangement of monomers that can be corresponded to a sequence of nucleotide bases, e.g., a DNA, RNA, peptide nucleic acid, or the like. A polynucleotide can be single- or double-stranded, and can be complementary to the sense or antisense strand of a gene sequence, for example. A polynucleotide can hybridize with a complementary portion of a target polynucleotide to form a duplex, which can be a homoduplex or a heteroduplex. The length of a polynucleotide is not limited in any respect. Linkages between nucleotides can be internucleotide-type phosphodiester linkages, or any other type of linkage. A "polynucleotide sequence" refers to the sequence of nucleotide monomers along the polymer. A "polynucleotide" is not limited to any particular length or range of nucleotide sequence, as the term "polynucleotide" encompasses polymeric forms of nucleotides of any length. A polynucleotide can be produced by biological means (e.g., enzymatically), or synthesized using an enzyme-free system. A polynucleotide can be enzymatically extendable or enzymatically non-extendable. A polynucleotide can be enzymatically cleaved, as by a nuclease, or enzymatically uncleavable. As used herein, it is not intended that the term "polynucleotides" be limited to naturally occurring polynucleotides sequences or polynucleotide structures, naturally occurring backbones or naturally occurring internucleotide linkages. One familiar with the art knows well the wide variety of polynucleotide analogues, unnatural nucleotides, non-natural phosphodiester bond linkages and internucleotide analogs that find use with the invention. Non-limiting examples of such unnatural structures include non-ribose sugar backbones, 3'-5' and 2'-5' phosphodiester linkages, internucleotide inverted linkages (e.g., 3'-3' and 5'-5'), and branched structures. Furthermore, unnatural structures also include unnatural internucleotide analogs, *e.g*., peptide nucleic acids (PNAs), locked nucleic acids (LNAs), C₁-C₄ alkylphosphonate linkages such as methylphosphonate, phosphoramidate, C₁-C₆ alkyl-phosphotriester, phosphorothioate and phosphorodithioate internucleotide linkages. Furthermore, a polynucleotide can be composed entirely of a single type of monomeric subunit and one type of linkage, or can be composed of mixtures or combinations of different types of subunits and different types of linkages (a polynucleotide can be a chimeric molecule). As used herein, a polynucleotide analog retains the essential nature of natural polynucleotides in that they hybridize to a single-stranded nucleic acid target in a manner similar to naturally occurring polynucleotides.

Amplification: As used herein, the terms "amplification," "amplifying" and the like can, in some aspects, refer generally to any process that results in an increase in the copy number of a molecule. As it applies to polynucleotide molecules, amplification means the production of multiple copies of a polynucleotide molecule, or a portion of a polynucleotide molecule, typically starting from a small amount of a polynucleotide (e.g., an mRNA species), where the amplification product (e.g., a PCR amplicon) is detectable. Amplification of polynucleotides can encompasses a variety of chemical and enzymatic processes. The generation of multiple DNA copies from one or a few copies of a template nucleic acid molecule during a polymerase chain reaction (PCR), a strand displacement amplification (SDA) reaction, a transcription mediated amplification (TMA) reaction, a nucleic acid sequence-based amplification (NASBA) reaction, or a ligase chain reaction (LCR) are forms of amplification. Amplification is not limited to the strict duplication of the starting molecule. For example, the generation of multiple cDNA molecules from a limited amount of cellular RNA using reverse transcription-polymerase chain reaction (RT-PCR) is a form of amplification. Furthermore, the generation of multiple RNA molecules from a single DNA molecule during the process of transcription is also a form of amplification. In other aspects, the term "amplification" can refer to a process whereby a signal strength corresponding to a biomolecule is made stronger, without raising the copy number of the biomolecule.

Polypeptide: As used herein, the term "polypeptide" refers to any oligomer of amino acids (natural or unnatural, or a combination thereof), of any length, typically but not exclusively joined by covalent peptide bonds. A polypeptide can be from any source, e.g., a naturally occurring polypeptide, a polypeptide produced by recombinant molecular genetic techniques, a polypeptide from a cell or translation system, or a polypeptide produced by cell-free synthetic means. A polypeptide is characterized by its amino acid sequence, e.g., the primary structure of its component amino acids. As used herein, the amino acid sequence of a polypeptide is not limited to full-length sequences, but can be partial or complete sequences. Furthermore, it is not intended that a polypeptide be limited by possessing or not possessing any particular biological activity. As used herein, the term "protein" is synonymous with polypeptide. The term "peptide" generally refers to a small polypeptide, for example but not limited to, from 2-40 amino acids in length.

Antibody: As used herein, the term "antibody" (or "antibodies") refers to any immunoglobulin that binds specifically to an antigenic determinant, and specifically, binds to proteins identical or structurally related to the antigenic determinant which stimulated their production. Thus, antibodies are useful in methods to detect the antigen which stimulated their production. Monoclonal antibodies are derived from a single B lymphocyte clone and are generally homogeneous in structure and have specificity for a single antigenic epitope. Polyclonal antibodies (as in polyclonal sera) originate from many different clones of antibody-producing cells, and thus are heterogeneous in their structure and epitope specificity, but are generally enriched in antibodies which bind to the same antigen. In some embodiments, purified monoclonal and/or polyclonal antibodies are used, while in other embodiments, crude preparations are used. For example, in some embodiments, polyclonal antibodies in crude antiserum are utilized. It is intended that the term "antibody" encompass any immunoglobulin (e.g., immunoglobulins of any class.) obtained from any source (e.g., humans, rodents, lagomorphs, non-human primates, caprines, bovines, equines, ovines, etc.).

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2. The "light chains" of antibodies (immunoglobulins) from any vertebrate species can be assigned to one of two clearly distinct types, called kappa and lambda, based on the amino acid sequences of their constant domains. Discussion of antibody structure and terminology can be found in a variety of sources, *e.g.,* Paul, Fundamental Immunology, 4th Ed., 1999, Raven Press, New York.

Antibody Fragments: As used herein, "antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 [1995]); single-chain antibody molecules; and multispecific antibodies formed from antibody fragments.

An "isolated" antibody is an antibody that has been enriched by the removal or partial removal of at least one contaminating component. Contaminant components are generally materials which could interfere with uses for the antibody, and may include, for example, enzymes, hormones, and other proteinaceous or nonproteinaceous solutes. In preferred embodiments, the antibody will be purified to greater than 95% by weight of antibody as determined by the Lowry method, and most preferably more than 99% by weight. Alternatively, an antibody will be purified to homogeneity or near homogeneity as assayed by SDS-PAGE under reducing or nonreducing conditions using Coomassie blue staining, or preferably, by silver staining. Isolated antibody includes the antibody *in situ* within recombinant cells since at least one component of the antibody's natural environment will not be present. Ordinarily, however, isolated antibody will be prepared using at least one enrichment step.

Epitope: As used herein, the terms "antigenic determinant" and "epitope" refer to that portion of an antigen that makes contact with a particular antibody variable region. When a protein or fragment (or portion) of a protein is used to immunize a host animal, numerous regions of the protein may induce the production of antibodies which bind specifically to a given region or three-dimensional structure on the protein (these regions or structures are referred to as antigenic determinants). In some embodiments, a cell surface protein was used previously to raise an antibody e.g., a monoclonal untidily) or polyclonal antisera), and the resulting antibody is used as a probe to detect a protein biomolecule of interest on the cell is the subject of the biochemical analysis

Quantitating: As used herein, the terms "quantitating," "quantitation" and the like refer to the assignment of a numerical value to some signal that can be measured. For example, fluorescence intensity or color development can be quantitated, where the fluorescence or color signal intensity is assigned a numerical value. In some aspects, the quantitation of a signal (such as fluorescence or color intensity) can be correlated to the concentration of a biomolecule, for example, copy number of a gene transcript, absolute nucleic acid concentration, number of protein molecules present, absolute concentration of a protein that is present, etc. Generally, quantitating is one aspect of the boarder term "detecting." In some aspects, the term "detecting" refers to a simple binary assignment of "present" or "absent," without any determination of degree if a particular biomolecule is present.

Probe: As used herein in its broadest sense, the term "probe" refers to any moiety of diverse composition that has an affinity for a target biomolecule. For example, a probe can refer to an antibody that has binding specificity for a specific polypeptide, or can refer to a polynucleotide that has an affinity for a specific nucleic acid target. It is not intended that the present invention be limited to any particular probe label or probe detection system. As used herein, the term "target" refers to any biomolecule that is the cognate component for which a probe has an affinity.

High Throughput: The term "high throughput format" refers generally to a relatively rapid completion of an analysis.

Highly Parallel: The term "highly parallel" refers to the simultaneous processing and/or analysis of many samples.

Platform: The term "platform" refers to the general methodologies and equipment associated with the use of a given technology. For example, microfluidics and nanofluidic platforms are described in a variety of sources in the art. "Platform" can include protocols as well as specific devices.

### BRIEF DESCRIPTION OF THE FIGURES

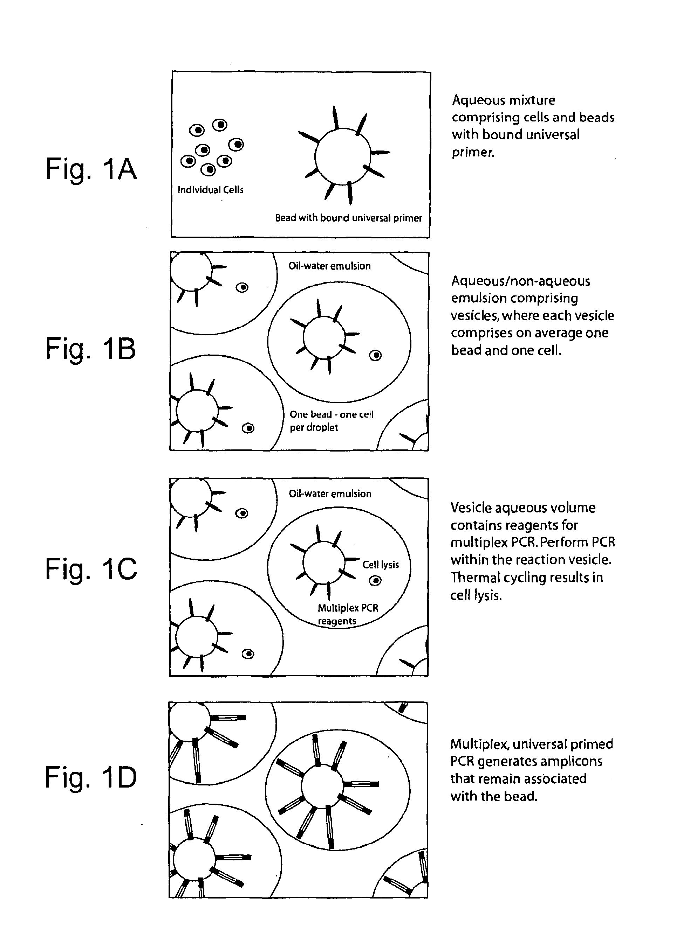

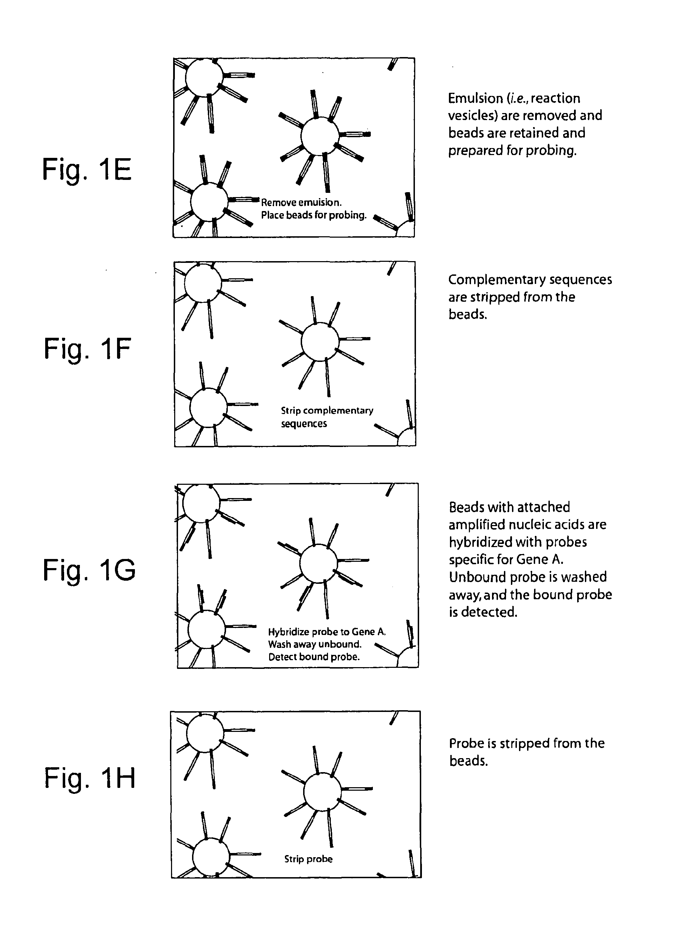

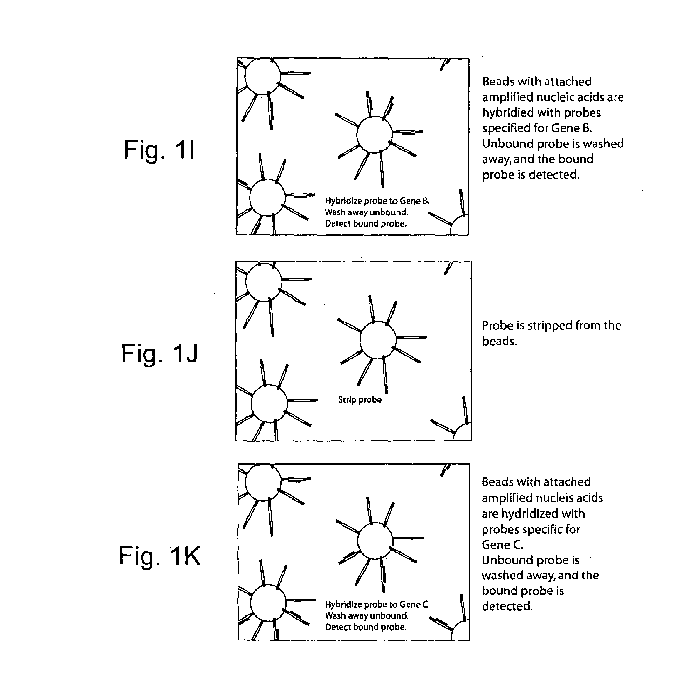

**FIGS. 1A-1K** provide illustrations of one embodiment of the invention for single cell gene expression analysis. In this embodiment, cells in an aqueous solution are combined with an immiscible phase in the presence of a solid phase bead reagent to form reaction vesicles that contain one cell and the beads (see **FIGS. 1A** and **1B****).** **FIGS. 1C-1K** illustrate the biochemical analysis of a biomolecule associated with the cells, which in this illustration is a nucleic acid.

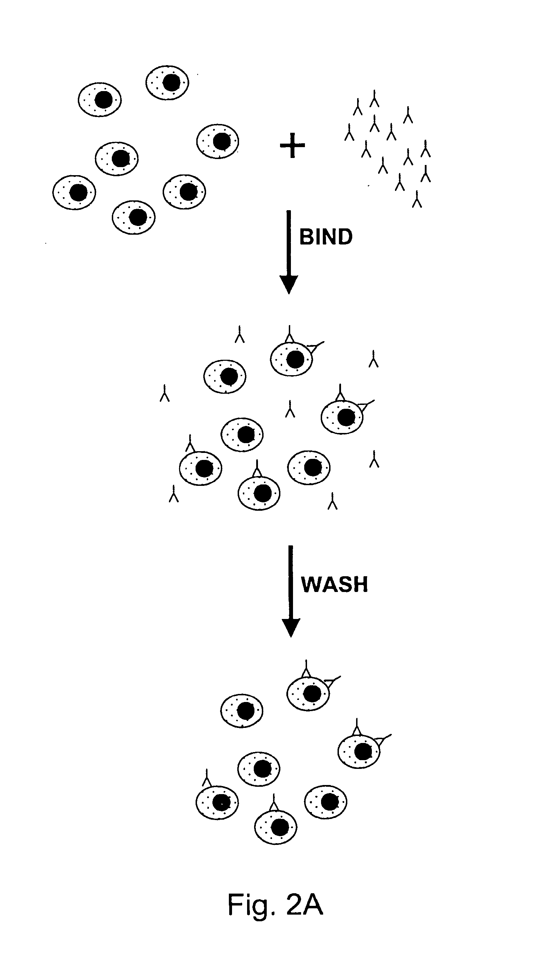

**FIGS. 2A** through **2C** provide illustrations of one embodiment of the invention for single-cell protein expression analysis. In this embodiment, cells in an aqueous solution are partitioned by an immiscible second solution to form reaction vesicles that contain single cells. Antibody-based reagents are used to detect protein expression.

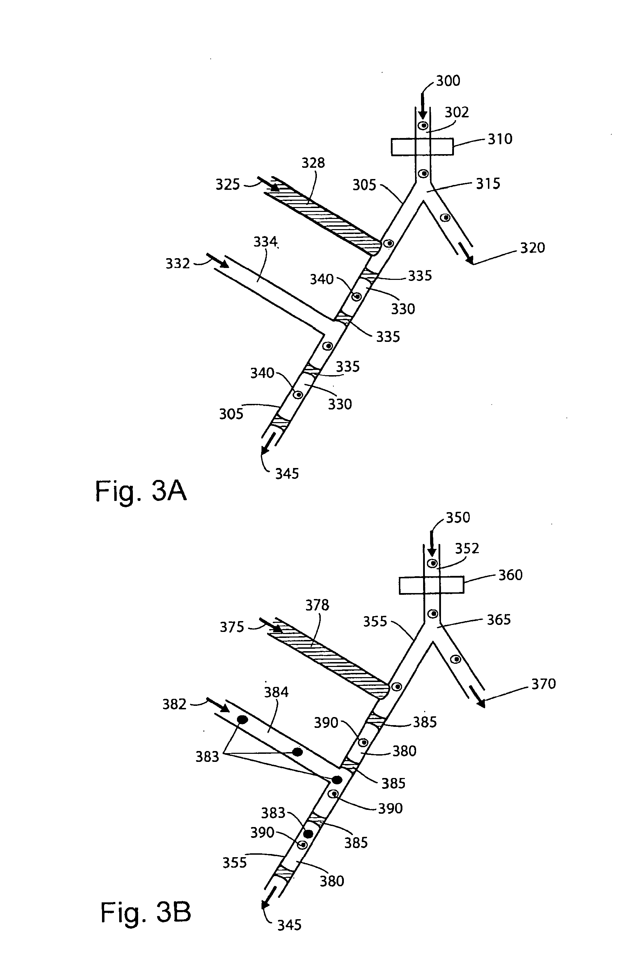

**FIGS. 3A** and **3B** provide illustrations of two embodiments of the invention. In these embodiments, cells in an aqueous solution are partitioned by an immiscible second solution to form partitioned aqueous reaction volumes in a flow channel. The system of **FIG. 3A** does not use solid phase beads in the optional delivery mechanism for aqueous regents. The system of **FIG. 3B** uses the optional delivery mechanism to deliver bead reagents into the partitioned aqueous reaction volumes.

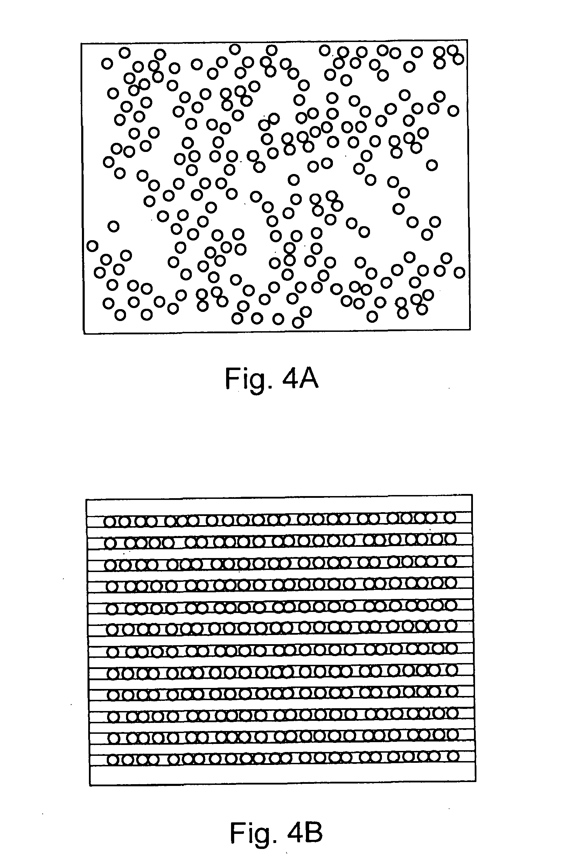

**FIGS. 4A** and **4B** provide illustrations of two embodiments of the invention. **FIG. 4A** shows an emulsion of reaction vesicles, where the vesicles in the emulsion are freely distributed in two dimensions. **FIG. 4B** shows an emulsion of reaction vesicles, where the vesicles are ordered in one dimension along a single channel.

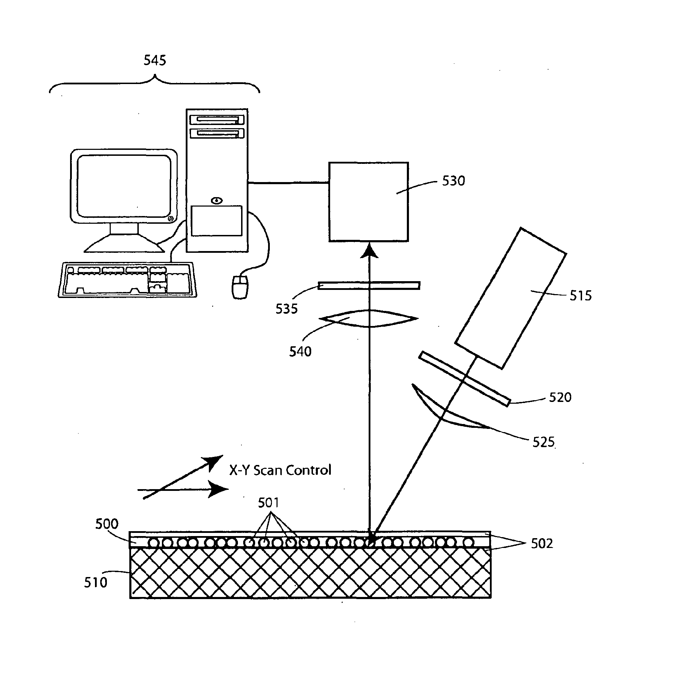

**FIG. 5** provides an illustration of an assay device of the invention.

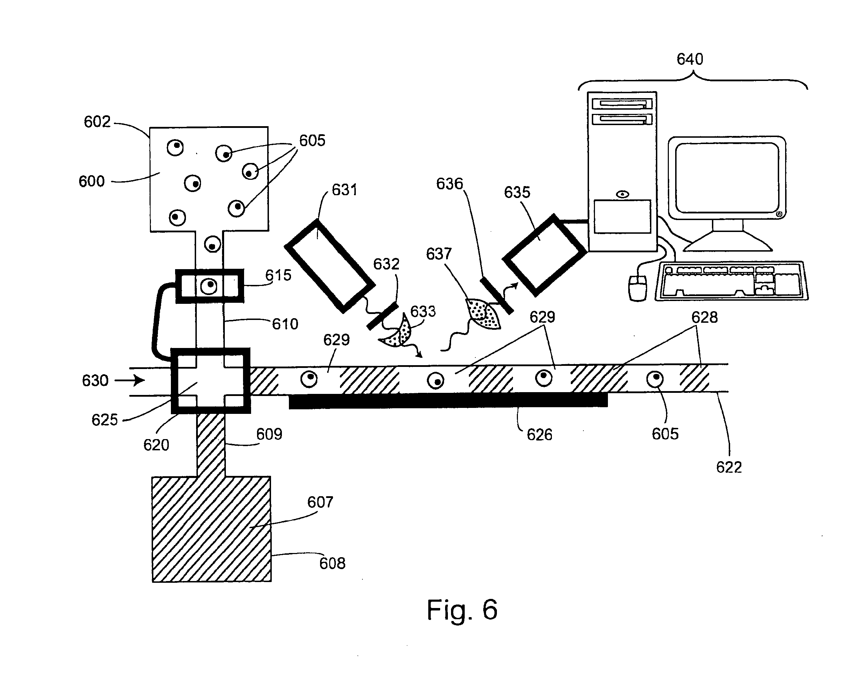

**FIG. 6** provides an illustration of an assay device of the invention.

### DETAILED DESCRIPTION OF THE INVENTION

The present invention provides compositions and methods for monitoring (e.g., detecting, quantitating, assaying) a plurality of biomolecules (e.g., proteins or nucleic acids) within or otherwise associated with individual cells or small groups of cells. In particularly advantageous embodiments, these compositions and methods for monitoring biomolecules associated with cells are used in high-throughput methodologies for the rapid assessment of large numbers of single cells. One of the beneficial features of the invention is the ability to apply analytical techniques to large numbers of single cells, thereby enabling the determination of a distribution of a particular characteristic in a population of individual cells, and not simply the population average of that particular trait.

### CELL PARTITIONING

The compositions and methods of the invention rely on various mechanisms to partition single cells into individual reaction volumes where a biochemical analysis can be performed and contained. These mechanisms have in common the use of two liquid phases; a first phase that is aqueous, and a second phase that is immiscible with the aqueous phase. Using various mechanisms, these two components are combined to form an aqueous reaction core solution that is partitioned from its local environment by an immiscible liquid shell formed from the second liquid. In some embodiments, such as when using partitioned flows in a flow channel, the immiscible phase the acts as a partition between aqueous phases can be an immiscible gas, such as air or its components or an inert gas such as helium or argon.

The consequence of the partitioning methods described herein is the creation of a physically isolated aqueous space containing individual cells or small groups of cells. These methods for creating the isolated aqueous volumes work by either completely surrounding the aqueous compartment with an immiscible phase barrier (i.e., vesicles) or by using the immiscible phase as a partition that separates adjacent aqueous reaction volumes in a flow channel (i.e., partitioned flows). The unifying characteristic of the partitioning step options is that the compartments, and the cells and reagents contained in those compartments, do not mingle with any other compartments or cells, and furthermore, permit self-contained biochemical assays in each compartment, and the biomolecules targeted for detection stay within their respective compartment.

### Reaction Vesicles

In some embodiments for generating the aqueous reaction core solution and associated immiscible liquid shell, the partitioning method involves the use of a multiphase system where there exists a first aqueous phase and a second liquid phase that is immiscible with the aqueous phase. This second phase is typically but not exclusively non-aqueous. The combination of these phases under appropriate conditions results in the formation of numerous aqueous microenvironments (also termed aqueous chambers or aqueous reaction cores) that are enveloped and contained by the immiscible (typically non-aqueous) liquid, resulting typically in an emulsion of vesicles (see **FIG. 1B****).** Methods for the generation of vesicles from an aqueous phase using a second immiscible component typically involve the combination of the two phases, e.g., by mixing, vibration, vortexing, , the injection of one phase into another, or the like. The combination of phases typically yields a vesicle emulsion where the vesicles are distributed evenly throughout the resulting mixture. In some embodiments, the vesicles are liposomes.

In some embodiments of the invention, the vesicles are formed from an aqueous solution containing cells that are the object of study. It is understood that when an aqueous solution comprises cells, that aqueous solution also typically comprises other components such as salts and buffers that create a physiologic or isotonic environment in order to preserve cell viability or structural integrity while encapsulated in the vesicle.

When the vesicles comprise cells, in some aspects, the resulting vesicles thus formed each contain not more than one cell. In other aspects, small numbers of cell can be encapsulated by a single vesicle, for example, between about 2 and about 100 cells can be encapsulated in a single vesicle, between about 2 and about 50 cells can be encapsulated in a vesicle, between about 2 and about 25 cells can be encapsulated in a vesicle, between about 2 and about 10 cells can be encapsulated in a vesicle, or between about 2 and about 5 cells can be encapsulated in a vesicle.

Methods for creating biphasic emulsions and encapsulating cells in vesicles rely on the use of specific ratios of the different non-miscible liquid components and concentrations of the cells in the aqueous phase. With proper mixing, a Poisson distribution of compartmentalized cells is achieved. In some embodiments, depending on the desired focus of the particular experiment, the mean distribution value can be one cell per partition. In general, for studies targeting single cell analysis, the mean distribution will be chosen to be less than one cell per partition to increase the total number of elements that have only one cell per partition. Thus, if a vesicle in a population of vesicles is predicted to contain only one cell, there exists a small probability that a vesicle may contain no cells, or more than one cell. As used herein, when the expression "a vesicle containing one cell" or similar expressions are used, it is understood that any one vesicle from the population of vesicles is statistically likely to contain one cell, but may not contain one cell. Similarly, vesicles can be created to contain any desired number of cells greater than one.

Methods and reagents for the generation of vesicles, and more specifically, vesicles that encapsulate cells, are widely known in the art and can be obtained from a variety of sources. See, e.g., Oberholzer et al. (1999), "Protein Expression in Liposomes," Biochem. Biophys. Res. Commun., 261:238-241; and Nir et al. (1990) "Single-cell entrapment and microcolony development within uniform microsperes amenable to flow cytometry," Applied and Environmental Microbiology, September 1990, p. 2870-2875.

The liquid that forms the encapsulation layer of the vesicle is typically non-aqueous. As used herein, the expression "non-aqueous phase" can be used synonymously with the term "immiscible in the aqueous phase." Fluids that find use in vesicle formation include, for example but not limited to, silicone oils, mineral oil, and the partially fluorinated alkane perfluorohexyloctane (F6H8).

Silicone oils (also known as polymerized siloxanes) are silicon analogues of carbon based organic compounds, and can form long and complex molecules based on silicon rather than carbon. Chains are typically formed of alternating silicon-oxygen atoms (...Si-O-Si-O-Si...) or siloxane, rather than carbon atoms (...C-C-C-C...). Substituents or other moieties can be attached to the tetravalent silicon atoms, but typically not to the divalent oxygen atoms that are fully committed to forming the siloxane chain. A typical example of a silicone oil is polydimethylsiloxane (also termed dimethicone), where two methyl groups attach to each silicon atom to form (H₃C)[SiO(CH₃)₂]ₙSi(CH₃).

Lipids can also be used to form vesicles. The vesicles formed from lipids are generally bilayer structures encapsulating an aqueous core. Lipids finding use in vesicle formation include, for example:
1-palmitoyl-2-oleoyl-*sn*-glycero-3-phosphocholine (POPC)
1-palmitoyl-2-oleoyl-*sn*-glycero-3-phospho-L-serine (POPS)
Mixtures of lipids are also commonly used vesicles.

It is understood that the immiscible phase need not be a pure solution comprising a single component, e.g., a pure liquid of a single lipid or a pure silicone oil. Mixtures of various liquid compounds can form the solution that is immiscible in the aqueous phase. Furthermore, the non-aqueous phase can contain dissolved components.

Numerous methods are known in the art for producing controlled sizes of vesicles. Various methods of mixing that employ shaking, vibration, sonication and the like all can be performed in a manner to generate relatively uniform sizes of vesicle. Alternatively, methods can be employed where the aqueous solution is introduced into the immiscible solution in a controlled manner. For example, port or direct injection of the aqueous solution, wherein passage through the port of a controlled flow of solution can naturally promote the formation of vesicles of uniform size and volume. Such injection systems can be used where there is a continuous flow of aqueous solution into the immiscible, non-aqueous phase, or where the flow is pulsed, introducing fixed sized boluses of aqueous solution into the immiscible solution. The immiscible solution that receives the aqueous phase can either be static or in motion, flowing by the point where the aqueous solution is being injected.

### Fluid transfer systems

As an alternative to formation of an emulsion and creation of vesicles, methods to mechanically divide the cell population in the aqueous solution into small volume quantities via micro and nanoliter fluid transfer systems can be employed. Alternative mechanisms further include the introduction of an aqueous solution in boluses into an immiscible, non-aqueous solution through the use of a liquid dispenser, or dispensing into physically partitioned containers such as chambers, wells or depressions in a plate. In these types of applications, the aqueous solution containing the cells can be delivered in a limiting dilution, thereby delivering one cell (on average) to the chambers, wells, depressions or vector positions on a surface. These single cell volumes can then be analyzed in any desired biochemical assay, advantageously using a high throughput and highly parallel methodology.

### Partitioned Aqueous Flows

In other aspects, the generation of the aqueous reaction core solution and associated immiscible liquid shell involves the use of partitioned flows of an aqueous solution and a second immiscible solution in a flow system to generate discrete aqueous reaction volumes (e.g., in a flow system channel) where a biochemical analysis can be performed and contained. The second phase that is used to partition the aqueous compartments is typically but not exclusively non-aqueous. In some embodiments, this aspect of the invention is practiced using a device that incorporates microfluidics technology, as widely known in the art. In preferred embodiments, the aqueous solution comprises cells, which are then contained in the partitioned aqueous compartments. In some embodiments, each partitioned aqueous compartment comprises on average only one cell. In some aspects, a biochemical assay is conducted in the partitioned aqueous compartment, in which case the compartment can be termed a partitioned aqueous reaction volume.

The non-aqueous liquids that find use a partitioning phases is not particularly limited. A variety of suitable non-aqueous liquids are known, for example, silicone oil. In the partitioned flow systems of the invention, the non-aqueous liquid needs to be both immiscible with the aqueous phase and also interact with the walls of the flow chamber in such a manner that they retain their partitioning property while at the same time continue to flow along the flow channel.

A partitioned aqueous reaction volume is depicted schematically in **FIG. 3A****.** In that figure, an aqueous flow is channeled through a flow channel (resulting in a channeled liquid flow). Coupled to the flow channel is a source for non-aqueous (i.e., immiscible) fluid. The non-aqueous fluid is delivered into the flow channel at regular intervals, thereby creating partitions between aqueous segments. In some aspects, the aqueous solution comprises cells, where each partitioned aqueous segment comprises on average one cell. In some embodiments, the aqueous solution further comprises one or more reagents for assaying a biomolecule associated with the cell, and the partitioned aqueous solution is termed a partitioned aqueous reaction volume.

In various embodiments as described above, the non-aqueous solution is delivered into the aqueous stream in such a manner that it interrupts the flow of the aqueous stream and divides the aqueous flow into multiple compartments to form the partitioned aqueous chambers (see **FIGS. 3A** and **3B****).** In other embodiments, the aqueous solution containing the cells is delivered into a non-aqueous stream in such a manner that it interrupts the flow of the non-aqueous stream and creates multiple pockets of aqueous solution, thereby forming the partitioned aqueous chambers. In still other embodiments, the aqueous solution and the non-aqueous solution are coupled to a microfluidics channel intersection, where the aqueous phase and the non-aqueous phase are alternatively delivered into a flow channel through the intersection, thereby forming the partitioned aqueous volumes (see **FIG. 6****).** It is not intended that the invention be limited to any particular method for forming the partitioned aqueous reaction volumes in a flow channel, as one of skill in the art will be familiar with existing liquid flow and microfluidics technologies that are adaptable for use with the present invention.

In some embodiments, the generation of partitioned aqueous reaction volumes contain only one cell in a flow channel partition. The present invention provides compositions and methods for improving the accuracy of one cell distributions in a flow system. As commonly used in flow cytometry, a stream of fluid containing single cells in the flow can be monitored optically for the presence of a passing cell (see **FIG. 6****).** Such optical monitoring can be incorporated into the flow systems of the present invention, where the detection of a cell passing in aqueous flow stream (for example, see optical sensor **615** and coupling **610** in **FIG. 6****)** initiates the delivery of the immiscible partition phase into the aqueous flow, thereby creating partitioned aqueous reaction volumes in the flow channel.

In some aspects, partitioning an aqueous flow in a flow system incorporates the following features: (a) a mechanism for detecting cells as they pass by a specific site in the flow stream, and (b) a means to introduce the non-aqueous solution into the aqueous flow between the cells in response to detecting the cells in the flow stream to interrupt the aqueous flow to create non-aqueous partitions between cells. For example, this can be accomplished by use of an optical sensor that can detect the passage of cells through the aqueous portion of the flow stream. The sensor is operably connected to the controller, such that detection of a cell by the sensor triggers the controller to insert a non-aqueous partition into the flow stream to compartmentalize the detected cell. Flow cytometry systems utilize optical methods such as light scattering to detect the presence of cells in the flow stream. Similarly, cellular optical sensing functionality can also be included in the devices of the present invention (see **FIGS. 3A, 3B** and **6****).** The process of cell detection and knowledge of the flow rate enable one to determine the movement and location of cells in a flow pipeline and provide the means to synchronize the introduction of the second immiscible solution via injection or merger into the flow pipeline. Additional elements can be incorporated into the flow pipeline to allow for the introduction of additional aqueous and non-aqueous solutions as well as solid phase elements such as beads. The different solutions can be injected into the flow system using precise injection technologies such as piezo injectors, or any number of different microfluidic valving systems know in the art where the flow of solution at fluidic junctions is controlled. Flow systems can also be optionally coupled with mechanical partitioning methods to provide a final disposition for the cells, where the cells are partitioned into separate physical locations or aqueous droplets.

Optical sensing capability in the flow systems of the invention also provides a mechanism for presorting the cell population for analysis so that only a desired subset of the cell population will be channeled for further analysis.

### HIGH DENSITY PARTITIONING

The invention provides compositions and methods for the compartmentalization of single cells or groups of cells into isolated reaction chambers, where the cell or cells in each chamber can be subject to a biochemical analysis. The invention makes use of a two immiscible phases to form the cell compartments. The first type of these compartments are reaction vesicles produced in an emulsion, where the vesicle contains a core aqueous reaction volume containing the cell or cells. The second type of compartment is a partitioned aqueous reaction volume in a flow channel, where the aqueous compartments are separated by immiscible fluid partitions.

It is an advantageous feature of the invention that the compartmentalization methods are intended to be used in a highly reiterative, high throughput, highly parallel manner. For all envisioned aspects of the invention described herein, high density compartmentalization of the individual cells or small groups of cells is a key aspect. Compartmentalization provides the means wherein the biomolecule to be detected can be characterized with regard to their diversity of representation within the different cells or small groups of cells that comprise the larger group. High density partitioning is essential when the goal is to uniquely analyze, for example, at least 100, at least 1,000, at least 10,000, at least 100,000 or at least 1,000,000 cells or groups of cells in parallel.

The detection of variance, or lack there of, in the distribution of a particular biomolecule in a collection of individual cells from a population is key to providing deeper insight into certain biological phenomena. For example, studying a biomolecule distribution (e.g., an expressed gene) in a large number of individual cells can yield a deeper understanding of rare cell types (e.g., stem cells, tumor cells or T and B cells) or rare events that give rise to particular cell phenotypes, e.g. infection, mutation, recombination, immune response, proliferation, apoptosis, necrosis, hypoxia, migration, invasion, differentiation, dedifferentiation, and cross cell signaling and the like, that give rise to one or more disease states, e.g., cancer, infection, autoimmunity, inflammation, organ dysfunction, cardiovascular abnormality, neurological disease and the like. Alternatively, the rare cell types to study may be different from the cells around them, e.g., a rare cell type within an organism or organ, fetal cells in maternal blood, bacterial cells, viruses and the like that have infected a host, a mutant or recombinant cell in a pool of normal cells, a rare eukaryotic or prokaryotic type or strain among more common types or strains, or mixed communities of eukaryotes and/or prokaryotes.

### CELLS

The invention provides compositions and methods for single cell biochemical analysis, and advantageously, high-throughput single cell analysis. It is not intended that the cells used in the analysis be limited in any way to any particular type of cell. Although some cells types are taught herein for the purpose of describing the invention, one of skill in the art will recognize that use of the invention is not bounded by any particular cell type, and the invention finds use with a wide variety of cell types. The biological question to be addressed determines the cell type to be used.

In some aspects, eukaryotic cells are the subject of analysis. The term "eukaryote" refers to organisms belonging to the Kingdom Eucarya. Eukaryotes are generally distinguishable from prokaryotes by their typically multicellular organization (but not exclusively multicellular, for example, yeast), the presence of a membrane-bound nucleus and other membrane-bound organelles, linear genetic material (*i.e*., linear chromosomes), the absence of operons, the presence of introns, message capping and poly-A mRNA, and other biochemical characteristics, such as a distinguishing ribosomal structure. Eukaryotic organisms include, for example, animals (*e.g.,* mammals, insects, reptiles, birds, etc.), ciliates, plants (*e.g.,* monocots, dicots, algae, etc.), fungi, yeasts, flagellates, microsporidia, protists, etc.

In particularly advantageous embodiments, the mammalian cells used in conjunction with the invention are human cells, such that the analysis of the population of human cells can give insight into the presence or absence of disease or pathology (i.e., diagnostic analysis) or disease prognosis.

In some aspects, prokaryotic cells are the subject of analysis. The term "prokaryote" refers to organisms belonging to the Kingdom Monera (also termed Procarya). Prokaryotic organisms are generally distinguishable from eukaryotes by their unicellular organization, asexual reproduction by budding or fission, the lack of a membrane-bound nucleus or other membrane-bound organelles, a circular chromosome, the presence of operons, the absence of introns, message capping and poly-A mRNA, and other biochemical characteristics, such as a distinguishing ribosomal structure. The Prokarya include subkingdoms Eubacteria and Archaea (sometimes termed "Archaebacteria"). Cyanobacteria (the blue green algae) and mycoplasma are sometimes given separate classifications under the Kingdom Monera.

In some aspects, the prokaryotic cells that are studied are bacteria. As used herein, the terms "bacteria" (and synonymously "eubacteria") refer to prokaryotic organisms that are distinguishable from Archaea. Similarly, Archaea refers to prokaryotes that are distinguishable from eubacteria. Eubacteria and Archaea can be distinguished by a number morphological and biochemical criteria. For example, differences in ribosomal RNA sequences, RNA polymerase structure, the presence or absence of introns, antibiotic sensitivity, the presence or absence of cell wall peptidoglycans and other cell wall components, the branched versus unbranched structures of membrane lipids, and the presence/absence of histones and histone-like proteins are used to assign an organism to Eubacteria or Archaea.

In general, the first step in the methods described herein is to obtain the cells to be analyzed. These cells can be single cells in suspension, single cells that are attached to a solid surface or single cells that are part of a greater structured grouping such as a tissue, organ or organism. In the latter cases, the methods require that the single cells be detached, disaggregated and/or separated from one another and from the cells not be analyzed. In a preferred embodiment, all of the cells of interest are segregated and suspended in an aqueous solution. Separation of cells can involve the use of enzymes to digest extracellular matrices, chemicals to dissolve solidifying elements such as fixing agents and paraffin, physical methods of cutting, dicing and slicing tissue, as well as, mechanical and optics-based methods of microdissection.

In some aspects, cells in an aqueous solution that are the subject of analysis are concentrated prior to compartmentalization in partitioned aqueous volumes in a flow channel or in vesicles in an emulsion. In other aspects, the cells to by analyzed are diluted prior to compartmentalization. For example, in order to achieve compartmentalization that results in one cell per partition (on average), it can be necessary to concentrate or dilute the cell aqueous volume prior to the compartmentalization step.

In the case where cells need to be concentrated prior to further analysis, it is often necessary to incorporate a resuspension step. For example, cells can be concentrated in a centrifuge, the supernatant removed, and the cell pellet resuspended in a preferred aqueous solution (e.g., a preferred buffer, physiological solution or isotonic solution). Cells can also be concentrated by other techniques known to one skilled in the art including dialysis or affinity capture. In some embodiments, the aqueous liquid used to resuspend the cells also comprises the reagent or reagents that are required to execute the biochemical assay to detect, quantitate or amplify the biomolecule of interest, or amplify a signal associated with detection.

### BIOMOLECULES

The invention provides compositions and methods for assaying the presence or absence (or quantity) of a biomolecule associated with a cell, preferably in a high-throughput single cell analysis. It is not intended that the biomolecule that is the target of the analysis be limited in any way to any particular cell component. Although some examples are discussed herein for the purpose of illustrating the invention, one of skill in the art will recognize that use of the invention is not bounded by any particular biomolecule target or biochemical assay, and the invention finds use with a wide variety of biochemical targets and assays.

As used herein, a biomolecule associated with a cell is any molecule that can be produced by a cell. The biomolecule can be internal to the cell (e.g., in the nucleus, in the cytoplasm, in any cell organelle, or integral to any intracellular cell membrane or external cell membrane), and/or exposed on the external surface of the cell (e.g., a cell membrane receptor). Examples of biomolecules include but are not limited to, nucleic acids, proteins (including peptides and polypeptides) carbohydrates, lipids and any other organic biomolecules (e.g., free amino acids, vitamins and hormones such as steroids). Methodologies and reagents for detecting and/or quantitating biomolecules such as those listed above are common in the art and find use with the invention.

In some embodiments, it is desirable to detect and/or quantitate a control biomolecule in the biomolecule assay, such as a reference standard, a calibration standard, a quantitation standard, an endogenous reference component, an externally supplied reference component, or similar such component. Inclusion of these types of assays and reagents are within the scope of the invention.

In some aspects, a reference biomolecule component (e.g., a reference protein or a reference nucleic acid) is coanalyzed along with the protein or gene of interest. The reference component can be internal (or endogenous) to the cell being analyzed, or it can be an artificially supplied to the system (e.g., is externally added (or exogenous) to the sample).

In the case where the biomolecule of interest is an expressed gene nucleic acid, a suitable "reference sequence" or "reference gene" can be coanalyzed along with the gene of interest. Constitutively expressed endogenous genes (alternatively termed housekeeping genes) are frequently used as reference genes. In addition to being well represented in multiple tissue types, reference genes tend to maintain very consistent levels of expression from sample to sample and individual to individual. This constitutive and stable expression provides a baseline, in terms of relative gene ratios, that can be used as part of the analysis of one or more other expressed genes.

A wide variety of reference genes are known in the art and which find use with the methods of the invention. The table below, which is not exhaustive, provides a representative list of such genes.

**TABLE**

| **Reference Genes** |
|---|
| β-2-microglobulin |
| Hypoxanthine ribosyl transferase |
| Transferrin Receptor |
| Transcription Factor IID |
| 18S rRNA |
| Acidic Ribosomal Protein |
| plycerol kinase |
| β-plucuronidase |
| Lysosomal hyaluronidase |
| Proteasome subunit Y |
| Elongation factor EF-1-alpha |
| Ribosomal protein L37a (RPL37A) |
| Ca₂-activated neutral protease large subunit |
| 18kDa Alu RNA binding protein |
| Nuclear factor NF45 |
| E2 Ubiquitin conjugating enzyme UbcH5B |
| Histone deacetylase HD1 |
| Ezrin |
| QRSHs glutaminyl-tRNA synthetase |
| 16S rRNA |
| MLN51 |
| ATP synthase |
| cyclophilin A |
| β-actin |
| GAPDH |

In one aspect, the reference proteins or genes used in conjunction with methods of the invention can be proteins or genes classically used as reference genes, e.g., β-actin and GAPDH. Alternatively, genes or proteins that are cell, tissue or organism specific can also be used in the methods of the invention, for example, in identifying a rare cell type in a population of cells.

With regard to the use of endogenous standards, there are many examples where the information of value is the relative quantities and ratios of different biomolecules that comprise the single cell or groups of cells. For example, chromosomal amplifications as measured by the increase in copy number of one segment of genomic DNA versus another segment of DNA, differences in gene expression response of one more RNA transcript compared to a "housekeeping" transcript, the relative levels of a protein in different functional forms, the presence and ratios of multiple metabolites, and combinations of any or all of the above. These types of assays are within the scope of the invention. Where a plurality of nucleic acids or proteins are analyzed in a multiplex manner, it is possible to generate a expression profile (e.g., a gene expression profile) of the various targets being analyzed.

### BIOCHEMICAL ASSAYS AND REAGENTS

In some embodiments of the invention, the aqueous phases contained within the vesicles or the partitioned aqueous reaction volumes in a flow channel comprise at least one cell, and further comprise at least one reagent for assaying (*i.e.*, determining the presence or absence, or quantitating) a biomolecule associated with the cell.

The compositions and methods of the invention can be used advantageously in high-throughput applications for conducting biochemical assays on large numbers of single cells, where the single cells correspond to a single vesicle or a single partitioned aqueous volumes in a flow channel. In some aspects of the invention, the detection methodology (and detection apparatus) used in the biochemical assay has the ability to resolve assay results for single vesicles or single partitioned aqueous volumes in a flow channel.

It is not intended that the invention be limited in any way as to the target or nature of the biochemical assay, nor limited to any particular reagent(s) that are included in the aqueous phase for the execution of a biochemical assay. For example, the biochemical assay of the invention can be used to detect or quantitate a genomic nucleic acid, an expressed gene sequence (e.g., a messenger RNA, or mRNA, a cDNA, ribosomal RNA, transfer RNA (tRNA), micro RNA), nucleic acid sequence polymorphisms, proteins, and other cellular components. The biochemical assays can be used to determine the presence or absence of any given biomolecule, the concentration of the biomolecule, the copy number of the biomolecule, the conformation of the biomolecule, the presence or absence of moieties that interact with a biomolecule, modifications of a biomolecule (i.e., proteolysis, post-translational modifications of a protein, or RNA splicing or editing), or localization of a biomolecule.

The method for introducing the biochemical assay reagent(s) to the aqueous phase (e.g., to the vesicle or to the partitioned aqueous reaction volumes in a flow channel) is not limited. The introduction of reagents can be via a number of different approaches prior to or post cell partitioning. The reagent(s) can be incorporated within the initial aqueous solution comprising the cell population, for example, at the time of mixing of the aqueous and non-aqueous phases during formation of an emulsion and reaction vesicles (see **FIGS. 1A** through **1C****,** and **2A** through **2C).** Reagents can also be added to existing vesicles post formation by the merger of a second population of vesicles that contain the reagents, thereby delivering the regents to the vesicles comprising the cells.

When using fluidic technologies to generate partitioned chambers in a flow channel, the reagent(s) can be incorporated within the initial aqueous solution comprising the cell population, for example, prior to partitioning in the flow channel, or alternatively, introduced into the flow stream after the partitioning step in a flow channel via an injection point (see, **FIGS. 3A** and **3B****).** Using fluidic technologies, the reagents can be added through the merger of two or more fluidic flows to form a new single partitioned aqueous reaction volume containing the desired reagent(s). The nature of structure of the fluidic mechanisms used is not limited. Further still, a third phase possessing a biochemical assay reagent (e.g., a second aqueous phase containing a solid phase such as a bead) can be merged with the partitioned aqueous reaction volume in a flow channel **(****FIG. 3B****).**

The structure or nature of the reagent(s) present in the aqueous phase is not limiting. For example, in order to illustrate but not limit the invention, reagents for detecting nucleic acid biomolecules can include: polynucleotide probes, amplification primers, fluorescently labeled polynucleotides including Molecular Beacon™ probes, TaqMan^{®} probes, Scorpion^{®} probes (Eurogentec), Sunrise™ primers, intercalating dyes such including ethidium bromide and SYBR^{®} Green, polymerases, nucleases, ligases, free nucleotides, universal primers, branched DNA molecules, biotinylated polynucleotides, biotinylated free nucleotides, streptavidin-chromophore conjugates, polynucleotide-bead conjugated, and streptavidin-bead conjugates.

In some aspects, a reagent generates the signal (e.g., an amplified signal) corresponding to the presence or absence of the biomolecule, and that signal that is detected by a detector. In other aspects, the reagent is only a participant in the reaction that generates the signal, and the reagent itself does not generate the signal. For example, a Molecular Beacon™ probe reagent generates a detectable signal. In contrast, a nucleic acid polymerase does not by itself generate a detectable signal.

Similarly, in order to illustrate but not limit the invention, reagents for detecting protein biomolecules can include: target-specific antibodies, secondary anti-target-specific-antibody antibodies, chromophore-labeled antibodies, antibody-conjugated enzymes (e.g., HRP, alkaline phosphatase); chromogenic enzyme substrates, labelled receptor ligands, antibody-polynucleotide conjugates, and antibody-bead conjugates.

Generally, reagents used with methods of the invention are provided in a stabilized form, so as to prevent degradation or other loss during prolonged storage, e.g., from leakage, exposure to moisture or exposure to light. A number of stabilizing processes are widely used for reagents that are to be stored, such as the inclusion of chemical stabilizers (i.e., enzymatic inhibitors, microcides/bacteriostats, anticoagulants), the physical stabilization of the material, e.g., through immobilization on a solid support, entrapment in a matrix (i.e., a gel), lyophilization, or the like.

Strategies for the addition of biochemical assay reagents (including biochemical probes) either prior to or post compartmentalization of the cells are envisioned. Embodiments where the addition occurs prior to high density partitioning are straightforward, where the cell population to be analyzed is mixed with the biochemical reagents, with no additional requirements for washing. These reagents must be compatible with the maintenance of the cells or the subcellular components to be analyzed, e.g., nucleic, mitochondria, etc. The biochemical reagents can include enzymes, binding ligands, buffers, salts, nucleotides, protein substrates, signaling moieties, and other elements required for one or more schemes of biomolecule detection, including amplification and signal generation.

The addition of biochemical reagents to the aqueous phase post partitioning can also be utilized. Methods for addition involve the addition to or merging of reagents in aqueous form with the existing cells in their existing aqueous environments. In the case where vesicles are utilized, the biochemical reagents can be merged into the vesicle. The merger process can be enabled through a variety of means including injection, the creation of confined environments promoting vesicle-vesicle merging, promoting of vesicle collisions in a controlled manner, e.g., having vesicles meet at an intersection in a flow channel or a microfluidic environment. Additional methods will be readily apparent to one of skill in the art.

In some embodiments, where at least part of the cell-containing aqueous environment is in contact with a solid phase, biochemical reagents can be added via dissociation, dissolution or desorption from the solid phase into the aqueous phase. In this case, the solid phase can be made out of any number of materials that can readily hold biochemical reagents and release them for use, including porous metals, plastics or glass, polymer matrices such as polyacrylamide, gels, fibers or filters. In addition, one or more biochemical reagents can remain bound to or covalently linked to the solid phase. With regards to the structure of the solid phase, the structure can be one where it is a surface in contact with the cell-containing environment, encapsulating or encasing the cell-containing environment, or contained partially or wholly within the cell-containing aqueous environment such as in the form of a bead.

### Signal Amplification

In some aspects of the invention, the detection method used to detect a signal corresponding to the presence or absence of a biomolecule associated with the cell or cells in a partitioned aqueous space uses an amplification scheme, where either a signal is amplified, or the molecule of interest is amplified.

In some embodiments, a signal is amplified using techniques well known in the art. For example, when the biomolecule of interest is a cell surface protein, that protein can be detected using a first antibody specific for that protein. That first antibody can in turn be detected (if present after a washing step) by a secondary antibody specific for the first that has been conjugated to a signal producing enzyme, e.g., horseradish peroxidase (HRP) enzyme. A colorimetric substrate (s) can be added, where the HRP produces a detectable product (P) after catalysis of the colorimetric substrate. In this methodology, it is the detectable signal that is amplified (not the protein being detected). See, for example,

### FIGS. 2A-2C.

In other aspects, the biomolecule of interest is amplified. When detecting a nucleic acid, the number of molecules of that nucleic acid can be increased (i.e., amplified) by the polymerase chain reaction (PCR) prior to detecting the nucleic acid.

In some aspects of the invention, the detection methods to detect a signal corresponding to the biomolecule of interest utilize signal or nucleic acid amplification schemes where a homogeneous detection method is utilized. A variety of methods known in the art have been established for homogeneous detection of biomolecules.

For proteins, multiple detection strategies involving antibody-mediated detection have been established including strategies that can be performed homogeneously. Nucleic acid amplification and detection methods as described herein can also be applied to protein detection via the use, for example, of antibody/ligand coupled to nucleic acids probes as described in methods of immuno-PCR. For nucleic acids, hybridization and amplification methods linked to the use of a dye that either increases in fluorescence when bound to double stranded nucleic acid, such a SYBR^{®} Green or ethidium bromide, or increases in fluorescence when separated from a second dye acting as a fluorescence quenching dye via fluorescence resonance energy transfer (FRET), such as in dye-labeled TaqMan^{®} probes, Sunrise™ primers, Scorpion™ primers and Molecular Beacons™, enables the homogeneous detection of nucleic acids. As known in the art, alternative strategies include, but are not limited to, the use of fluorescence polarization and decay, phosphorescence, absorption, light scatter, magnetic resonance.

For the detection of specific nucleic acids within a cell or linked to a cell, amplification is generally required, although high sensitivity detection systems not requiring amplification do exist. Improved optical and other detection methods make possible detection methods that do not require nucleic acid amplification. Multiple methods of amplification involving linear and exponential strategies of polymerization, ligation, and nuclease cutting have been established and are known to one skilled in the art. All of these methods create increasing concentrations of selected nucleic acid sequences wherein the presence of and increasing concentrations of these selected nucleic acid sequences can be detected in a homogeneous assay through the use of one or more of the dye/label strategies that are also known to one skilled in the art. Alternatively, these amplification events can lead to the generation of substrates that can be acted upon to generate a detectable moiety, such as through enzyme catalysis.

### Homogenous Detection Assays

A key feature of the invention is the coupling of cell compartmentalization strategies with the use of homogeneous detection assays within the aqueous volume of the compartments. These aqueous volumes contain at least one cell and at least one biochemical assay reagent. The discrete aqueous volumes can take the form of vesicles as found in an emulsion, or partitioned aqueous reaction chambers formed in a flow channel. The use of homogenous detection assays within the aqueous compartments in the methods of the invention has various advantages.

In one aspect, in the preparation of vesicles, the aqueous solution comprises cells and the specific biochemical reagents necessary for the amplification and homogeneous detection of the nucleic acid sequences of interest. In strategies utilizing homogeneous detection, the vesicles are not disrupted prior to detection but are examined intact so as to maintain the specific partitioning of the different cells and the linked detectable amplified products. In this embodiment, the products are detected by direct observation of single vesicles and the levels of fluorescence or other types of signal present within each vesicle is measured.

In some embodiments, the utilization of homogeneous amplification schemes enables the vesicles to be observed in real-time as the amplification is occurring. In such circumstances, the mechanics for both enabling amplification and the detection need to coexist or be performed in a sequential repeated manner. The amplification and detection steps can be occurring simultaneously or the mechanics can be arranged in an exchangeable format where the vesicles are alternately subjected to amplification and detection at multiple time points. In other embodiments, the light or other signal emission emanating from the vesicles is detected at only one time point, typically the final end point for the amplification reaction.

### Biochemical Probes

In some embodiments of the invention, the cells to be analyzed are contacted with one or more moieties (e.g., biochemical probes), where the moiety binds and/or detects one or more biomolecule associated with the cell. The term "biochemical probe" is broadly defined, and can refer to any molecule, regardless to structure, that has the ability to specifically bind to a biomolecule target.

Cell surface molecule detection is relatively straightforward, and such methods are well known in the art. For example, the use of antibody probes to bind to a cognate cell surface protein is a well established methodology for using a probe/target pair. Receptor ligands are another example of a cell surface biochemical probe. Other forms of biochemical probes can be used to readily enter the cells and bind to their target biomolecule within the cell. For example, many relatively small molecules can enter cells and tightly bind to their target biomolecule. Other larger biochemical probes can be made to enter cells via multiple methods known in the art, such as methods that temporarily permeabilize the outer membrane, including electroporation or the use of DMSO, methods for encapsulating the biomolecular probe via the uses of lipids or viral encapsulation, or linking the biomolecular probe to other biomolecules that transport across one or more cellular membranes. For the purposes of molecular detection, the biochemical probes further can comprise a detectable component where the detectable component is a molecule that is used to trigger a detection, e.g., a substrate or catalyst for amplification and signal generation, or catalyzes the transformation of a substrate into a product that can be detected, e.g., an enzyme or metal. Biochemical probes can be linked to the detectable component through one or more secondary biochemical probes that selective bind to the primary biochemical probes, e.g., anti-antibody antibodies.

Prebinding of the probe to the cell or cells prior to compartmentalizing the cells (e.g., in vesicles or by partitioning in a flow system) provides the further option to allow for the removal of excess biochemical probes from the system, where the excess probe have not bound to one or more biomolecule of the cell. Generally, removal of unbound probe makes use of the fact that unbound, excess probe is capable of being diffused or washed from the cells in a manner wherein bound probe is retained. Multiple methods for washing, extraction, diffusion and dialysis as known in the art can be employed. Other methods are also known in the art that do not require the removal of excess, unbound probe where only bound probe by virtue of the binding event becomes altered and is capable of promoting detection only in its altered form.

### METHODOLOGIES FOR ASSAYING NUCLEIC ACIDS

Any available method for detecting nucleic acids (amplified or unamplified) can be used in conjunction with the present invention. Common approaches include real time amplification detection with molecular beacons or TaqMan™ probes, detection of intercalating dyes (ethidium bromide or SYBR^{®} Green), detection of labels incorporated into the amplification probes or the amplified nucleic acids themselves, e.g., following electrophoretic separation of the amplification products from unincorporated label), and/or detection of secondary reagents that bind to the nucleic acids. Descriptions of these common methods are widely available, as are the reagents for conducing the detection assays.

In some cases, the direct detection of nucleic acids without an amplification step can be employed. In this case, unamplified genomic material or unamplified expressed transcripts can optionally be the target of analysis. Techniques for the analysis of (*e.g.,* fluorescence detection of) nucleic acids with very low copy number are known in the art and find use with the invention for detecting unamplified nucleic acids. See, for example, Mirkin et al., "PCR-Less detection of genomic DNA with nanoparticle probes," Abstracts of Papers, 222nd ACS National Meeting, Chicago, IL, United States (August 26-30, 2001). Such methods find use with the invention, and are within the scope of the claimed invention.

In preferred embodiments, methods for nucleic acid detection utilize a number of different amplification techniques involving copying specific nucleic acid sequences that represent specific regions of interest to an investigator. Common methods of amplification include polymerase chain reaction (PCR), transcription mediated methods of amplification (TMA and cRNA), as well as other enzyme mediated cascades known to one skilled in the art. Alternative methods involve amplification of a detectable moiety, such as using detectable substrates generated via enzymatic substrate conversion, e.g., the exonuclease-based Invader^{®} methodologies (Third Wave Technologies, Inc.) or condensation of signal moieties to a location through the use of branched DNA or multiple branched polymers. Most of these methods can be performed to detect and quantitate the relative levels of one or more nucleic acid sequences from within whole cell lysates.

To use an amplification and labeling technique to detect one or more nucleic acids, it is necessary to introduce the necessary biochemical reagents. Depending on the partitioning method, these reagents can be added to the cell at appropriate concentrations at a number of different processing steps. For example, in the use of mixing methods for the formation of emulsions that contain vesicles, it is preferred to introduce the biochemical reagents to the cells prior to emulsion formation.

The introduction of reagents when using mechanical flow systems or microfluidic methods to generate partitioned aqueous reaction volumes (containing cells) in a liquid flow can occur either prior to or after separation of the cells into individual partitioned aqueous compartments. Preferred methods and timing depend on the mechanics of the system used. For example, microdispensing systems can be used to dispense reagents individually into each well of a multiwell system. Alternatively, it would be operationally simpler to mix the biochemical reagents with the cell population prior to physically separating the cells. Similarly, reagents can be mixed with the cell population prior to introduction into a flow system or injected into the flow stream as some point prior to or post partitioning of the cells in concert with the introduction of the immiscible partitioning fluid.

In some aspects, methods of the invention have their greatest benefit when analyzing a plurality of biomolecules within each cell (e.g., a multiplex methodologies to simultaneously amplify and detect a plurality of nucleic acids in a cell). Generally, such methods can involve the steps of:
(a) compartmentalizing the individual cells or small subsets of cells (e.g., in reaction vesicles or in partitioned aqueous reactions volumes in a liquid flow);
(b) detecting each of the specific biomolecules of interest (e.g., multiple expressed genes) utilizing the biochemical reagents;
(c) linking the detection of the biomolecule to an event on a solid phase component present within the partitioned area (e.g., a bead conjugated to a suitable bait nucleotide sequence);
(d) monitoring (e.g., detecting, measuring, quantitating, real-time monitoring) the reaction or amplification products on the solid phase component utilizing detection methods known in the art (e.g., fluorescence detection); and
(e) correlating the monitored products on the solid phase with the plurality of biomolecules.

One example of this process is illustrated in **FIGS. 1A-1K****.** In step **1A,** a collection of cells in suspension are prepared in a cell mix solution that further comprises reagents to enable the performance of multiplex PCR with a universal priming system. The reagents include (but may not be limited to) a first set of oligonucleotide primers (i.e., primer pairs consisting of forward primers and reverse primers specific for genes of interest), specific enzymes (e.g., one or more polymerases), buffers, and free nucleotides. Also included are small beads, e.g., polymeric beads 2-20 microns (µm) in size, that have, in this instance (but not limited to) covalently linked universal primer sequence that is specifically able to hybridize to the PCR products as generated by the first set of oligonucleotide primers. Alternatively, the beads can contain a second set of gene-specific oligonucleotide probes that are able to specifically hybridize to the PCR products as generated by the first set of oligonucleotide primers.

In the step illustrated in **FIG. 1B****,** the cell-containing solution is first mixed, e.g., via vortexing, in order to suspend and evenly distribute all of the components (i.e., the cells, reagents and beads) throughout the solution. This solution is then combined with a second immiscible solution (e.g. silicone oil), thus forming a two-phase system. The two phases are admixed to form an emulsion wherein the cell mix solution is compartmentalized into a plurality of vesicles containing aqueous reaction cores, where the vesicles contain, on average, one cell and one bead along with the other PCR reagents the cell mix solution.

As shown in **FIG. 1C****,** the emulsion is sealed within a reaction chamber (e.g., an eppendorf tube) and subjected to thermal cycling conditions that promote PCR amplifications. The product of such a reaction will be a specific set of amplicons as targeted by the first set of oligonucleotide primers reflecting the presence and quantity of the different target nucleic acids within the cell, e.g., DNA and/or RNA. These amplicons, in turn, will hybridize to the complementary universal primers that are linked to the beads within each reaction vesicle. This results in the beads coupled to the products of the multiplex PCR **(****FIG. 1D****).** Upon PCR, products as hybridized to the second set of oligonucleotides act as templates for the extension of the second set of oligonucleotides using DNA polymerase.

Following multiplex amplification, hybridization and extension, the beads can be isolated (e.g., by centrifugation) and placed on a surface such as a slide, microchannel plate or the bottom of a microtiter plate well, for analysis. Various means can then be used to detect the PCR products. Most commonly, multiple labeled (e.g. fluorescent) probes can be used to interrogate the extended oligonucleotide products by hybridizing to the beads. In preferred embodiments, the beads can be placed or arrayed in such a manner that the detection apparatus can detect and differentiate a signal that corresponds to a single bead.

In the present example of **FIG. 1 A-K****,** the resulting beads are coupled to the products of the multiplex PCR through amplification using the universal priming sequence on the beads. These beads then isolated from the vesicle emulsion **(FIG. IE),** and prepared for probing by first removing the non-covalently coupled single strand complementary sequence **(****FIG. 1F****).** These beads can now be probed with nucleic acid probes specific for a product of the multiplex PCR reaction, e.g., a probe specific for Gene A **(****FIG. 1G****).** These beads can then be alternately stripped and reprobed for other amplification products generated during the multiplex PCR **(****FIGS. 1H** through **1K****).**

As illustrated in **FIGS. 1A-1K****,** amplification methods can be employed where the amplification products become covalently linked to the beads via a biochemical reaction. In using the example given above, and utilizing methods that employ universal primer strategies (e.g., as described in US Patent No. 6,618,679), the first set of oligonucleotide primers for use in PCR comprise pairs of target specific primers where the primers further comprise one or more universal primer sequences located 5' from the 3' target-specific primer sequences. In these embodiments, the second set of oligonucleotides, that are linked to the solid phase (e.g., the beads) are comprised of one or more universal sequences. These sequences can participate in the PCR amplification reaction, and as a consequence, yield a plurality of PCR products that are linked to the solid phase. When combined with partitioning methods as provided herein for isolating individual or small subsets of cells during the PCR, the amplified DNA sequences linked to the bead will then represent the specific sequences (as targeted by the target specific primers) present within the cell(s), and in some embodiments, also represent the relative amounts of these specific target sequences.

In other embodiments, amplification of an artificial nucleic acid sequence can be coupled to the detection of a non-nucleic acid molecule. In these embodiments, the aqueous reaction solution further comprises one or more ligands that are linked to the artificial target nucleic acid sequences, thereby providing a mechanism for linking nucleic acid amplification (and subsequent detection) to the presence of one or more non-nucleic acid biomolecular targets, e.g., proteins. In these embodiments, a ligand, (e.g., an antibody, antibody fragment, or polypeptide) is linked to an artificial target nucleic acid and is provided in the aqueous reaction volume. In the aqueous phase, the coupled-ligand is allowed to specifically bind to its biomolecular target. Unbound ligand is then removed, e.g., by washing, filtration, selective capture or other comparable means, preferably prior to partitioning of the individual or groups of cells. Amplification of the artificial nucleic acid sequence then proceeds, where the quantity of cell bound artificial target nucleic acid is amplified, linked to the solid phase, detected, and in particular embodiments, the relative amounts of these artificial target sequences quantitated. For example, see US Patent No. 5,635,602. In these particular embodiments, both a cellular target nucleic acid sequence and an artificial target nucleic acid sequence can be simultaneously amplified and detected. Using this technique, it is possible that a polypeptide and a nucleic acid from the same cell can be detected in the same partitioned reaction volume.

### Polymerase Chain Reaction (PCR)

Methods of the invention for detecting nucleic acids most typically use one of the many variant protocols of the polymerase chain reaction (PCR). As used herein, the term "polymerase chain reaction" (PCR) refers to a method for amplification well known in the art for increasing the concentration of a segment of a target polynucleotide in a sample, where the sample can be a single polynucleotide species, or multiple polynucleotides. Generally, the PCR process consists of introducing a molar excess of two or more extendable oligonucleotide primers to a reaction mixture comprising the desired target sequence(s), where the primers are complementary to opposite strands of the double stranded target sequence. The reaction mixture is subjected to a program of thermal cycling in the presence of a DNA polymerase, resulting in the amplification of the desired target sequence flanked by the DNA primers. Reverse transcriptase PCR (RT-PCR) is a PCR reaction that uses RNA template and a reverse transcriptase, or an enzyme having reverse transcriptase activity, to first generate a single stranded DNA molecule prior to the multiple cycles of DNA-dependent DNA polymerase primer elongation. Multiplex PCR refers to PCR reactions that produce more than one amplified product in a single reaction, typically by the inclusion of more than two primers in a single reaction. Methods for a wide variety of PCR applications are widely known in the art, and described in many sources, for example, in Current Protocols in Molecular Biology, Ausubel et al., eds., Current Protocols, a joint venture between Greene Publishing Associates, Inc. and John Wiley & Sons, Inc., (supplemented through 2006).

### Nucleic Acid Amplification and Detection Reagents

Reactions for the amplification of nucleic acids can comprise a wide variety of reagents, and are well known in the art. No attempt is made herein to recite the litany of possible regents that can be included in a nucleic acid amplification reaction. One of skill is well aware of the diversity of protocols and regents that can be used for nucleic acid amplification.

Most all nucleic acid amplification reactions make use of at least one nucleic acid polymerase, this is, an enzyme capable of generating a second nucleic acid molecule using a first nucleic acid molecule as a template. A wide variety of polymerases having different specificities and characteristics are know and are widely available, e.g., commercially available. The invention is not limited to any particular polymerase as a reagent for amplification or detection.

As used herein, the term "DNA-dependent DNA polymerase" refers to a DNA polymerase enzyme that uses deoxyribonucleic acid (DNA) as a template for the synthesis of a complementary and antiparallel DNA strand. Thermostable DNA-dependent DNA polymerases find use in PCR amplification reactions. Suitable reaction conditions (and reaction buffers) for DNA-dependent DNA polymerase enzymes, and indeed any polymerase enzyme, are widely known in the art, and are described in numerous sources (see, e.g., Ausubel et al. (eds.), Current Protocols in Molecular Biology, Vol. 1-4, John Wiley & Sons, Inc., New York [supplemented through 2006]). Reaction buffers for DNA-dependent DNA polymerase enzymes can comprise, for example, free deoxyribonucleotide triphosphates, salts and buffering agents.

As used herein, the term "RNA-dependent DNA polymerase" refers to a DNA polymerase enzyme that uses ribonucleic acid (RNA) as a template for the synthesis of a complementary and antiparallel DNA strand. The process of generating a DNA copy of an RNA molecule is commonly termed "reverse transcription," or "RT," and the enzyme that accomplishes that is a "reverse transcriptase." Some naturally-occurring and mutated DNA polymerases also possess reverse transcription activity.

As used herein, the term "thermostable," as applied to an enzyme, refers to an enzyme that retains its biological activity at elevated temperatures (e.g., at 55°C or higher), or retains its biological activity following repeated cycles of heating and cooling. Thermostable nucleic acid polymerases find particular use as reagents in PCR amplification reactions.

Nucleic acid amplification generally uses (with exceptions) at least one polynucleotide primer to initiate nucleotide polymerization from a nucleic acid template, thereby generating a complementary molecule. Any primer that is complementarity to and has specificity for any particular nucleotide sequence can find use with the invention, e.g., as an aqueous phase reagent for detecting a nucleic acid biomolecule.

As used herein, the term "primer" refers to an enzymatically extendable oligonucleotide, generally with a defined sequence that is designed to hybridize in an antiparallel manner with a complementary, primer-specific portion of a target sequence. A primer can initiate the polymerization of nucleotides in a template-dependent manner to yield a polynucleotide that is complementary to the target polynucleotide. The extension of a primer annealed to a target uses a suitable DNA or RNA polymerase in suitable reaction conditions. A primer can comprise nucleotide subunits that are naturally occurring or, advantageously, can incorporate unnatural features. The unnatural subunits can advantageously incorporate unnatural backbone structures and/or unnatural bases. One of skill in the art knows well that polymerization reaction conditions and reagents are well established in the art, and are described in a variety of sources.

A primer nucleic acid does not need to have 100% complementarity with its template target sequence for primer elongation to occur; primers with less than 100% complementarity can be sufficient for hybridization and polymerase elongation to occur. Optionally, a primer nucleic acid can be labeled, if desired. The label used on a primer can be any suitable label, and can be detected by, for example, by spectroscopic, photochemical, biochemical, immunochemical, chemical, or other detection means.

Primers used in the invention are generally amplification primers. As used herein, the expression "amplification primer" refers to a primer that is generally (but not required to be) in molar excess relative to its target polynucleotide sequence, and primes template-dependent enzymatic DNA synthesis and amplification of the target sequence (and sequence downstream from the site of hybridization) to yield a single-stranded amplicon.

The invention also includes the use of primers pairs. As used herein, the expressions "primer pair" or "amplification primer pair" refer to a set of two primers that are generally in molar excess relative to their target polynucleotide sequence, and together prime template-dependent enzymatic DNA synthesis and amplification of the target sequence to yield a double-stranded amplicon. The expression "primer pairs" also incorporates the concept of using three or more primers. For example, a single reaction can include one downstream primer and two upstream primers, thereby generating two overlapping amplicons of differing length.

Amplification reactions generally generate an amplicon. As used herein, the term "amplicon" refers to a polynucleotide molecule (or collectively the plurality of molecules) produced following the amplification of a particular target nucleic acid. The amplification method used to generate the amplicon can be any suitable method, most typically, for example, by using a PCR methodology. An amplicon is typically, but not exclusively, a DNA amplicon. An amplicon can be single-stranded or double-stranded, or in a mixture thereof in any concentration or ratio.

Reagents finding use with the invention also includes universal primers and universal priming sequences. As used herein, the term, "universal primer" refers to a primer comprising a nucleotide sequence that is complementary to a universal priming sequence. A universal priming sequence can be advantageously incorporated into an amplification product in order to re-amplify one or any number of multiple amplification reactions, for example, in the reaction products of a multiplex PCR reaction. The term "semi-uni versal primer" refers to a primer that is capable of hybridizing with more than one (e.g., a subset), but not all, of the potential target sequences in a multiplexed reaction. The terms "universal sequence," "universal priming sequence" or "universal primer sequence" or the like refer to a sequence contained in a plurality of primers, where the universal primer contains sequence that is complementary to a target universal priming sequence.

### Nucleic Acid Probes

In its most general sense, a probe can be any moiety that has an affinity for a target biomolecule. In some aspects, the methods of the invention can utilize a probe for detecting a nucleic acid biomolecule target of interest. As used herein in reference to polynucleotides, the term "probe" refers to any polynucleotide that is capable of hybridizing to a target nucleic acid of interest. Thus, probes can find use as reagents for the detection of nucleic acid biomolecule targets. As used herein, a "nucleic acid probe" is a probe that detects a nucleic acid biomolecule. Generally (but not exclusively), a nucleic acid probe will comprise a polynucleotide, although it is not intended that the invention be limited to nucleic acid probes comprising polynucleotides. Indeed, it is known that antibodies can be generated that have specificity for various nucleotide sequences.

In the case of polynucleotide probes (including polymerase primers), the probe is able to hybridize in solution with its target by the phenomenon of base pairing to form a hybridization complex. The probe and the target can be 100% complementary or in some cases less than 100% complementary. Nucleic acids generally hybridize according the Watson-Crick model for antiparallel base pairing, and stability (or relative instability) of the hybridization complex thus formed is the result of well characterized physico-chemical forces, including hydrogen bonding, solvent exclusion and base stacking. Extensive discussion on the theory and practical application of nucleic acid hybridizations are widely available and are well known to one of skill in the art.

It is not intended that the present invention be limited to any particular polynucleotide probe label or probe detection system. The source of the polynucleotide used in the probe is not limited, and can be produced synthetically in a non-enzymatic system, or can be a polynucleotide (or a portion of a polynucleotide) that is produced using a biological (*e.g.,* enzymatic) system (*e.g.,* in a bacterial cell).

In some aspects, the terms "target", "target polynucleotide", "target sequence" and the like refer to a specific polynucleotide sequence that is the subject of hybridization with a complementary polynucleotide probe, e.g., a labelled probe or a DNA polymerase primer. The complex formed as a result of the annealing of a polynucleotide probe with its nucleic acid target is termed a hybridization complex. The hybridization complex can form in solution (and is therefore soluble), or one or more component of the hybridization complex can be affixed to a solid phase (*e.g.,* to a dot blot, affixed to a bead system to facilitate removal or isolation of target hybridization complexes, or in a microarray). For example, as described in the example of **FIGS. 1A-K****,** the universal primer in a multiplex PCR reaction can be affixed to a solid phase.

Typically, but not exclusively, a probe is associated with a suitable label or reporter moiety so that the probe (and therefore its target) can be detected, visualized, measured and/or quantitated following hybridization (and optional washing to remove unbound probe). Detection systems for labelled probes include, but are not limited to, the detection of fluorescence, fluorescence quenching (e.g., when using a FRET pair detection system), enzymatic activity, absorbance, molecular mass, radioactivity, luminescence or binding properties that permit specific binding of the reporter (*e.g.,* where the reporter is an antibody).

### Real Time Amplicon Detection and 5'-Nuclease (TaqMan) Assay -

As used herein, the expression "real-time detection of amplicon accumulation" refers to the detection of, and typically the quantitation thereof, of a specific amplicon or amplicons, as the amplicon(s) is/are being produced (typically by PCR) without the need for a detection or quantitation step following the completion of the amplification. The terms "real-time PCR" or "kinetic PCR" refer to real-time detection and/or quantitation of amplicon generated in a PCR. Real-time detection may be performed wherein the detection occurs in real time as the reaction proceeds or it may involve detection at one or more endpoints wherein the reaction is paused or stopped and the products of the reaction are detected, typically using fluorescence methods.

A common method for real-time detection of amplicon accumulation is by a 5'-nuclease assay, also termed a fluorogenic 5'-nuclease assay, *e.g.,* a TaqMan analysis; *see,* Holland et al., Proc. Natl. Acad. Sci. USA 88:7276-7280 (1991); and Heid et al., Genome Research 6:986-994 (1996). In the TaqMan PCR procedure, two oligonucleotide primers are used to generate an amplicon specific to the PCR reaction. A third oligonucleotide (the TaqMan probe) is designed to hybridize with a nucleotide sequence in the amplicon located between the two PCR primers. The probe may have a structure that is non-extendible by the DNA polymerase used in the PCR reaction, and is typically (but not necessarily) colabeled with a fluorescent reporter dye and a quencher moiety in close proximity to one another. The emission from the reporter dye is quenched by the quenching moiety when the fluor and quencher are in close proximity, as they are on the probe. In some cases, the probe may be labeled with only a fluorescent reporter dye or another detectable moiety.

The TaqMan PCR reaction uses a thermostable DNA-dependent DNA polymerase that possesses a 5'-3' nuclease activity. During the PCR amplification reaction, the 5'-3' nuclease activity of the DNA polymerase cleaves the labeled probe that is hybridized to the amplicon in a template-dependent manner. The resultant probe fragments dissociate from the primer/template complex, and the reporter dye is then free from the quenching effect of the quencher moiety. Approximately one molecule of reporter dye is liberated for each new amplicon molecule synthesized, and detection of the unquenched reporter dye provides the basis for quantitative interpretation of the data, such that the amount of released fluorescent reporter dye is directly proportional to the amount of amplicon template.

One measure of the TaqMan assay data is typically expressed as the threshold cycle (C_{T}). Fluorescence levels are recorded during each PCR cycle and are proportional to the amount of product amplified to that point in the amplification reaction. The PCR cycle when the fluorescence signal is first recorded as statistically significant, or where the fluorescence signal is above some other arbitrary level (*e.g.,* the arbitrary fluorescence level, or AFL), is the threshold cycle (C_{T}).

Protocols and reagents for 5'-nuclease assays are well known to one of skill in the art, and are described in various sources. For example, 5'-nuclease reactions and probes are described in U.S. Pat. No. 6,214,979, entitled "HOMOGENEOUS ASSAY SYSTEM," issued April 10, 2001 to Gelfand et al.; U.S. Pat. No. 5,804,375, entitled "REACTION MIXTURES FOR DETECTION OF TARGET NUCLEIC ACIDS," issued September 8, 1998 to Gelfand *et al.;* U.S. Pat. No. 5,487,972, entitled "NUCLEIC ACID DETECTION BY THE 5'-3' EXONUCLEASE ACTIVITY OF POLYMERASES ACTING ON ADJACENTLY HYBRIDIZED OLIGONUCLEOTIDES," issued January 30, 1996 to Gelfand *et al.;* and U.S. Pat. No. 5,210,015, entitled "HOMOGENEOUS ASSAY SYSTEM USING THE NUCLEASE ACTIVITY OF A NUCLEIC ACID POLYMERASE," issued May 11, 1993 to Gelfand et al., all of which are incorporated by reference.

Variations in methodologies for real-time amplicon detection are also known, and in particular, where the 5'-nuclease probe is replaced by double-stranded DNA intercalating dye resulting in fluorescence that is dependent on the amount of double-stranded amplicon that is present in the amplification reaction. See, for example, U.S. Pat. No. 6,171,785, entitled "METHODS AND DEVICES FOR HEMOGENEOUS NUCLEIC ACID AMPLIFICATION AND DETECTOR," issued January 9, 2001 to Higuchi; and U.S. Pat. No. 5,994,056, entitled "HOMOGENEOUS METHODS FOR NUCLEIC ACID AMPLIFICATION AND DETECTION," issued November 30, 1999 to Higuchi, each of which are incorporated by reference.

TaqMan^{®} PCR can be performed using commercially available kits and equipment, such as, for example, ABI PRISM^{®} 7700 Sequence Detection System (Applied Biosystems, Foster City, Calif.), or LightCycler^{®} (Roche Applied Sciences, Mannheim, Germany). In a preferred embodiment, the 5' nuclease assay procedure is run on a real-time quantitative PCR device such as the ABI PRISM^{®} 7700 Sequence Detection System. The system consists of a thermocycler, laser, charge-coupled device (CCD), camera and computer. The system amplifies samples in a 96-well microtiter plate format on a thermocycler. During amplification, laser-induced fluorescent signal is collected in real-time through fiber optics cables for all 96 wells, and detected at the CCD camera. The system includes software for running the instrument and for analyzing the data.

### LABELS

As used herein, the terms "label" or "reporter," in their broadest sense, refer to any moiety or property that is detectable, or allows the detection of, that which is associated with it. For example, a polynucleotide or polypeptide that comprises a label, or to which a label is associated, is detectable (and in some aspects is referred to as a probe). Ideally, a labeled polynucleotide permits the detection of a hybridization complex that comprises the polynucleotide. In some aspects, *e.g.,* a label is attached (covalently or non-covalently) to a polynucleotide. In various aspects, a label can, alternatively or in combination: (i) provide a detectable signal; (ii) interact with a second label to modify the detectable signal provided by the second label, *e.g.,* FRET; (iii) stabilize hybridization, *e.g.,* duplex formation; (iv) confer a capture function, *e.g.,* hydrophobic affinity, antibody/antigen, ionic complexation, or (v) change a physical property, such as electrophoretic mobility, hydrophobicity, hydrophilicity, solubility, or chromatographic behavior. Labels vary widely in their structures and their mechanisms of action.

Labeling can be accomplished using any one of a large number of known techniques employing known labels, linkages, linking groups, reagents, reaction conditions, and analysis and purification methods. Labels include light-emitting or light-absorbing compounds which generate or quench a detectable fluorescent, chemiluminescent, or bioluminescent signal (Kricka, L. in Nonisotopic DNA Probe Techniques (1992), Academic Press, San Diego, pp. 3-28).

Examples of labels include, but are not limited to, fluorescent labels (including, e.g., quenchers or absorbers), non-fluorescent labels, colorimetric labels, chemiluminescent labels, bioluminescent labels, radioactive labels, mass-modifying groups, antibodies, antigens, biotin, haptens, enzymes (including, e.g., peroxidase, phosphatase, etc.), and the like. To further illustrate, fluorescent labels may include dyes that are negatively charged, such as dyes of the fluorescein family, or dyes that are neutral in charge, such as dyes of the rhodamine family, or dyes that are positively charged, such as dyes of the cyanine family.

Essentially any labeling moiety is optionally utilized to label a probe and/or primer by techniques well known in the art. In some embodiments, for example, labels comprise a fluorescent dye (e.g., a rhodamine dye, e.g., Texas Red, R6G, R110, TAMRA, ROX, etc., see U.S. Pat. Nos. 5,366,860; 5,847,162; 5,936,087; 6,051,719; 6,191,278), or a fluorescein dye (e.g., JOE, VIC, TET, HEX, FAM, NAN and ZOE, etc.; 6-carboxyfluorescein; 2',4',1,4,-tetrachlorofluorescein; and 2',4',5',7', 1,4-hexachlorofluorescein; see U.S. Pat. Nos. 5,188,934; 6,008,379; 6,020,481). FAM, HEX, TET, JOE, NAN, ZOE, ROX, R110, R6G, and TAMRA are commercially available from, *e.g.,* Perkin-Elmer, Inc. (Wellesley, MA, USA), and Texas Red is commercially available from, *e.g.,* Molecular Probes, Inc. (Eugene, OR). Labels also include benzophenoxazines (U.S. Pat. No. 6,140,500), a halofluorescein dye, a cyanine dye (e.g., CY2, CY3, CY3.5, CY5, CY5.5, CY7, etc., commercially available from, *e.g.,* Amersham Biosciences Corp. (Piscataway, NJ, USA), see Published International Application No. WO 97/45539 by Kubista), a BODIPY® dye (e.g., FL, 530/550, TR, TMR, etc.), an ALEXA FLUOR® dye (e.g., 488, 532, 546, 568, 594, 555, 653, 647, 660, 680, etc.), a dichlororhodamine dye, an energy transfer dye (e.g., BIGDYE™ 1 dyes, BIGDYE™ v 2 dyes, BIGDYE™ v 3 dyes, etc.), Lucifer dyes (e.g., Lucifer yellow, etc.), CASCADE BLUE®, Oregon Green, and the like. Additional examples of fluorescent dyes are provided in, e.g., Haugland, Molecular Probes Handbook of Fluorescent Probes and Research Products, Ninth Ed. (2003) and the updates thereto, which are each incorporated by reference. Fluorescent dyes are generally readily available from various commercial suppliers including, e.g., Molecular Probes, Inc. (Eugene, OR), Amersham Biosciences Corp. (Piscataway, NJ), Applied Biosystems (Foster City, CA), etc.

FRET labeling techniques are commonly used in both real-time amplicon quantitation and for monitoring nucleic acid probe hybridization. In some preferred embodiments, FRET label systems are used with the probes of the invention. It is not intended that the invention be limited to any particular FRET pair system. One of skill in the art recognizes the wide range of FRET labels that can be used with the probes of the invention. Fluorescent energy-transfer dye pairs of donors and acceptors include, e.g., U.S. Pat. Nos. 5,863,727; 5,800,996; 5,945,526, as well as any other fluorescent label capable of generating a detectable signal.

Whether a fluorescent dye is a label or a quencher is generally defined by its excitation and emission spectra, and the fluorescent dye with which it is paired. Fluorescent molecules commonly used as quencher moieties in probes and primers include, e.g., fluorescein, FAM, JOE, rhodamine, R6G, TAMRA, ROX, DABCYL, and EDANS. Many of these compounds are available from the commercial suppliers referred to above. Examples of non-fluorescent or dark quenchers that dissipate energy absorbed from a fluorescent dye include the Black Hole Quenchers™ or BHQ™, which are commercially available from Biosearch Technologies, Inc. (Novato, CA, USA). Other quenchers include Iowa Black quenchers (*e.g.,* Iowa Black FQ™ and Iowa Black RQ™) and Eclipse^{®} Dark Quenchers (Epoch Biosciences, Inc, Bothell, WA). Anchor probe FRET systems are also known in the art, and are described, for example, in Schroter et al., (2002) Jour. Clin. Microbiol., 40(6):2046-2050.

Other labels include, e.g., biotin, weakly fluorescent labels (Yin et al. (2003) Appl Environ Microbiol. 69(7):3938, Babendure et al. (2003) Anal. Biochem. 317(1):1, and Jankowiak et al. (2003) Chem Res Toxicol. 16(3):304), non-fluorescent labels, colorimetric labels, chemiluminescent labels (Wilson et al. (2003) Analyst. 128(5):480 and Roda et al. (2003) Luminescence 18(2):72), Raman labels, electrochemical labels, bioluminescent labels (Kitayama et al. (2003) Photochem Photobiol. 77(3):333, Arakawa et al. (2003) Anal. Biochem. 314(2):206, and Maeda (2003) J. Pharm. Biomed. Anal. 30(6):1725), and an alpha-methyl-PEG labeling reagent as described in, e.g., U.S. Patent Application Serial No. 10/719,257, filed on Nov. 21, 2003, which references are each incorporated by reference.

Another class of labels are hybridization-stabilizing moieties which serve to enhance, stabilize, or influence hybridization of duplexes, e.g., intercalators, minor-groove binders, and cross-linking functional groups (Blackbum, G. and Gait, M. Eds. "DNA and RNA structure" in Nucleic Acids in Chemistry and Biology, 2.sup.nd Edition, (1996) Oxford University Press, pp. 15-81).

Yet another class of labels effect the separation or immobilization of a molecule by specific or non-specific capture, for example biotin, digoxigenin, and other haptens (Andrus, "Chemical methods for 5' non-isotopic labelling of PCR probes and primers" (1995) in PCR 2: A Practical Approach, Oxford University Press, Oxford, pp. 39-54).

Non-radioactive labelling methods, techniques, and reagents are reviewed in: Non-Radioactive Labelling, A Practical Introduction, Garman, A. J. (1997) Academic Press, San Diego.

In some embodiments, the two types of probes that are used in the invention, namely the HCV typing probe and the HCV quantitation probe, use the same label, for example, a fluorescein label. This provides various advantages, as the HCV quantitation assay and the HCV typing assay can be read in the same detector, e.g., a fluorescence spectrophotometer. However, it is not intended that the invention be limited to that type of configuration. For example, the two different probes can use two different fluorescent labels that have non-identical emission spectra (or even different labelling systems, such as fluorescent and non-fluorescent label systems).

### FLUORESCENT RESONANCE ENERGY TRANSFER (FRET)

As used herein, the term "FRET" (fluorescent resonance energy transfer) and equivalent terms refers generally to a dynamic distance-dependent interaction between electron states of two dye molecules in which energy is transferred from a donor molecule to an acceptor molecule without emission of a photon from the donor molecule. The efficiency of FRET is dependent on the inverse of the intermolecular separation between the dyes, making it useful over distances comparable with the dimensions of biological macromolecules. Generally, FRET allows the imaging, kinetic analysis and/or quantitation of colocalizing molecules or conformational changes in a single molecule with spatial resolution beyond the limits of conventional optical microscopy. In general, FRET requires, (a) the donor and acceptor molecules must be in close proximity (typically, e.g., 10-100 Å), (b) the absorption spectrum of the acceptor must overlap the fluorescence emission spectrum of the donor, and (c) the donor and acceptor transition dipole orientations must be approximately parallel.

In most FRET applications, the donor and acceptor dyes are different, in which case FRET can be detected by the appearance of sensitized fluorescence of the acceptor or by quenching of donor fluorescence. In some cases, the donor and acceptor are the same, and FRET can be detected by the resulting fluorescence depolarization. Use of a single donor/acceptor molecule in a FRET system is described, for example, in Published US Patent Application No. 2004/0096926, by Packard and Komoriya, published May 20, 2004, entitled "COMPOSITIONS FOR THE DETECTION OF ENZYME ACTIVITY IN BIOLOGICAL SAMPLES AND METHODS OF USE THEREOF," which is hereby incorporated by reference.

FRET has become an important technique for investigating a variety of biological phenomena that are characterized by changes in molecular proximity. FRET techniques are now pervasive in many biological laboratories, and have been adapted for use in a variety of biological systems, including but not limited to, detection of nucleic acid hybridization, real-time PCR assays and SNP detection, structure and conformation of proteins, spatial distribution and assembly of protein complexes, receptor/ligand interactions, immunoassays, probing interactions of single molecules, structure and conformation of nucleic acids, primer-extension assays for detecting mutations, automated DNA sequencing, distribution and transport of lipids, membrane fusion assays (lipid-mixing assays of membrane fusion), membrane potential sensing, fluorogenic protease substrates, and indicators for cyclic AMP and zinc.

As used herein, the term "FRET donor" refers typically to a moiety that produces a detectable emission of radiation, e.g., fluorescent or luminescent radiation, that can be transferred to a suitable FRET acceptor in sufficient proximity. The expression "FRET donor" can be used interchangeably with "FRET label" or "FRET label moiety."

As used herein, the terms "quencher," "quencher moiety," "acceptor," "acceptor moiety" and "light emission modifier" and similar and equivalent terms refer generally to a moiety that reduces and/or is capable of reducing the detectable emission of radiation, for example but not limited to, fluorescent or luminescent radiation, from a source that would otherwise have emitted this radiation. Generally, a quencher refers to any moiety that is capable of reducing light emission. The degree of quenching is not limited, *per se,* except that a quenching effect should minimally be detectable by whatever detection instrumentation is used. In some aspects, a quencher reduces the detectable radiation emitted by the source by at least 50%, alternatively by at least 80%, and alternatively and most preferably by at least 90%.

In some embodiments, the quencher results in a reduction in the fluorescence emission from a donor, and thus the donor/quencher forms a FRET pair, and the quencher is termed a "FRET quencher," or "FRET acceptor," and the donor is a "FRET donor."

It is not intended that that the term "quencher" be limited to FRET quenchers. For example, quenching can involve any type of energy transfer, including but not limited to, photoelectron transfer, proton coupled electron transfer, dimer formation between closely situated fluorophores, transient excited state interactions, collisional quenching, or formation of non-fluorescent ground state species. In some embodiments, a quencher refers to a molecule that is capable of reducing light emission. There is no requirement for a spectral overlap between the fluorophore and the quencher. As used herein, "quenching" includes any type of quenching, including dynamic (Förster-Dexter energy transfer, etc.), and static (ground state complex). Alternatively still, a quencher can dissipate the energy absorbed from a fluorescent dye in a form other than light, *e.g.,* as heat.

In some embodiments, some quenchers can re-emit the energy absorbed from a FRET donor at a wavelength or using a signal type that is distinguishable from the FRET donor emission, and at a wavelength or signal type that is characteristic for that quencher, and thus, in this respect, a quencher can also be a "label."

For general discussion on the use of flourescence probe systems, see, for example, Principles of Fluorescence Spectroscopy, by Joseph R. Lakowicz, Plenum Publishing Corporation, 2nd edition (July 1, 1999) and Handbook of Fluorescent Probes and Research Chemicals, by Richard P. Haugland, published by Molecular Probes, 6th edition (1996).

As used herein, the expression "FRET pair" and similar and equivalent terms refers to the pairing of a FRET donor moiety and a FRET acceptor moiety, such that FRET is observed when the donor and the acceptor are within suitable proximity to each other. Generally, but not exclusively, the donor moiety and the acceptor moiety are attached to various molecules of interest (*e.g.,* polynucleotide probes).

A wide variety of dyes, fluors, quenchers, and fluorescent proteins, along with other reagents and detection/imaging instrumentation have been developed for use in FRET analysis and are widely commercially available. One of skill in the art recognizes appropriate FRET protocols, reagents and instrumentation to use for any particular analysis.

Molecules commonly used in FRET include, for example but not limited to, fluorescein, FAM, JOE, rhodamine, R6G, TAMRA, ROX, DABCYL, and EDANS. Whether a fluorescent dye is a label or a quencher is defined by its excitation and emission spectra, and also by the fluorescent dye with which it is paired. For example, FAM is most efficiently excited by light with a wavelength of 488 nm, and emits light with a spectrum of 500 to 650 nm, and an emission maximum of 525 nm. FAM is a suitable donor label for use with, e.g., TAMRA as a quencher, which has at its excitation maximum 514 nm. Examples of non-fluorescent or dark quenchers that dissipate energy absorbed from a fluorescent dye include the Black Hole Quenchers™ marketed by Biosearch Technologies, Inc. (Novato, CA, USA). The Black Hole Quenchers™ are structures comprising at least three radicals selected from substituted or unsubstituted aryl or heteroaryl compounds, or combinations thereof, wherein at least two of the residues are linked via an exocyclic diazo bond (see, e.g., International Publication No. WO 01/86001, entitled "DARK QUENCHERS FOR DONOR-ACCEPTOR ENERGY TRANSFER," published November 15, 2001 by Cook et al., which is incorporated by reference). Examples of quenchers are also provided in, e.g., U.S. Patent No. 6,465,175, entitled "OLIGONUCLEOTIDE PROBES BEARING QUENCHABLE FLUORESCENT LABELS, AND METHODS OF USE THEREOF," which issued October 15, 2002 to Horn et al., which is incorporated by reference.

### METHODOLOGIES FOR ASSAYING PROTEINS

The invention provides compositions and methods for the assay of proteins in highly-partitioned, high-throughput systems. The invention is readily adapted for use with a number of different enzyme catalyzed signaling systems as commonly used in the analysis of proteins. For example, antibodies are often coupled with enzymes, e.g., horseradish peroxidase or alkaline phosphatase, which are capable of generating fluorogenic or chromogenic reaction products from suitable substrates. By using the partitioning techniques of the invention, one can utilize standard immunoassay techniques to examine different biomolecules in a large number of samples (e.g., single cells or small groups of cells). In some embodiments, the biomolecules are protein and/or protein modifications.

For example, solely for the purpose of illustration, a plurality of individual cells are analyzed for expression of a particular cell surface protein of interest. A standard approach is to obtain an antibody to the protein of interest, fluorescently label this antibody, mix the labeled antibody with the cell population under conditions where the antibody binds to the protein of interest, and then to introduce these now labeled cells into a flow cytometry system for detection. Such systems generally work with reasonable efficiency, but are often limited in sensitivity since there is no convenient system for amplifying the signal associated with the detection of the protein on a single cell, i.e., only a limited amount of label can be directly linked to the detecting antibody. The present invention provides an alternative where the antibody is not directly labeled with a fluorogenic compound, but rather, like a standard immunoassay, it is linked to an enzyme capable of generating signal from a enzyme substrate that becomes fluorescent or otherwise optically detectible.

Solely for the purpose of illustrating the invention, one embodiment of a protein detection system of the invention is provide in **FIGS. 2A-2C****.** It is not intend that the invention be limited to this one embodiment or any other embodiment described herein, as alternative systems commonly used in the art also find use with the invention.

Procedurally, the methods of the invention can involve, for example, obtaining a cell population of interest where the cells will be analyzed for expression of one or more proteins of interest and where the investigator is interested in measuring the relative quantities of these proteins for each cell individually. As with standard flow cytometry methods, the cell population is mixed with one or more antibodies (in some embodiments, termed a primary antibody) directed toward the one or more proteins of interest under conditions promoting the binding of the antibodies to the proteins (see **FIG. 2A****).** This process effectively links the antibodies to the cells. In some aspects, the number of antibodies bound reflects the relative quantities of the one or more proteins of interest. In some aspects, the protein of interest is a cell surface protein, such as a receptor, and the bound antibody thus binds the surface of the cell. The cells and their linked antibodies are then washed to remove unbound antibody **(****FIG. 2A****).**

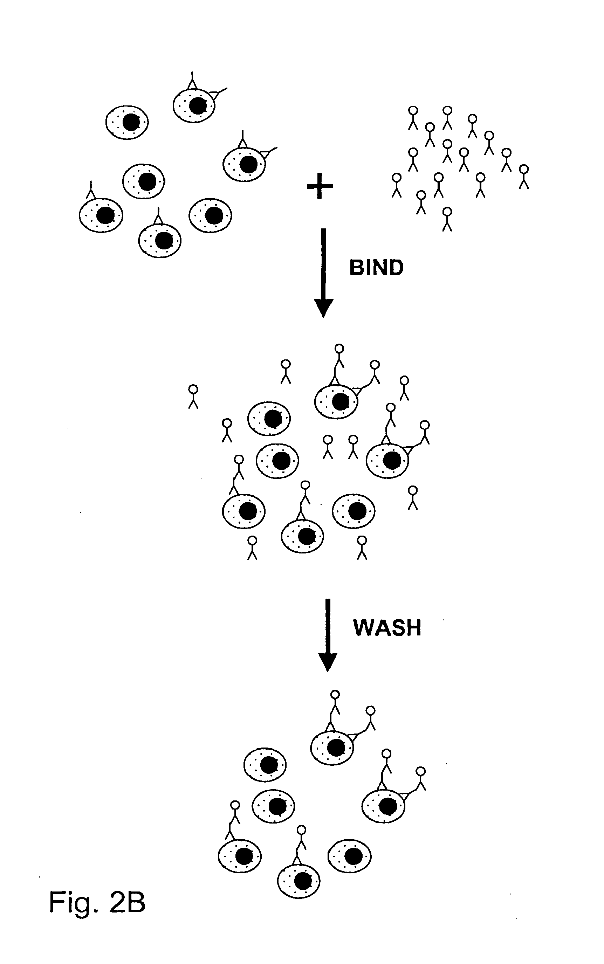

In the next step (see **FIG. 2B****),** the cells with their bound antibodies are exposed to a secondary reporter antibody that has specificity for the first antibody. This second antibody is most typically coupled to an enzyme reporter that permits colorimetric of fluorimetric signal generation upon catalysis of an appropriate chromogenic or fluorogenic enzyme substrate. Such enzyme systems are common in the art, and all find use with the present invention. Binding of the secondary antibody is followed by a wash step that removes unbound secondary antibody **(****FIG. 2B****).** In some embodiments of the invention, the enzyme reporter can be directly linked to the primary antibody or antibodies.

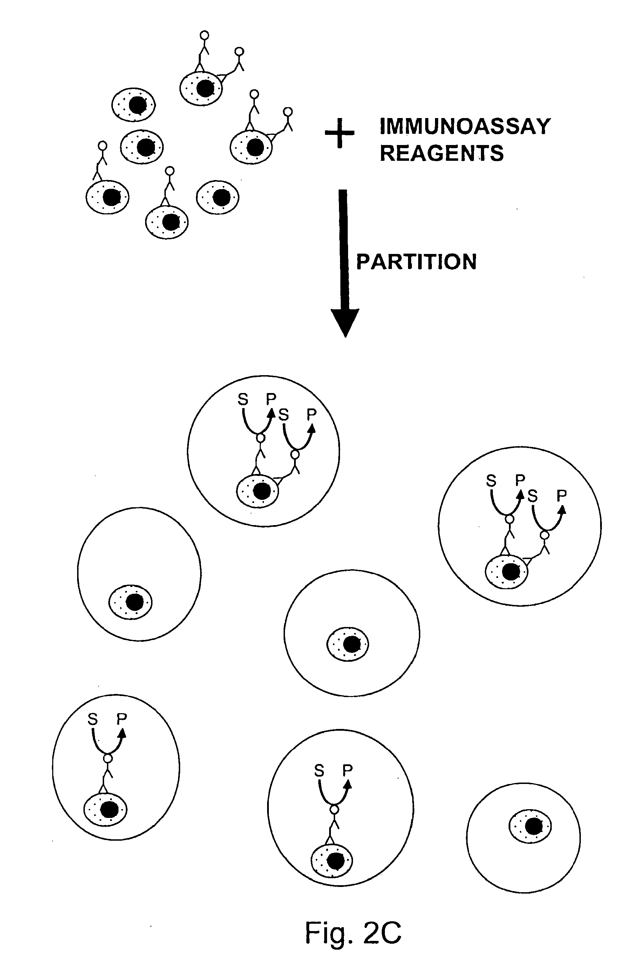

In the next step (see **FIG. 2C****),** the cells with their bound primary and secondary antibodies are compartmentalized using a partitioning method of the invention (e.g., formation of vesicles in an emulsion or creating partitioned aqueous reaction volumes in a liquid flow system). Furthermore, the necessary biochemical reagents to conduct the assay are introduced into the aqueous compartments. The introduction of the biochemical assay reagents to the aqueous reaction volumes can involve addition of reagents either prior to or post partitioning. Most significantly, the principal reagent to be added at this step in this particular embodiment is the chromogenic or fluorogenic enzyme substrate (termed "S" in FIG. 2C). Addition of the substrate will result in generation of a detectable reaction product ("P") only when the reporter enzyme on the secondary antibody is present.

Following compartmentalization of the aqueous phase reaction volumes comprising the individual cells and assay reagents, the partitioned volumes in the flow channel, or the vesicles in an emulsion, are placed into a detection system wherein each of the cells can be examined individually. Within each confined aqueous compartment, the presence of one or more enzyme-coupled antibodies will induce the turnover of the enzyme substrate reagents generating a detectible signal (e.g., color or fluorescence that can be observed at specific time points or in real time, and specific wavelengths and measures of polarity) that increases in quantity as the catalytic reaction proceeds.

Depending on the specific method of detection, there can be a greater or lesser need to control the size and volume of the vesicle or partitioned reaction volume. Specific enzymatic signaling processes proceed at a linear rate as long as there is a relative excess of reagents, and as the reagents are consumed the reaction will slow. Nucleic acids amplification exhibits this phenomenon with the amplification proceeding in a linear or simple logarithmic fashion during the central phase of amplification and then reducing in reaction rate during the final plateau phase. Depending on the size and volume of the reaction, these different phases can change in length of time. To control for this issue, one can implement methods for the control of vesicle or reaction chamber size and volume to provide a relatively equivalent or homogenous environment, provide a compensating factor for the normalization of signals based on variations in vesicle/reaction chamber size and volume, or utilize endogenous or incorporated standards within each vesicle/reaction chamber to provide relative quantitation.

It is not intended that the proteins detected by this or any other method be limited to proteins expressed on the cell surface. Known methods for detection of proteins that are not localized to the outer cell membrane are readily adapted for use with the present invention.

### Antibody Use and Production

Antibodies find use in a variety of methods of the invention for detection of polypeptide biomolecules associated with cells. Methods wherein antibodies find use with the invention include any standard immunoassay protocol. These include but are not limited to immunoassays such as Western blotting, enzyme-linked immunosorbent assays (ELISAs), radioimmunoassays (RIAs), immunofluorescence assays (IFAs), immunoprecipitation, immunohistochemistry and immunoaffinity purification. All of these methods are well known in the art (See, e.g., Harlow and Lane (eds.), Antibodies: A Laboratory Manual, Cold Spring Harbor Laboratory Press [1988]; Ausubel et al. (eds.), Current Protocols in Molecular Biology, Vol. 1-4, John Wiley & Sons, Inc., New York [1994]).

In various embodiments, antibodies are used in the methods of the invention for the specific detection of polypeptides. Antibodies finding use with the invention include polyclonal antisera comprising a heterogeneous pool of antibodies. In some aspects, the antibodies used in methods of the invention are monoclonal, and are derived from a single B-cell clone. Furthermore, antibodies finding use with the invention can be engineered or are artificial, for example, can be single chain antibodies. The invention also encompasses the use of fragments of immunoglobulins, including Fab fragments.

It is not intended that the invention be limited to the immunological methodologies and reagents specifically recited herein. One of skill in the art is familiar with the wide variety of methods and regents for the production and manipulation of antisera and antibodies. See, for example, Harlow and Lane (1988) Antibodies, A Laboratory Manual, Cold Spring Harbor Publications, New York, for descriptions of standard antibody generation, immunoassay formats and conditions that can be used to determine specific immunoreactivity. Details regarding standard immunobiology methodologies (e.g., production of proteins, antibodies, antisera, etc.) are widely available and are well known to one of skill in the art.

In order to produce antisera, e.g., for use in detecting a polypeptide biomolecule in a cell analysis method of the invention, one or more immunogenic polypeptides corresponding to the biomolecule is produced and purified. For example, recombinant protein or native protein can be used. In some embodiments, inbred strains of mice are immunized with the immunogenic protein(s). Inbred mice specifically for this purpose are typically used because results are more reproducible due to the minimal genetic variability between the mice. A standard mouse immunization protocol can be used, as well known to one of skill in the art (*see, e.g.,* Harlow and Lane (1988) Antibodies, A Laboratory Manual, Cold Spring Harbor Publications, New York). In other aspects, other types of host animals are immunized, for example, rats, rabbits, goats or chickens.

Immunization of the host animal is most typically in combination with a suitable adjuvant, which are widely known to one of skill in the art. As used herein, an adjuvant is any substance co-injected with an antigen (usually mixed with them but alternatively can be given before or after administration of the antigens) which non-specifically enhances the immune response to the antigens. Dosage of the antigen given to the animal can vary. In various embodiments, antigen is injected via intravenous, subcutaneous or intraperitoneal routes, and it is not intended that the interval of immunization, boosts or serum collection be limited to specific time points.

When polyclonal antisera is desired, it is not intended that the present invention be limited to any particular method for the production, collection technique or collection schedule of polyclonal antisera. One of skill in the art recognizes that there exist numerous protocols and reagents that find use with the present invention to produce antisera.

When monoclonal antibodies are desired, any technique that provides for the production of monoclonal antibody by continuous cell lines in culture can be used. These methods include but are not limited to the hybridoma technique originally developed by Kohler and Milstein (Kohler and Milstein, Nature 256:495-497 [1975]), as well as the trioma technique, the human B-cell hybridoma technique (See e.g., Kozbor et al. Immunol. Today 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]). See also Harlow and Lane (eds.), Antibodies: A Laboratory Manual, Cold Spring Harbor Laboratory Press (1988); Harlow and Lane (eds.), Using Antibodies: A Laboratory Manual, Cold Spring Harbor Laboratory Press (1999); Coligan et al. (eds.), Current Protocols in Immunology, Vol. 1-4, John Wiley & Sons, Inc., New York (1991). It is not intended that the present invention be limited to the use of any particular protocol, as numerous protocols for generating monoclonal antibody-producing cells are known, and find use in the present invention.

Following the production of polyclonal antisera or monoclonal antibodies, the antibodies can be optionally purified using any suitable method, including but not limited to Protein A/Protein G affinity, ammonium sulfate salting out, ion exchange chromatography, gel filtration, affinity chromatography, or any of these methods in combination. In view of numerous alternative protocols known in the art for the production and purification of polyclonal and monoclonal antibodies, it is not intended that the present invention be limited to any particular method for antibody purification. Numerous methods for the production and purification of polyclonal (i.e., antisera) and monoclonal antibodies are well known in the art, and can be found in various sources.

### AMPLIFICATION AND DETECTION SYSTEMS IN BIOCHEMICAL ASSAYS

The use of at least one reagent is required to convert the presence (or absence) of a particular biomolecule or property into a detectable signal. The compositions and methods of the invention are readily adapted for use with many known detection systems, including for example, optical, electrochemical, nanomechanical, isotopic, radioisotopic, mass spectrometric etc. However, it is not intended that the invention be limited to any particular detection system, readout, or apparatus. One of skill in the art will recognize that the compositions and methods described herein can be readily adapted to make use of many alternative detection schemes and apparatus, all of which are within the scope of the invention.

Some aspects of the invention described herein are illustrated using optical detection systems simply because of the straightforward and widespread use and flexibility of optical systems. For example, using apparatus and reagents common in most laboratories, the presence of a biomolecule can trigger luminescence, fluorescence, phosphorescence or development of color, for example. The light emission in these optical systems can be passive or induced, and can include real-time, time resolved and steady state measurement. An optics system can be used to then detect the light via either a passive or active illumination. In most practical applications detection will require that each of the detection events associated with each individual cell be physically separated, though multiplexed detection and deconvolution is also feasible. The source of luminescence can be linked to a solid phase or present in solution, but in all cases, linked to the detection of the biomolecule of interest.

The use of multiple chromophores with resolvable optical emissions, e.g., multiple fluorescent dyes having different emission wavelengths, can optionally be used to simultaneously detect more than one biomolecule in a single partitioned aqueous reaction. For example, more than one expressed gene can be detected by using a multiplex PCR reaction, and the amplification products detected using specific probes differentially labelled with fluorescent dyes having different emission wavelengths.

The invention provides various methods for generating compartmentalized aqueous environments containing a cell or groups of cells, and in some embodiments, creates highly compartmentalized systems with high throughput, highly parallel capability. In some aspects, these highly partitioned systems enable the use of various methods of biomolecule and signal amplification to detect the presence or absence (or optionally quantitate) a biomolecule associated with a cell, further where the assay has single cell resolution capability.

In some aspects, the addition of biochemical assay reagents to the high density partitions of cells or small groups of cells creates an environment for (a) the amplification of the biomolecule of interest, or (b) the amplification of the signal generated from detection of the biomolecule. The detectable signals can be created as part of the amplification event or can be created due to the presence of an enzyme or other active agent that was encapsulated due to a binding event creating a signal amplification event, as is the case where a biomolecular probe or coupled enzyme is prebound to the biomolecular target prior to partitioning (see, e.g., FIGS. 2A-2C). As used herein, the term "amplification" is used in its broadest sense, and is not limited to any particular format, target molecule, method of detection or reagents used. For example, amplification can include PCR amplicons, Invader™ assay cleavage products, TMA RNA products, and any amplification of an optically detectable signal.

The detection process can involve the use of environmental changes to initiate and/or promote the amplification events. Such environmental changes can include the introduction of changes in temperature, including thermal cycling, changes in pH, permeation of gases, ions or small molecules, exposure to visible light or other forms of electromagnetic radiation, changes in pressure, g-force, vibration, sonication or other environmental factors as can be envisioned by one skilled in the art.

In other embodiments, performance of the detection process can involve additional steps. For example, amplified products generated for each cell can be captured or linked to a solid phase that in whole or in part comprises a component of the material in contact with the aqueous solution containing a cell. One or a plurality of these amplified elements, such as nucleic acids, polypeptides or other products of enzymatic reactions can further comprise a label/detectable moiety or provide for a novel sequence or structure that can be further probed by a second molecule comprising a detectable label. In still other embodiments, additional rounds of detection and amplification of signal can be performed where the components that are captured (e.g., bound) by the solid-phase are isolated and mixed with biochemical reagents to enable additional amplification and detection events.

The integration of solid phase capture of the resultant amplified products provides for embodiments wherein the vesicles that contain cells can be disrupted and the solid phase components, e.g., beads or surfaces, are further processed, e.g., washed and/or purified from solution phase biochemical and reactive elements, to enable the detection of one or more amplified and/or captured detectable probes. For example, nucleic acid products generated during the biomolecule detection process and captured or linked to the solid phase components can be detected through a variety of means primarily involving hybridization of a complementary nucleic acid that either directly links a detectable moiety or label to the solid phase, or mediates a process, e.g., ligation, cleavage, or polymerization, that ultimately leads to the linking of a detectable moiety or label to the presence of the captured nucleic acid.

### SOLID PHASE COMPONENTS (e.g., BEADS) IN BIOCHEMICAL ASSAYS

In some embodiments, it is a further characteristic of the invention that one or more biochemical reagents are linked to a solid phase, and the solid phase is in contact with the aqueous phase comprising the cell or cells. The utilization of a solid-phase biochemical reagent (e.g., beads, channel surface, plate surface) enables the capture of certain reaction products to the solid phase during the biochemical detection process. Additional biochemical steps can then be performed following the capture of the solid-phase, where the initial partitioning is no longer required and can be removed. Methods for the use of solid phase reagents such as beads are well established and are well known to one of skill in the art, and the reagents are widely available from a number of manufacturers. It is not intended that the present invention be limited to any particular type of solid support material. One familiar with the art recognizes that the materials and configurations for the solid support chosen for use in the methods of the invention will depend on the particular biomolecule assay that is being used, of which there are many possibilities.

A wide variety of solid phase components (synonymously termed "solid supports") find use with the invention. Generally, the term "solid support" refers to a matrix of material in a substantially fixed arrangement that can be functionalized to allow synthesis, attachment or immobilization of polynucleotides or polypeptides, either directly or indirectly. The term "solid support" also encompasses terms such as "resin" or "solid phase."

A solid support can be composed of polymers, e.g., organic polymers such as polystyrene, polyethylene, polypropylene, polyfluoroethylene, polyethyleneoxy, and polyacrylamide, as well as co-polymers and grafts thereof, or plastics. A solid support can also be inorganic, such as glass or other ceramics, silica, silicon, controlled-pore-glass (CPG), reverse-phase silica, any suitable metals, metalloids, alloys, and composites.

For instance, the solid supports (such as beads) can comprise a material selected from a group consisting of: silicon, silica, quartz, glass, controlled pore glass, carbon, alumina, titania, tantalum oxide, germanium, silicon nitride, zeolites, and gallium arsenide. Many metals such as gold, platinum, aluminum, copper, titanium, and their alloys are also options for use as solid supports. In addition, many ceramics and polymers can also be used as solid supports. Polymers which can be used as solid supports include, but are not limited to, the following: polystyrene; poly(tetra)-fluoroethylene (PTFE); polyvinylidenedifluoride; polycarbonate; polymethylmethacrylate; polyvinylethylene; polyethyleneimine; poly(etherether)ketone; polyoxymethylene (POM); polyvinylphenol; polylactides; polymethacrylimide (PMI); polyatkenesulfone (PAS); polypropylene; polyethylene; polyhydroxyethylmethacrylate (HEMA); polydimethyl-siloxane; polyacrylamide; polyimide; and block-copolymers. Preferred substrates for the array include silicon, silica, glass, and polymers. The solid support can be composed of a single material (e.g., glass), mixtures of materials (e.g., co-polymers) or multiple layers of different material (e.g., metal coated with a monolayer of small molecules, glass coated with a BSA, etc.).

In addition to those described herein, it is also intended that the term "solid support" include any solid support that has received any type of coating or any other type of secondary treatment, *e.g*., Langmuir-Blodgett films, self-assembled monolayers (SAM), sol-gel, or the like.

Solid supports can be flat or planar, or can have substantially different conformations. For example, the solid support can exist as particles, beads, strands, precipitates, gels, sol-gels, sheets, tubing, spheres, containers, capillaries, channels, pads, slices, films, plates, dipsticks, slides, etc. Magnetic beads or particles, such as magnetic latex beads and iron oxide particles, are examples of solid substrates that can be used in the methods of the invention. Magnetic particles are described in, for example, US Patent No. 4,672,040, and are commercially available from, for example, PerSeptive Biosystems, Inc. (Framingham MA), Ciba Coming (Medfield MA), Bangs Laboratories (Carmel IN), and BioQuest, Inc. (Atkinson NH). The solid support is chosen to maximize signal to noise ratios, primarily to minimize background binding, for ease of washing and cost. In addition, certain solid supports such as beads can easily be used in conventional fluid handling systems such as microwell plates. The separation of materials that can be achieved by such conventional fluid handling systems can be used to construct arrays according to the present invention, e.g., to provide beads comprising different un-natural amino acid-containing polypeptides, or contact with different reagents, or both.

The configuration of a solid support is in any appropriate form, e.g., can comprise beads, spheres, particles, granules, a gel, a sol-gel, a self-assembled monolayer (SAM) or a surface (which can be flat, or can have shaped features). The term "solid support" includes semisolid supports. Surfaces of the solid support can be planar, substantially planar, or non-planar. Solid supports can be porous or non-porous, and can have swelling or non-swelling characteristics. A solid support can be configured in the form of a well, depression or other container, vessel, feature or location. A plurality of solid supports can be configured in an array at various locations, addressable for robotic delivery of reagents, or by detection means including scanning by laser or other illumination and CCD, confocal or deflective light gathering.

For example, in one embodiment, the solid supports can be in the form of beads (synonymous with particle). In general, as used in the art, the beads that find use with the invention are typically coupled to a second molecule that provides a capture function. For example, a bead used in conjunction with the invention can be coupled to a polynucleotide or a polypeptide (e.g., an antibody). See, for example, **FIGS. 1A-1K****.**

In addition to beads that carry biomolecular probes such as specific antibodies or specific polypeptide sequences, the beads can also utilize other tethering mechanisms for connecting a protein or nucleic acid target to the bead. Such tethering methods include: chemical tethering, biotin-mediated binding, cross-linking to the solid support matrix (e.g., UV, or florescence activated cross-linking) and the use of 'soluble' matrix, such as PEG, which can be precipitated by ethanol or other solvents to recover bound material (*see also,* Wentworth (1999) TIBTECH 17:448-452).

A bead can be made of any substrate material, including biological, non-biological, organic, inorganic, polymer, metal, or a combination of any of these. The surface of the bead can be chemically modified and subject to any type of treatment or coatings, e.g., coatings that contain reactive groups that permit binding interactions with polypeptides or polynucleotides. In some embodiments, the beads can be produced in a way that facilitates their rapid isolation and/or purification. For example, magnetic beads can be manipulated by applying a magnetic field to rapidly isolate the beads from a liquid phase within a plate well.

In another embodiment, a solid support comprises or consists of a sol-gel. Sol-gel technologies are well known, and described, e.g., in Kirk-Othmer Encyclopedia of Chemical Technology third and fourth editions, esp. volume 20, Martin Grayson, Executive Editor, Wiley-Interscience, John Wiley and Sons, NY, e.g., at volume 22 and the references cited therein. Sols are dispersions of colloidal particles (typically nanoscale elements) in a liquid such as water, or a solvent. Sol particles are typically small enough to remain suspended in the liquid, e.g., by Brownian motion. Gels are viscoelastic bodies that have interconnected pores of submicrometeric dimensions. Sol-gels are used in the preparation of glass, ceramics, composites, plastics or the like by preparation of a sol, gelation of the sol and removal of the liquid suspending the sols. This process is used in the many relatively low-temperature processes for the construction of fibers, films, aerogels, and the like (any of which can be the solid support in the present invention). Three general processes for making sol-gels are typically used. In the first, gelatination of a dispersion of colloidial particles is performed. In the second, hydrolysis and polycondensation of alkoxide or metal salt precursors is performed. In the third, hydrolysis and polycondensation of alkoxide precursors followed by aging and drying at room temperature is performed. For further details, see, Kirk-Othmer, id.

In some embodiments of the invention, the preferred compositions and methods of the invention incorporate a solid phase capture step. The solid phase can be incorporated as a third phase component within the system in a form such as a bead (see FIG. 3B) or it can comprise part of the apparatus used for enclosure. Examples of apparatus used for enclosure include, but are not limited to, the walled surfaces within a flow system or the presence of a filter or matrix that enables solution to flow against or through. The solid phase can optionally comprise specific reagents to enable the capture of certain biomolecules via molecular affinities between the solid-phase linked molecular reagents and the biomolecules derived from the cells or the amplified products created due to the presence of biomolecules derived from the cells and their initiation of biochemical reactions. Examples of reagents for biomolecule capture include antibodies, fragments of antibodies, and proteins derived from antibodies, or non-antibody proteins that have a specific affinity for another protein (e.g., specific protein-protein interactions), non-protein molecules with specific protein affinities, antibodies or other molecules with specific affinities to other biomolecules other than proteins and nucleic acid probes.

A second class of solid-phase linked biochemical reagents include activated chemical functional groups with selective reactivity for specific types of biomolecules, chemical functional groups having selective hydrophobic or hydrophilic properties creating specific affinities for certain types of biomolecules, or chemical functional groups having selective cationic or anionic properties with specific affinities for certain types of biomolecules. Examples further include the capture of reaction products such as reacted enzyme substrates including small molecules, peptides, and nucleic acid probes where the enzymatic process either adds to or removes functionality from the substrate, most typically to create a detectable element such as an attached and unquenched chromophore or other detectable moiety.

The invention provides methods and compositions that make use of two immiscible phases, one of which is an aqueous phase comprising one or more cells and the second phase is a partitioning phase or forms a vesicle structure. In some embodiments, systems of the invention can include more than two phases, for example three phases, where the third phase sequesters, binds or absorbs specific biomolecules of the cells, or some reaction product that can be detected. In other embodiments, the solid phase can provide specific reagents that are necessary for an assay that detects a biomolecule associated with the cell.

Most typically, the third phase is a solid phase, for example but not limited to, beads or other types of small particles. Third phase reagents can be optionally solid phase wherein the reagents are bound to the surface of the solid phase or are locked within the solid phase to be release upon induction of the biochemical reaction by a method such as heating. In other embodiments, the partitioning of the cells can be performed using a solid phase material. In either of these embodiments, the third phase is either present within the vesicle comprising the aqueous solution or in contact with the aqueous phase so that the biomolecules of the cells can interact with the third phase reagents, solid phase or otherwise presented.

Solid phase components can be incorporated into flow systems of the invention by a variety of means. In some embodiment where partitioned cells are propagated along a channel, the channel itself can function as the solid phase where specific biochemical elements are used to capture a detectable molecule that is either an amplified product derived from one or more biomolecules present within the cell or group of cells, or are the biomolecules themselves. In a second embodiment, a solid phase can be introduced in the form of a bead that is introduced into the flow stream in such a manner that it merges with the aqueous phase comprising the cell or group of cells (see **FIGS. 3A** and **3B****).** Introduction can be via various microfliudic mechanism as known in the art. In other embodiments where the cell or groups of cells are partitioned into wells of a microtiter plate, the solid phase can be the surface of the well or any number of different materials such as beads, filters and the like, that can be placed in the wells. In still other embodiments, the solid phase can be introduced in pre-solid phase form, e.g., polymer precursors such as acrylamide, and induced to become a solid phase at some point during the cell processing or biochemical assay.

### INSTRUMENTATION AND DEVICES

The invention provides instrumentation that facilitates the methods of the invention. This instrumentation can take advantage of existing fluid handling technologies, for example, flow cytometry, microfluidics and nanofluidics. It is not intended that the instrumentation described herein be limited to any particular configuration, as one of skill in the art recognizes that various components described herein for constructing the instrumentation can be substituted with a variety of substantially equivalent components, all of which find use with and are within the scope of the claimed invention.

The instrumentation of the invention (e.g., devices of the invention) enable or facilitate the methods described herein. The devices of the invention provide various features and functions, which can include all desired functions for analysis of a biomolecule associated with cells, or a subset of desired functions. A device of the invention can, in part or in total, provide:
(a) a means for generating partitioned aqueous reaction volumes in a flow channel that is part of a liquid flow system. The flow channel can be of any form that can divert or direct liquid flow, such as but not limited to, pipes, channels, tubes, grooves, capillaries, a microchanneled plate, or any suitable structure for channeling a liquid flow;
(b) a means for generating partitioned aqueous volumes that comprise one cell or a group of cells;
(c) a means for generating partitioned aqueous reaction volumes that comprise at least one reagent for detecting the presence or absence of a biomolecule of interest;
(d) a means for sorting cells into various fractions, including a selected fraction that will continue in the biomolecule analysis, and an unselected fraction that will not continue in the biomolecule analysis;
(e) a means for thermally regulating reaction vesicles or partitioned aqueous reaction volumes in a flow channel, or in any other part of a device of the invention. Such a thermally controlled environment is required for PCR, as well as other biochemical assays;
(f) a means for excitation of a reaction vesicle or partitioned aqueous reaction volume (e.g., an excitation light source for the excitation of fluorescent moieties);
(g) a means for signal detection from a reaction vesicle or partitioned aqueous reaction volume (e.g., a light emission detector). For example, a device can comprise a detector for detecting fluorescence or other light emissions at the appropriate wavelengths, where the detection is performed at a resolution capable of uniquely resolving all or a significant number of the reaction vesicles or partitioned aqueous reaction volumes;
(h) a means for collection, storage, analysis, manipulation and/or display of collected data from a detector, e.g., an electronic module such as a computer having a hard drive.

In some aspects, the devices of the invention pertain to the analysis of reaction vesicles (e.g., an emulsion of aqueous vesicles). In other aspects, the devices of the invention pertain to the generation and analysis of partitioned aqueous reaction volumes in a flow channel that is part of a liquid flow system. In still other aspects, a device of the invention pertains to both the analysis of reaction vesicles and partitioned aqueous reaction volumes in a flow channel that is part of a liquid flow system.

Example devices of the invention are provided in **FIGS. 3A, 3B****,** 5 and 6. The devices depicted in these figures are intended only to illustrate various embodiments of the devices of the invention. It is not intended that the invention be limited to these devices, as one of skill in the art will recognize various other combinations of elements and substantially similar or equivalent components of the devices that also are within the scope of the invention.

**FIG. 3A** provides a device for the creation of single cell aqueous reaction volumes in a flow channel. In this device, aqueous solution **300** comprising cells **340** flows in a flow channel **305** past optical sensor **310.** Optical sensor **310** regulates flow sorting point **315,** which sorts the cells in the aqueous flow into selected cells for partitioning and unselected cells that exit the flow stream **320.** Flow channel **305** is fluidly coupled to a source **325** of immiscible non-aqueous partitioning solution **328.** Immiscible partitioning solution **328** is delivered into flow channel **305** comprising the selected aqueous flow comprising cells **340,** thereby generating aqueous reaction volume **330** that is partitioned from adjacent aqueous reaction volumes by non-aqueous immiscible partitions **335,** wherein partitioned aqueous reaction volumes **330** comprise, on average, one cell per partitioned space. Flow channel **305** is further optionally fluidly coupled to source **332** of aqueous phase reagents **334,** wherein aqueous phase reagents can be delivered into said partitioned aqueous reaction volumes **330** within flow channel **305.** Partitioned aqueous reaction volumes **330** continue in flow channel **305** for analysis **345.**

A second embodiment of this device is shown in **FIG. 3****B.** This device is similar to the device of **FIG. 3A****,** with the exception of including a solid phase bead reagent. The device of **FIG. 3B** finds use in the creation of single cell aqueous reaction volumes in a flow channel, and further, where a solid phase component such as a bead is delivered into the aqueous reaction volume post-partitioning.

In this device, aqueous solution **350** comprising cells **390** flows in a flow channel **355** past optical sensor **360.** Optical sensor **360** regulates flow sorting point **365,** which sorts the cells in the aqueous flow into selected cells for partitioning and unselected cells that exit the flow stream **370.** Flow channel **355** is fluidly coupled to a source **375** of immiscible non-aqueous partitioning solution **378.** Immiscible partitioning solution **378** is delivered into flow channel **355** comprising the selected aqueous flow comprising cells **390,** thereby generating aqueous reaction volume **380** that is partitioned from adjacent aqueous reaction volumes by non-aqueous immiscible partitions **385,** wherein partitioned aqueous reaction volumes **380** comprise, on average, one cell per partitioned space. Flow channel **355** is further optionally fluidly coupled to source **382** of aqueous phase reagents **384,** wherein aqueous phase reagents can be delivered into said partitioned aqueous reaction volumes **380** within flow channel **355.** In the present embodiment, aqueous reagent flow **384** delivers solid phase beads **383** to partitioned aqueous reaction volumes **380** within flow channel **355.** Partitioned aqueous reaction volumes **380** continue in flow channel **355** for analysis **395.**

As described above, optical sensors **310** or **360** can be used to in accordance with standard flow cytometry platforms, where the sensor can be used to prescreen cells and regulate a controller that controls a flow sorting mechanism **315** or **365** to place the cells into selected and unselected fractions based on a desired trait (for example, the presence or absence of cell surface protein). Alternatively, in other embodiments, optical sensor **310** or **360** can be used to monitor the rate of passage and/or concentration of cells **340** or **390** in the flow channel **305** or **355.** In this alternative embodiment, the sensor regulates a controller that controls the rate and timing of delivery of immiscible partitioning solution 328 or 378 into the aqueous flow in flow channel **305** or **355,** thereby optimizing the formation of partitioned aqueous volumes comprising one cell (on average).

Another device of the invention is illustrated in **FIG. 5****.** This device is for the spectrophotometric analysis of compartmentalized aqueous volumes comprising cells, for example, vesicles that are in an emulsion. In this device, vesicles **501** that are contained in a fluid emulsion **500** are provided to the system in either a one dimensional (see **FIG. 4B****)** or two dimensional (see **FIG. 4A****)** aspect. In a one dimensional scenario, containing faces **502** form a flow channel through which emulsion **500,** comprising vesicles **501,** flow. In a two dimensional scenario, containing faces **502** are transparent flat plates used to create a thin layer space comprising vesicles **501.**

The vesicles in the system can be temperature controlled, for example, by thermal control element **510.** Thermal control of vesicles can be required for the biochemical assay to detect the presence or absence of a biomolecule of interest (for example, as in PCR to detect a nucleic acid of interest).

Following the processing of the vesicles for the detection of a biomolecule, the vesicles are excited using excitation light source **515.** Excitation light source **515** further comprises one or more filter **520** and one or more optics element **525** that are suitable for the analysis and the wavelength used to excite the vesicles. Following excitation of vesicles **501,** light emission signals from vesicles **501** are detected by detector **530.** Detector **530** further comprises one or more filter **535** and one or more optics element **540** that are suitable for the analysis and the wavelength being detected. Emission signals that are detected by detector **530** are transmitted to operably coupled electronic module **545,** which can be, for example, a computer system.

As shown in **FIGS. 4A** and **4B****,** the detection system used, as illustrated in the device of **FIG. 5****,** can vary. In one aspect, the specific detection arrangement involves the placement of vesicles **501** (in an emulsion **500)** between flat plates **502,** one of which is transparent to the detection method, so as to create a thin layer that can be scanned, optically or otherwise, at a resolution where all or a significant number of the vesicles can be uniquely observed (see **FIG. 4A****).** In this embodiment, either the apparatus comprising vesicles **501** or the apparatus housing excitation light source **515** and/or detector **530** is movable in the X-Y plane of the vesicles in order to systematically focus on single vesicles.

Alternatively, emulsion **500** (containing partitioned reaction vesicles) or other partitioning event can be present in narrow microfluidic channels, tubes or capillaries where the channels can be optically scanned (e.g., by detector **530)** at a resolution where all or a significant number of the vesicles can be uniquely observed (see **FIG. 4B****).** Other physical formats can be envisioned by one skilled in the art, where the critical feature is that the optical detection system (e.g., detector **530)** coupled with a specific presentation of vesicles **501** is capable of detecting the light emanating from distinct individual vesicles. In general, vesicles **501** in an emulsion are prepared in a thin layer so that partitioning is primarily in one or two dimensions with the optical observation occurring in a third dimension above, below or to the side of the one or two dimensional partitioning. Such a strategy limits the possibility of detecting multiple vesicles that might be overlapping in the third dimension. Alternatively, methods for focusing the detector **530** to different depths within the third dimension can be used to discretely detect individual vesicles in all three dimensions such as can be performed by scanning confocal optical systems.

Another device of the invention is provided in **FIG. 6****.** The device of **FIG. 6** is for the generation and analysis of compartmentalized (e.g., partitioned) single cell aqueous reaction volumes in a flow channel in a liquid flow system. In this device, reservoir **602** of aqueous solution **600** comprising cells **605** is fluidly coupled to flow channel **622** via coupling **610** and channel junction **625.** In some embodiments, aqueous solution **600** also comprises at least one reagent for detecting the presence or absence of a biomolecule associated with cells **605.**

Reservoir **608** of immiscible, non-aqueous solution **607** is also fluidly coupled to flow channel **622** via coupling **609** and channel junction **625.** Flow channel **622** and couplings **609** and **610** can be of any form that can divert or direct liquid flow.

The coordinated intermittent flows of aqueous solution **600** (through coupling **610)** and immiscible, non-aqueous solution **607** (through coupling **609)** into channel junction **625** and into flow channel **622** generates partitioned aqueous reaction volumes **629** (also termed aqueous chambers or aqueous reaction core), where each partitioned volume can comprise one cell or a group of cells, as desired. Each aqueous reaction volume **629** is partitioned from adjacent aqueous reaction volumes in flow channel **622** by non-aqueous immiscible partitions **628** formed from immiscible, non-aqueous solution **607** (which is acting as a partitioning solution).

Following the generation of partitioned aqueous reaction volumes **629** in flow channel **622,** the aqueous compartments are subjected to conditions for detecting the presence or absence of a biomolecule associated with the cells. The aqueous compartments in the system can be temperature controlled, for example, by thermal control element **626.** Thermal control of compartments can be required for the biochemical assay to detect the presence or absence of a biomolecule of interest (for example, as in PCR to detect a nucleic acid of interest).

Following a suitable biochemical assay, the aqueous compartments can be excited using excitation light source **631.** Excitation light source **631** further comprises one or more filter **632** and one or more optics element **633** that are suitable for the analysis and the wavelength used to excite the aqueous chambers. Following excitation of aqueous chambers **629,** light emission signals from aqueous chambers **629** are detected by detector **635.** Detector **635** further comprises one or more filter **636** and one or more optics element **637** that are suitable for the analysis and the wavelength being detected. Emission signals that are detected by detector **635** are transmitted to operably coupled electronic module **640,** which can be, for example, a computer system.

In this device, coupling **610** is operably connected to optional optical sensor **615** that detects cells **605 as** they pass through coupling **610.** Optical sensor **615** is operably connected to controller **620** that controls the flow of aqueous solution **600** (and cells **605)** and immiscible solution **607** into flow channel **622.** In some embodiments, optical sensor **615** can be used to monitor the rate of passage and/or concentration of cells **605** passing through coupling **610.** In response to detection of cells **605,** optical sensor **615** regulates controller **620** that controls the rate and timing of delivery of aqueous solution **600** and immiscible, non-aqueous solution **607** through channel junction **625** and into flow channel **622,** thereby optimizing the formation of partitioned aqueous volumes comprising one cell (on average).

### Flow Cytometry

The compositions, methods and devices of the invention can be utilized in combination with traditional flow cytometry, including both simple flow detection systems and flow detection sorting systems. Aspects of flow cytometry technology can be incorporated into the devices of the invention. In these embodiments, the cells are first presented to the flow system and detected using standard techniques. The detection events can then used to direct and coordinate the timing of events to enable the partitioning of the individual cells or subgroups of cells into separate reaction chambers or compartments. Two strategies for separation are (a) the diversion of the flow stream wherein a detected cell is sorted into a specific, physically separate reaction chamber (see **315** in **FIG. 3A** and **365** in **FIG. 3B****),** and (b) a controller triggers the merger of a non-aqueous partitioning solution with an aqueous flow containing cells, where the non-aqueous solution forms partitions between cells in the aqueous phase, thereby creating an immiscible barrier between adjacent cells that have been detected in the flow system (see the device of **FIG. 6****,** specifically, controller **620).** In some embodiments of the device of **FIG. 6****,** an optical sensor instructs the controller in the generation of the partitioned aqueous phases (see **FIG. 6****,** specifically, optical sensor **615).**

In the diversion strategy, the flow cytometry is used as an observational tool to detect each of the cells and to provide information so that the downstream mechanics can be triggered to direct the flow stream, resulting in the observed cell (usually along with some volume of aqueous solution) being placed in a physically partitioned location. Such mechanisms, for example, can comprise a multiwelled microtiter plate that is placed on an X-Y moveable platform, where the platform can then be moved to place a particular well of the microtiter plate directly below the output of the flow stream at a suitable time. The result is that the solution exiting the flow stream is directed into the desired well of the microtiter plate. In some aspects, the well will then contain the cell or group of cells that were detected within the flow system.

This diversion strategy flow system can optionally comprise a sensor suitably positioned to monitor cells passing through the flow system prior to the sorting point (see **FIGS. 3A****, 310** and **3B****, 360).** In some aspects, the sensor can be a simple optical sensor that detects the passing of any cell through the flow system. In other aspects, the sensor can be a spectrophotometric detector that can detect emitted light at any desired wavelength (e.g., a fluorescence emission). Also, as commonly used in flow cytometry systems, the sensor can be operably coupled to a controller (or a controller system) that sorts the detected cells into desired subpopulations based on the presence, absence or intensity of the emitted signal detected by the detector.

In the partitioning strategy that uses intermingled flows of two immiscible liquid phases, the flow solution (usually the aqueous phase) is interrupted via the injection of the immiscible second phase into the aqueous stream. The injection of the second immiscible phase can be performed utilizing a variety of mechanisms, including various microfluidic valving strategies, syringe pumps, piezo electric injection, regulated sheath flow, and the like as known in the art. This results in a stream comprising a repeated pattern of aqueous solution containing one cell or groups of cells partitioned from each other by a second immiscible phase, e.g. silicone oil, or other suitable liquid as known in the art. The resulting partitioned stream can then be continued in a pipe, channel, tube, groove, capillary, a microchanneled plate, or any suitable structure for channeling a flow. Alternatively, the stream can be introduced into a chamber containing a non-aqueous, immiscible liquid where the aqueous partitioning become vesicles, similar to what is observed with the formation of emulsions.

In some embodiments, as shown in **FIGS. 3A** and **3B****,** the partitioned flow is formed by injecting a non-aqueous partition into an aqueous flow in the flow channel. Alternatively, a partitioned flow can be formed by injecting the aqueous phase (containing the cells) into a flow of an immiscible, non-aqueous liquid. However, this distinction is inconsequential, because both methodologies yield the same result (i.e., partitioned aqueous volumes in a flow channel). Moreover, this distinction can become blurred depending on the apparatus used, where it becomes a matter of semantics in describing which solution is being injected into the other solution. In still other systems, the flow of both of the phases (e.g., one aqueous and one non-aqueous) is controlled by a common controller that alternately delivers the liquids into a common channel to form the partitioned liquid volumes (see, **FIG. 6****).**

Flow cytometry offers the opportunity to characterize (e.g., prescreen) the cells prior to performance of the detection of a biomolecule or a plurality of biomolecules. As well known in the art, cells can be labeled via labeled antibodies, antibody fragments, receptor ligands, polypeptides, and the like, to specifically signal the presence of particular proteins on the cell surface, or the presence of particular cell-cell interactions. Alternatively, various agents for *in situ* observation that are compatible with flow cytometry can be used to identify the presence of various biomolecules associated with a cell or provide information about the phenotypic state of a cell. These agents include small molecules, dyes, quantum dots, and the like, as known in the art for use in cell staining. In some embodiments, where the cells in the flow system are tracked through the analysis, the information derived from the flow cytometry can be linked with the downstream events used in the detection of the cellular biomolecule.

In other embodiments, the cells or groups of cells in the flow system need not require a biochemical detection step (e.g., a detection step that will sort cells based on the presence or absence of a cell surface protein). In this case, the information gathered from the flow system is simply to quantitate the number of cells passing through the flow stream. This information can be used to optionally regulate a basic fractionation or partitioning of the flow solution, where there will be a statistical chance, as determined by the relative concentration of the cells within the aqueous solution and the quantity of solution per fractionation or partition, that a cell or group of cells will be placed within each well or aqueous partition. Like the emulsion formation strategy to form vesicles, the cell concentration and solution volume per fractionation can be optimized to a preferred probability of approximately one cell per well or partition.

### Liquid Flow Systems and Microfluidics Technology

The invention provides instrumentation (e.g., devices) that facilitate the methods of the invention. This instrumentation of the invention can readily adapt and incorporate existing fluid handling platform technologies, for example, flow cytometry, other liquid flow technologies, microfluidics and nanofluidics.

In some embodiments, a device of the invention includes a flow channel (within a liquid flow system) wherein partitioned aqueous reaction chambers are generated or through which reaction vesicles can flow. The flow channel can be of any form that can divert or direct liquid flow. Such forms include, but are not limited to, pipes, channels, tubes, grooves, capillaries, a microchanneled plate, or any suitable structure for channeling a liquid flow.

Solutions used with the invention (e.g., the aqueous phase comprising cells, and the non-aqueous partitioning solution) can be manipulated (e.g., aliquotted and/or diluted) using standard or microfluidic fluid handling approaches (or combinations thereof). Standard fluid handling approaches for dilution/ aliquotting include, e.g., pipetting appropriate volumes of the sample into microtiter trays and adding an appropriate diluent. These operations can be performed manually or using available high throughput fluid handlers that are designed to use microtiter trays. High throughput equipment (e.g., incorporating automated pipettors and robotic microtiter tray handling) is preferred.

Many automated systems for fluid handling are commercially available and can be used for aliquotting and/or diluting fluids in the context of devices of the present invention. For example, a variety of automated systems are available from the Zymark Corporation (Zymark Center, Hopkinton, MA), which utilize various Zymate systems, which typically include, e.g., robotics and fluid handling modules. Similarly, the common ORCA® robot, which is used in a variety of laboratory systems, e.g., for microtiter tray manipulation, is also commercially available, e.g., from Beckman Coulter, Inc. (Fullerton, CA). In any case, conventional high throughput systems can be used in place of, or in conjunction with microfluidic systems (for example, conventional systems can be used to aliquot samples into microtiter trays, from which microfluidic systems can draw materials) in devices of the invention and in practicing the methods of the invention.

Microfluidic systems provide a preferred fluid handling and amplification technology that can conveniently be applied to the present invention. In typical embodiments, samples are drawn into microfluidic devices that comprise networks of microscale cavities (channels, chambers, etc., having at least one dimension less than about 500 µm in size and often less than about 100µm) and the samples are mixed, diluted, aliquotted or otherwise manipulated in the network of cavities. For example, the microscale device can comprise one or more capillary, in fluid communication with the network, extending outward from a body structure of the microscale device. Negative pressure (vacuum) is applied to the capillary and fluids are drawn into the network from a container (e.g., a well on a microtiter tray). This process can be multiplexed by using a device that comprises multiple capillary channels, permitting many samples to be drawn into the network and processed simultaneously. Sample interfaces with dried samples can also be performed using this basic system, e.g., by partly or completely expelling fluid from the capillary to hydrate samples prior to drawing them into the microfluidic device (the fluid is typically contacted to the samples as a hanging drop on the tip of the capillary and then drawn back into the capillary). For either approach, *see also,* US Patent No. 6,482,364 to Parce, et al. (November 19, 2002) MICROFLUIDIC SYSTEMS INCLUDING PIPETTOR ELEMENTS; US Patent No. 6,042,709 to Parce, et al. (March 28, 2000) MICROFLUIDIC SAMPLING SYSTEM AND METHODS; US Patent No. 6,287,520 to Parce, et al. (September 11, 2001) ELECTROPIPETTOR AND COMPENSATION MEANS FOR ELECTROPHORETIC BIAS and US Patent No. 6,235,471 to Knapp, et al. (May 22, 2001) CLOSED-LOOP BIOCHEMICAL ANALYZERS.

Essentially any fluid manipulation that occurs in devices of the invention (aliquotting, channeling, diluting, mixing. combining, heating and cooling) can be performed using known available platforms. Details regarding dilution and aliquotting operations in microscale devices can be found in the patent literature, e.g., USP 6,149,870 to Parce, et al. (November 21, 2000) APPARATUS FOR IN SITU CONCENTRATION AND/OR DILUTION OF MATERIALS IN MICROFLUIDIC SYSTEMS; USP 5,869,004 to Parce, et al. (February 9,1999) METHODS AND APPARATUS FOR IN SITU CONCENTRATION AND/OR DILUTION OF MATERIALS IN MICROFLUIDIC SYSTEMS; and USP 6,440,722 to Knapp, et al. (August 27, 2002) MICROFLUIDIC DEVICES AND METHODS FOR OPTIMIZING REACTIONS. Samples and components to be mixed/ diluted or aliquotted can be brought into the microscale device through pipettor elements or from reaction component reservoirs on the device itself, or, commonly, both. For example, the sample can be brought into the microfluidic device through a pipettor channel and diluted and supplied with common reagents from an on device dilution and/ or reagent reservoir(s). Locus specific reagents (e.g., amplification primers) can be on the device in wells, or stored off the device, e.g., in microtiter plates (in which case they can be accessed by the pipettor channel). Any or all of these operations can be performed in a continuous or stopped flow format.

The instrumentation provided by the invention can also include a chip that typically performs reaction assembly (assembly of reaction components), thermocycling, and acting as a "cuvette" for an optical system during an imaging step. In the reaction assembly, the reaction mixture components which get combined at the last second before heating begins are assembled. This is called a "hot start" and provides advantages of specificity. During thermocycling, the system optionally provides both constant fluid movement and constant temperature change. During imaging, a high data rate CCD is useful in providing an adequate dynamic range using the dispersion/diffusion methods of quantification.

Commercial systems that perform all aspects of fluid handling and analysis that can be used in the practice of the present invention are available. Examples include the 250 HTS system and AMS 90 SE from Caliper Technologies (Mountain View, CA). These systems performs experiments in serial, continuous flow fashion and employ a "chip-to-world" interface, or sample access system, called a sipper through which materials in microwell plates are sipped into a capillary or capillaries attached to the chip and drawn into the channels of the chip. There they are mixed with components of interest and a processing and result detection steps are performed.

Whether conventional fluid handling or microfluidic approaches (or both) are used, the aliquotting and/or dilution events can be performed to achieve particular results. For example, an aqueous solution comprising cells can be diluted equally in each aliquot, or, alternately, the aliquots can be differentially diluted (e.g., a dilution series can be made). The aliquots themselves are of a volume that is appropriate to the fluid handling approach being used by the system, e.g., on the order of a few microliters for microtiter plates to 100 nL, 10nL or even 1nL or less for microfluidic approaches.

### Amplification in Microfluidic Systems

A number of high throughput approaches to performing PCR and other amplification reactions have been developed, *e.g*., involving amplification reactions in microfluidic devices, as well as methods for detecting and analyzing amplified nucleic acids in or on the devices. Aspects of this art can be adapted and incorporated in the devices of the invention.

Details regarding such technology is found, e.g., in the technical and patent literature, e.g., Kopp et al. (1998) "Chemical Amplification: Continuous Flow PCR on a Chip" Science, 280 (5366):1046; USP 6,444,461 to Knapp, et al. (September 3, 2002) MICROFLUIDIC DEVICES AND METHODS FOR SEPARATION; USP 6,406,893 to Knapp, et al. (June 18,2002) MICROFLUIDIC METHODS FOR NON-THERMAL NUCLEIC ACID MANIPULATIONS; USP 6,391,622 to Knapp, et al. (May 21, 2002) CLOSED-LOOP BIOCHEMICAL ANALYZERS; USP 6,303,343 to Kopf-Sill (October 16, 2001) INEFFICIENT FAST PCR; USP 6,171,850 to Nagle, et al. (January 9, 2001) INTEGRATED DEVICES AND SYSTEMS FOR PERFORMING TEMPERATURE CONTROLLED REACTIONS AND ANALYSES; US Patent No. 5,939,291 to Loewy, et al. (August 17, 1999) MICROFLUIDIC METHOD FOR NUCLEIC ACID AMPLIFICATION; USP 5,955,029 to Wilding, et al. (September 21, 1999) MESOSCALE POLYNUCLEOTIDE AMPLIFICATION DEVICE AND METHOD; USP 5,965,410 to Chow, et al. (October 12,1999) ELECTRICAL CURRENT FOR CONTROLLING FLUID PARAMETERS IN MICROCHANNELS; Service (1998) "Microchips Arrays Put DNA on the Spot" Science 282:396-399), Zhang et al. (1999) "Automated and Integrated System for High-Throughput DNA Genotyping Directly from Blood" Anal. Chem. 71:1138-1145 and many others.

See also, US Patent No. 6,391,622 to Knapp, et al. (May 21, 2002) CLOSED-LOOP BIOCHEMICAL ANALYZERS and the references cited therein for description of systems comprising microfluidic elements that can access reagent storage systems and that can perform PCR or other amplification reactions by any of a variety of methods in the microfluidic system. For example, the microfluidic system can have one or more capillaries extending outwards from the body structure of the microfluidic system for drawing materials into the body structure. Within the body structure are microfluidic cavities (channels, chambers, or the like having at least one dimension smaller than about 500 microns, and, typically smaller than about 100 microns) in which the amplification reactions are performed. The capillaries that extend out from the body structure can access standard reagent storage elements (microtiter plates, or the like) by drawing fluid into the capillary, e.g., due to application of a vacuum or electroosmotic force. Similarly, the capillaries can access dried reagent libraries on substrates (e.g., the LibraryCard™ reagent array made by Caliper Technologies) by partly or completely expelling fluid to rehydrate library members and then by drawing the rehydration fluid back into the capillary. For example, the capillary can partly expel fluid to form a hanging drop on the capillary, which is then contacted to the material to be hydrated. The material in the hanging drop is then drawn back into the capillary. In any case, MolecularBeacons™ or TaqMan™ probes can be incorporated into the relevant amplification reaction and detected in the microfluidic device to provide for real time PCR detection. Alternately, PCR amplicons can be detected by conventional methods, such as hybridization to a labeled probe, e.g., prior to or following a separation operation that separates unhybridized probe from hybridized probe. For example, an electrophoretic separation can be performed in a channel of the microscale device.

### Adaptation of Preexisting Platforms

The instrumentation systems of the invention (e.g., devices for single cell biomolecule analysis) can incorporate preexisting technology platforms such as microfluidic devices, detectors, sample storage elements (microtiter plates, dried arrays of components, etc.), flow controllers, amplification devices or microfluidic modules, computers and/or the like. These systems can be used for aliquotting, amplifying and analyzing the biomolecules of interest associated with cells, for example, proteins and nucleic acids. Microfluidic devices, biomolecule amplification and detection techniques and reagents, excitation light sources, suitable detector systems and storage and housing elements for device construction are all well established arts. One of skill in the art will be familiar with such technologies and will recognize how to assemble and integrate the various components into the devices of the present invention.

The following discussion describes additional considerations for various system components, such as thermal control components, appropriate controllers, detectors, and electronic components such as computers. Many configurations and equivalent components are available, all of which find use with the devices of the invention. No attempt is made to summarize all known possible manufactured products or possible combinations of products that find use with the invention. Many configurations are available and one of skill would be expected to be familiar in their use and would understand how they can be applied to the present invention. Further, one of skill will also recognize that equivalent components not recited herein can be substituted for the components described herein. Devices comprising these equivalent components are also within the scope of the invention.

### Flow Controllers

A variety of controlling instrumentation is optionally utilized in conjunction with the microfluidic devices described herein, for controlling the transport and direction of fluids and/or materials within the devices of the present invention, e.g., by pressure-based or electrokinetic control.

For example, in many cases, fluid transport and direction are controlled in whole or in part, using pressure based flow systems that incorporate external or internal pressure sources to drive fluid flow. Internal sources include microfabricated pumps, e.g., diaphragm pumps, thermal pumps, Lamb wave pumps and the like that have been described in the art. See, e.g., U.S. Patent Nos. 5,271,724, 5,277,556, and 5,375,979 and Published PCT Application Nos. WO 94/05414 and WO 97/02357. The systems described herein can also utilize electrokinetic material direction and transport systems.

Preferably, external pressure sources are used, and applied to ports at channel tenmini. These applied pressures, or vacuums, generate pressure differentials across the lengths of channels to drive fluid flow through them. In the interconnected channel networks described herein, differential flow rates on volumes are optionally accomplished by applying different pressures or vacuums at multiple ports, or preferably, by applying a single vacuum at a common waste port and configuring the various channels with appropriate resistance to yield desired flow rates. Example systems are described in US Application Publication No. US2002/0019059, entitled "DEVICES, SYSTEMS AND METHODS FOR TIME DOMAIN MULTIPLEXING OF REAGENTS," published February 14, 2002.

Typically, the controller systems are appropriately configured to receive or interface with a microfluidic device or system element as described herein. For example, the controller and/or detector, optionally includes a stage upon which a microfluidic device is mounted to facilitate appropriate interfacing between the controller and/or detector and the device. Typically, the stage includes an appropriate mounting/alignment structural element, such as a nesting well, alignment pins and/or holes, asymmetric edge structures (to facilitate proper device alignment), and the like. Many such configurations are described in the references cited herein.

The controlling instrumentation discussed above is also optionally used to provide for electrokinetic injection or withdrawal of material downstream of the region of interest to control an upstream flow rate. The same instrumentation and techniques described above are also utilized to inject a fluid into a downstream port to function as a flow control element.

### Excitation/Detection Systems

A detection system (e.g., a "detector") is used in devices of the invention to detect an emission (i.e., a signal) received from, for example, a reaction vesicle or a compartmentalized aqueous volume traveling through a flow channel. As used herein, the detectors in the presently described devices are generally (but not exclusively) spectrophotometric detectors, where any desired wavelength of light emission can be detected. In some aspects, the spectrophotometric detector systems are capable of detecting only one type of light emission (e.g., fluorescence). As used herein, a spectrophotometric detector can also be termed an optical detector. Detector elements that form part of a device of the invention can be any type of detector. The detection systems envisioned expand beyond fluorescence detection, and include a plurality of electromagnetic-based systems wherein the reaction system generates a detectable moiety that generates a new electromagnetic signal or a perturbation in an existing electromagnetic signal such as a change in intensity, shift in frequency, change in polarization, or some combination therein that occurs as a shift from one state to another or as a time-resolved transition to and from one or more states.

Virtually all of these devices and methods of the invention can be utilized in a format wherein the system provides for a high resolution of detection. As an example of the resolution required, the development of a vesicle-based amplification and detection system for single cells requires the vesicle to be large enough to encapsulate the single cells, though in certain embodiments subcellular components such as nuclei or mitochondria can be used. In addition to the cell, sufficient reagents need to be contained within each vesicle to promote the detection of the specific biochemical elements. Given the average cell is approximately 10*µ*m in diameter it will require vesicles greater than 10*µ*m. In some embodiments, the detection resolution lower limit is the equivalent to or less than (i.e., at least) the size of the vesicles, e.g., a resolution of 10*µ*m or smaller, including 5*µ*m, 2*µ*m and 1*µ*m or smaller. In other embodiments, the detection resolution lower limit is greater than 10*µ*m but is still capable of resolving a majority of vesicles as individual entities. Furthermore, methods for signal processing and the use of data from multiple scans can improve resolution can be used to achieve the same goal of resolving a majority of vesicles as individual entities.

Amplification and detection are commonly integrated in a system comprising a microfluidic device in the present invention. Available microfluidic systems that include detection features for detecting nucleic acids include the 250 HTS system and AMS 90 SE from Caliper Technologies (Mountain View, CA), as well as the Agilent 2100 bioanalyzer (Agilent, Palo Alto, CA). Additional details regarding systems that comprise detection (and separation/ detection) capabilities are well described in the patent literature, e.g., the references already noted herein and in Parce et al. "High Throughput Screening Assay Systems in Microscale Fluidic Devices" WO 98/00231.

In general, the devices herein optionally include signal detectors, e.g., which detect fluorescence, phosphorescence, radioactivity, pH, charge, absorbance, luminescence, temperature, magnetism or the like. Fluorescent detection is especially preferred and can be used for detection of polypeptides and amplified nucleic acids (however, upstream and/or downstream operations can be performed, which can involve other detection methods).

It is understood that some detection systems (e.g., fluorescence detection systems) also incorporate an appropriate excitation light source that functions with the detector. For example, in order to read fluorescence from many reporting systems, the reagents must first be excited at one wavelength and will be detected at another wavelength (a fluorescent wavelength). Fluorescent labels are characterized by their excitation and emission spectra. For example, FAM is most efficiently excited by light with a wavelength of 488 nm, and emits light with a spectrum of 500 to 650 nm, with an emission maximum at 525 nm. Thus, a suitable excitation/detection system must be capable of emitting light at approximately 488 nm, and capable of detecting light at about 525 nm. To achieve this, emission sources and detectors are used in conjunction with appropriate light filters to achieve the desired excitation emission and detector specificity. Further, these light sources and detectors are also used in conjunction with optics components to focus the light to the desired position in a device and achieve an appropriate intensity.

A detector as used in the devices of the invention, e.g., a fluorescence spectrophotometer, is typically operably connected to a computer for controlling the spectrophotometer operational parameters (e.g., wavelength of the excitation and/or wavelength of the detected emission) and/or for storage of data collected from the detector (e.g., fluorescence measurements from individual reaction vesicles or partitioned aqueous volumes in a flow channel).

The detector(s) optionally monitors one or a plurality of signals from a single aqueous volume, (e.g., one reaction vesicle or one partitioned aqueous reaction volume). In the case of multiplex analyses, the probes used can each be tagged with a label that demonstrates different excitation/emission spectra. At the detection step, the detector can be electronically instructed to simultaneously gather emission signal data for more than one wavelength, corresponding to the multiple probes. This multiplexing strategy can apply to either nucleic acid or protein detection systems.

In some aspects, the measurement of an optical (i.e., spectrophotometric) emission, such as a fluorescence emission, in the highly partitioned amplification events as described herein requires the development of specific instrumentation as provided by the present invention. Such platforms as described herein can be generally applied to single cell analysis systems that do not require a real time determination of signal, including those systems described herein that utilize solid phase capture and detection. In some aspects, devices of the invention are capable of "real time" analysis (e.g., can measure increases in fluorescence over time).

Example detectors include photo multiplier tubes, spectrophotometers, CCD arrays, scanning detectors, microscopes, galvo-scanns and/or the like. Amplicons or other components which emit a detectable signal can be flowed past the detector, or, alternatively, the detector can move relative to the site of the amplification reaction (or, the detector can simultaneously monitor a number of spatial positions corresponding to channel regions, or microtiter wells e.g., as in a CCD array).

The detector can include or be operably linked to a computer, e.g., which has software for converting detector signal information into assay result information (e.g., presence of a nucleic acid of interest), or the like.

Signals are optionally calibrated, e.g., by calibrating the microfluidic system by monitoring a signal from a known source.

A microfluidic system can also employ multiple different detection systems for monitoring a signal in the system. Detection systems of the present invention are used to detect and monitor the materials in a particular channel region (or other reaction detection region). Once detected, the flow rate and velocity of cells in the channels are also optionally measured and controlled as described above.

Examples of detection systems include optical sensors, temperature sensors, pressure sensors, pH sensors, conductivity sensors, and the like. Each of these types of sensors is readily incorporated into the microfluidic systems described herein. In these systems, such detectors are placed either within or adjacent to the microfluidic device or one or more channels, chambers or conduits of the device, such that the detector is within sensory communication with the device, channel, or chamber. The phrase "within sensory communication" of a particular region or element, as used herein, generally refers to the placement of the detector in a position such that the detector is capable of detecting the property of the microfluidic device, a portion of the microfluidic device, or the contents of a portion of the microfluidic device, for which that detector was intended. For example, a pH sensor placed in sensory communication with a microscale channel is capable of determining the pH of a fluid disposed in that channel. Similarly, a temperature sensor placed in sensory communication with the body of a microfluidic device is capable of determining the temperature of the device itself.

Particularly preferred detection systems include optical detection systems for detecting an optical property of a material within the channels and/or chambers of the microfluidic devices that are incorporated into the microfluidic systems described herein. Such optical detection systems are typically placed adjacent to a microscale channel of a microfluidic device, and are in sensory communication with the channel via an optical detection window that is disposed across the channel or chamber of the device. Optical detection systems include systems that are capable of measuring the light emitted from material within the channel, the transmissivity or absorbance of the material, as well as the materials spectral characteristics. In preferred aspects, the detector measures an amount of light emitted from the material, such as a fluorescent or chemiluminescent material. As such, the detection system will typically include collection optics for gathering a light based signal transmitted through the detection window, and transmitting that signal to an appropriate light detector. Microscope objectives of varying power, field diameter, and focal length are readily utilized as at least a portion of this optical train. The light detectors are optionally spectrophotometers, photodiodes, avalanche photodiodes, photomultiplier tubes, diode arrays, or in some cases, imaging systems, such as charged coupled devices (CCDs) and the like. The detection system is typically coupled to a computer, via an analog to digital or digital to analog converter, for transmitting detected light data to the computer for analysis, storage and data manipulation.

In the case of fluorescent materials such as labeled amplicons, the detector typically includes a light source that produces light at an appropriate wavelength for activating the fluorescent material, as well as optics for directing the light source through the detection window to the material contained in the channel or chamber. The light source can be any number of light sources that provides an appropriate wavelength, including lasers, laser diodes and LEDs. Other light sources are used in other detection systems. For example, broad band light sources are typically used in light scattering/ transmissivity detection schemes, and the like. Typically, light selection parameters are well known to those of skill in the art.

The detector can exist as a separate unit, but can also be integrated with the system or microfluidic device, into a single instrument. Integration of these functions into a single unit facilitates connection of these instruments with the computer, by permitting the use of few or a single communication port(s) for transmitting information between the controller, the detector and the computer.

As used herein, a signal (e.g., an amplified signal) that is detected by a detector is termed a "detection result." Integration with an electronic module permits the displaying of detection results. A detection result can be displayed for one single compartmentalized event (e.g., a partitioned aqueous volume containing one cell, or a reaction vesicle), or the detection result can be displayed for a plurality of events, e.g., a plurality of a partitioned aqueous volumes containing one cell, or a plurality of reaction vesicles each containing one cell. Various software can facilitate in useful comparative presentation of detection results.

### Electronic Module (e.g., a Computer)

Devices of the invention will typically require electronics (for example, an electronics module) to control various functions of the device. For example, a controller system and/or an excitation/detection system will typically be operably coupled to an appropriately programmed electronic module (a processor or computer) which functions to instruct the operation of these instruments in accordance with preprogrammed or user input instructions, receive data and information from these instruments, and interpret, manipulate and report this information to the user. As such, the computer is typically appropriately coupled to one or more component of the device of the invention (e.g., the detector). Analog to digital or digital to analog converters are incorporated as needed. In addition, software for the handling, management and/or display of collected data (for example, data from a detector) can also be integrated into the electronic module.

A detector, e.g., a fluorescence spectrophotometer, is typically operably connected to a computer for controlling the spectrophotometer operational parameters (e.g., wavelength of the excitation and/or wavelength of the detected emission) and/or for storage of data collected from the detector (e.g., fluorescence measurements from individual reaction vesicles or partitioned aqueous reaction volumes in a flow channel). The computer can also be operably connected to the thermal cycling device to control the temperature, timing, and/or rate of temperature change in the system.

The computer typically includes appropriate software for receiving user instructions, either in the form of user input into a set parameter fields, e.g., in a GUI, or in the form of preprogrammed instructions, e.g., preprogrammed for a variety of different specific operations. The software then converts these instructions to appropriate language for instructing the operation of the fluid direction and transport controller to carry out the desired operation. The computer then receives the data from the one or more sensors/detectors included within the system, and interprets the data, either provides it in a user understood format, or uses that data to initiate further controller instructions, in accordance with the programming, e.g., such as in monitoring and control of flow rates (including for continuous flow), temperatures, applied voltages, and the like.

The systems (e.g., devices) of the invention can include instructions (e.g., embodied in a computer or in a computer readable medium, e.g., as system software) for practicing any of the methods described herein. System software can also be included that manipulates, analyzes or displays the collected data from the detector. For example, the system can optionally include system software that converts detector signal information into assay result information, and further, for example, correlates an expression level of a gene in one cell with the expression level of that same gene in a plurality other cells, and displays the date to the user.

The statistical functions noted above can also be incorporated into system software, e.g., embodied in the computer, in computer memory or on computer readable media. For example, the computer can include statistical or probabilistic system software that performs one or more statistical or probabilistic analysis of signals received from one or more of the aliquots subjected to amplification (e.g., via thermocycling). For example, the statistical or probabilistic analysis can include Poisson analysis, Monte Carlo analysis, application of a genetic algorithm, neural network training, Markov modeling, hidden Markov modeling, multidimensional scaling, PLS analysis, and/ or PCA analysis. The statistical or probabilistic analysis software optionally quantitatively determines a concentration, proportion, or number of the nucleic acids of interest in the sample.

In the present invention, the computer typically includes software for the monitoring of materials in the channels. Additionally, the software is optionally used to control electrokinetic or pressure modulated injection or withdrawal of material. The injection or withdrawal is used to modulate the flow rate as described above, to mix components, and the like.

### Thermal Control Elements

The physical components to create the thermally controlled environment (e.g., a thermal control element, or any similar expression) are critical for controlling a PCR reaction as well as other types of biochemical assays. As used herein, a "thermal control element" can refer to more than one discrete article, and more generally refers to an instrumentation system that is involved in regulating temperature. A thermal control system can be dispersed in a device or reside in one discrete location in a device. For example, a thermal control system can include, alternatively or in any combination, a temperature sensor, a heating element, a cooling element, and an electronic module. The electronic module can be operably coupled to the temperature sensor and/or the heating/cooling elements, and the electronic module can store the parameters for thermal cycling programs, and where the electronic module can control the heating or cooling elements, and can store measured temperatures.

Temperature regulation need not be specific for one particular item in the device. All or some subset of aspects of the larger device (e.g., a flow system) can be thermally controlled. For example, a reaction chamber, a holding vessel, or a flow channel can each be collectively thermally controlled, or can be independently controlled. A thermal control element in a larger device can regulate that space where PCR will occur, but that is not the only location in the device where temperature will be regulated. For example, reagent holding reservoirs, motor systems or light sources can be thermally controlled. A thermal control element can be used to maintain something at a constant temperature, or can be used to implement a thermal cycling program that requires multiple temperature settings of various duration (such as typical with PCR) in proper order.

The physical components necessary to create an environment to promote amplification depend on the specific form of amplification. The primary components that impact amplification are the reagents used in the amplification, e.g., enzymes, nucleotides, co-factors, buffers, salts, etc., as well as temperature. In general, nucleic acid amplification schemes fall into two categories, isothermal, wherein the amplification occurs at a single temperature, and thermal cycling, wherein the amplification requires the cycling of the reaction chamber from between two or more different temperatures. In addition, some reactions require or are enhanced by the performance of stepwise changes in temperature during different stages of reaction. Multiple methods are known in the art for providing either a uniform or changing temperature. These methods include various forms of heat transfer from elements which are either hot or cold that either stabilize the temperature within the reaction chamber or drive the chamber to a different reaction temperature. Heat transfer can occur via solid, liquid, gas or optical means. Exemplary instruments include those using basic heating elements and refrigeration systems under thermostat control, combined heating and cooling elements utilizing Peltier principles, systems that utilize heated and cooled air to flow over and/or near the reaction chamber, systems using infrared light for heating, chambers with warmer and cooler liquids, and systems that utilize a combination of one or more of the elements described above.

In some aspects, the measurement of an optical (i.e., spectrophotometric) emission, such as a fluorescence emission, in the highly partitioned amplification events as described herein requires the development of specific instrumentation as provided by the present invention. Such platforms as described herein can be generally applied to single cell analysis systems that do not require a real time determination of signal, including those systems described herein that utilize solid phase capture and detection. In some aspects, these devices are capable of performance of real time analysis (e.g., can measure increases in fluorescence over time).

### USES OF THE INVENTION

The invention finds a wide variety of uses that will be apparent to one of skill in the art upon reading the present disclosure. In various aspects, applications include individual cell determinations of DNA sequence, nucleotide sequence polymorphisms, and variations in RNA abundance (including ribosomal RNA, messenger RNA (mRNA) and micro RNA) that can be characteristic of individual cells. These compositions and methods are also readily adapted to detect polypeptides, quantitate polypeptides, and assess protein modifications and nucleic acid modifications. Additional applications include analysis of specific molecular dynamics and characterization of cellular responses associated with the occurrence of specific cell-cell interactions and studying the detailed response of individual cells to external agents such as small molecules, proteins and other cell-based organisms. These applications include the detection of genetic changes in cancer cells, detection of genotypes in fetal cells derived from maternal blood or amniotic fluid, and monitoring of leukocyte subclasses derived from blood.

The detection of nucleic acids and polypeptides is central to medicine, forensic science, industrial processing, crop and animal breeding and many other fields. The ability to detect disease conditions (e.g., cancer), infectious organisms (e.g., HIV), genetic lineage, genetic markers, and the like, is ubiquitous technology for disease diagnosis and prognosis, marker assisted selection, correct identification of crime scene features, the ability to propagate industrial organisms and many other techniques.

One of the most powerful and basic technologies for nucleic acid detection is nucleic acid amplification. That is, in many typical formats, such as the polymerase chain reaction (PCR), reverse-transcriptase PCR (RT-PCR), ligase chain reaction (LCR), and Q-β replicase and other RNA/transcription mediated techniques (e.g., NASBA), amplification of a nucleic acid of interest precedes detection of the nucleic acid of interest, because it is easier to detect many copies of a nucleic acid than it is to detect a single copy.

Despite the wide-spread use of amplification technologies and the adaptation of these technologies to high throughput systems, certain technical difficulties persist in amplifying and detecting nucleic acids, particularly rare copy nucleic acids and nucleic acids that are only expressed in rare cell types in a larger population of cells. This is particularly true where the amplification reagents amplify high copy nucleic acids in a given sample (e.g., a sample comprising a mixed population of cells) in addition to the rare nucleic acid.

For example, if a set of primers hybridizes to a high copy nucleic acid found in a population of cells, as well as to a low copy nucleic acid, or to nucleic acids expressed only in a small subpopulation of cells, the geometric amplification of the high copy nucleic acid proportionately dominates the amplification reaction and it is difficult or impossible to identify the low copy nucleic acid in any resulting population of amplified nucleic acids. Thus, low copy number alleles of a gene in a sample or cell population can be very difficult to detect, e.g., where a primer set cannot easily be identified that only amplifies the rare nucleic acid (and the practitioner will realize that perfect reagent specificity is rare or non-existent in practice). Amplification of the higher copy number nucleic acids in a sample derived from multiple cells can swamp out any signal from the low copy nucleic acid (or a nucleic acid expressed in only a small number of cells). The ability to identify rare cells that express a particular gene (or protein) of interest can be critical to identifying disease or infection in the early stages, as well as in many other applications.

It is worth noting that these problems have not been addressed by the prior art. While there are reports of single copy amplification of expressed genes, as well as reports of PCR reactions and protein expression analysis on single cells, none of these prior approaches are suitable for truly high throughput automation, as would be required to build a single cell gene or protein expression profiles for a large numbers of individual cells, for example, thousands of cells. The inability to identify rare cell types in a population of cells (based on gene or protein expression data from large numbers of individual cells) hampers the ability to diagnose disease or establish disease prognosis.

The subject invention, which includes methods, compositions and devices, overcomes these difficulties by providing robust high throughput methodologies for identifying and quantifying nucleic acid and protein expression in individual cells. Adaptation of modem high-throughput systems make the methods and devices of the invention possible, i.e., the ability to run massively high numbers of biochemical reactions (e.g., PCR reactions) on individual cells at low cost. Using microfluidic technologies makes it possible to much more exhaustively sample a population of cells for any particular protein or nucleic acid of interest (for example, where that protein or nucleic acid of interest is indicative of a rare cell type or subpopulation of cells). Employment of continuous flow or high throughput stopped flow systems further facilitate the approach. Furthermore, examination of a population of cells by such exhaustive sampling methods provides a great deal of quantitative information (and the concomitant possibility of statistical analysis) with respect to the heterogeneity of a cell population and cell phenotypes (e.g., the expression levels of a given gene or protein in individual cells).

### Biomolecule Targets of Interest

The nucleic acid of interest to be detected in the methods of the invention can be essentially any nucleic acid. It is not intended that the invention be in any way limited in this respect. The sequences for many nucleic acids are available, and furthermore, associations between specific gene expression patterns and disease are also known. No attempt is made to identify the hundreds of thousands of known nucleic acids, any of which can be detected in the methods of the invention. Also, no attempt is made herein to provide an overview of all known gene/disease associations.

Similarly, a polypeptide of interest to be detected in the methods of the invention can be essentially any protein. If necessary, antibody directed to any desired protein can be readily generated using methodologies well known in the art (and commercial services). It is not intended that the invention be in any way limited in this respect. The sequences for many proteins are available, as are known protein/disease associations. No attempt is made to identify the hundreds of thousands of known protein sequences, any of which can be detected in the methods of the invention. Similarly, no attempt is made herein to provide an overview of all known protein/disease associations.

Common sequence repositories for known nucleic acids and proteins include, but are not limited to, GenBank, EMBL, DDBJ and the NCBI. The nucleic acid can be an RNA (e.g., where amplification includes RT-PCR or LCR) or DNA (e.g., where amplification includes PCR or LCR), or an analogue thereof (e.g., for detection of synthetic nucleic acids or analogues thereof). Any variation in a nucleic acid can be detected, e.g., lack of gene expression, overexpression of a gene, deletion of a genomic copy of a gene, a mutation, a single nucleotide polymorphism (SNP), an allele, an isotype, etc. Further, in some aspects, the methods of the invention can be used quantitatively to detect variation in expression levels or gene copy numbers.

In some aspects, the methods of the invention are particularly useful in screening cell populations derived from patients for a protein or nucleic acid of interest, e.g., from bodily fluids, tissue sample and/or waste from the patient. This is because cells derived from relatively large volumes of such materials can be screened in the methods of the invention (removal of such materials is also relatively non-invasive). The cells of interest in such samples can be rare in the population of cells that is collected from the patient (e.g., cancer cells or infective organisms). Identification of expressed genes indicative of cancer equates to the identification of a cancer cell. Stool, sputum, saliva, blood, lymph, tears, sweat, urine, vaginal secretions, ejaculatory fluid, or any tissue of interest, or the like, can easily be screened for rare nucleic acids (and corresponding rare cell types) by the methods of the invention. These cell samples are typically taken, following informed consent, from a patient by standard medical laboratory methods.

### Identification of Cancer Cells

One preferred class of nucleic acids or cells of interest to be detected in the methods of the invention are those involved in cancer. Any nucleic acid that is associated with cancer can be detected in the methods of the invention, e.g., those that encode over expressed or mutated polypeptide growth factors (e.g., sis), overexpressed or mutated growth factor receptors (e.g., erb-B1), over expressed or mutated signal transduction proteins such as G-proteins (e.g., Ras), or non-receptor tyrosine kinases (e.g., abl), or over expressed or mutated regulatory proteins (e.g., myc, myb, jun, fos, etc.) and/or the like. In general, cancer can often be linked to signal transduction molecules and corresponding oncogene products, e.g., nucleic acids encoding Mos, Ras, Raf, and Met; and transcriptional activators and suppressors, e.g., p53, Tat, Fos, Myc, Jun, Myb, Rel, and/or nuclear receptors. p53, colloquially referred to as the "molecular policeman" of the cell, is of particular relevance, as about 50% of all known cancers can be traced to one or more genetic lesion in p53.

Taking one class of genes that are relevant to cancer as an example for discussion, many nuclear hormone receptors have been described in detail and the mechanisms by which these receptors can be modified to confer oncogenic activity have been identified. For example, the physiological and molecular basis of thyroid hormone action is reviewed in Yen (2001) "Physiological and Molecular Basis of Thyroid Hormone Action" Physiological Reviews 81(3):1097-1142, and the references cited therein. Known and well characterized nuclear receptors include those for glucocorticoids (GRs), androgens (ARs), mineralocorticoids (MRs), progestins (PRs), estrogens (ERs), thyroid hormones (TRs), vitamin D (VDRs), retinoids (RARs and RXRs), and the peroxisome proliferator activated receptors (PPARs) that bind eicosanoids. The so called "orphan nuclear receptors" are also part of the nuclear receptor superfamily, and are structurally homologous to classic nuclear receptors, such as steroid and thyroid receptors. Nucleic acids that encode any of these receptors, or oncogenic forms thereof, can be detected in the methods of the invention. About 40% of all pharmaceutical treatments currently available are agonists or antagonists of nuclear receptors and/or oncogneic forms thereof, underscoring the relative importance of these receptors (and their coding nucleic acids) as targets for analysis by the methods of the invention. Following the analysis of a population of cells for the expression (nucleic acid or protein) of one or more hormone receptor, the detection in any one cell of inappropriate expression, overexpression, under expression, or expression of mutated forms can in various contexts indicate the presence of cancer.

As already mentioned, one preferred class of nucleic acid of interest are those that are diagnostic of colon cancer, e.g., in samples derived from stool. Colon cancer is a common disease that can be sporadic or inherited. The molecular basis of various patterns of colon cancer is known in some detail. In general, germline mutations are the basis of inherited colon cancer syndromes, while an accumulation of somatic mutations is the basis of sporadic colon cancer. In Ashkenazi Jews, a mutation that was previously thought to be a polymorphism may cause familial colon cancer. Mutations of at least three different classes of genes have been described in colon cancer etiology: oncogenes, suppressor genes, and mismatch repair genes. One example nucleic acid encodes DCC (deleted in colon cancer), a cell adhesion molecule with homology to fibronectin. An additional form of colon cancer is an autosomal dominant gene, hMSH2, that comprises a lesion. Familial adenomatous polyposis is another form of colon cancer with a lesion in the MCC locus on chromosome #5. For additional details on Colon Cancer, *see,* Calvert et al. (2002) "The Genetics of Colorectal Cancer" Annals of Internal Medicine 137 (7): 603-612 and the references cited therein. For a variety of colon cancers and colon cancer markers that can be detected in stool, *see,* e.g., Boland (2002) "Advances in Colorectal Cancer Screening: Molecular Basis for Stool-Based DNA Tests for Colorectal Cancer: A Primer for Clinicans" Reviews In Gastroenterological Disorders Volume 2, Supp. 1 and the references cited therein. As with other cancers, mutations in a variety of other genes in individual cells that correlate with cancer, such as Ras and p53, are useful diagnostic indicators for cancer.

Cervical cancer is another preferred target for detection, e.g., in samples obtained from vaginal secretions. Cervical cancer can be caused by soemstrains of papova virus (e.g., human papiloma virus; HPV), which has two oncogenes, E6 and E7. E6 binds to and removes p53 and E7 binds to and removes PRB. The loss of p53 and uncontrolled action of E2F/DP growth factors without the regulation of pRB is one mechanism that leads to cervical cancer. Identification of rare cells that express these HPV gene products using methods and/or devices of the invention is a useful diagnostic tool for detecting cancerous or pre-cancerous cells.

Another preferred target for detection by the methods of the invention is retinoblastoma, e.g., in samples derived from tears. Retinoblastoma is a tumor of the eyes which results from inactivation of the pRB gene. It has been found to transmit heritably when a parent has a mutated pRB gene (and, of course, somatic mutation can cause non-heritable forms of the cancer). Identification of cells that do to express or have mutated forms of the Rb gene using methods and/or devices of the invention is a useful diagnostic tool for detecting cancerous or pre-cancerous cells.

Neurofibromatosis Type 1 can be detected in the methods of the invention. The NF1 gene is inactivated, which activates the GTPase activity of the ras oncogene. If NF1 is missing, ras is overactive and causes neural tumors. The methods of the invention can be used to assess NF1 expression pattern in individual cells, and detect rare cells that express Neurofibromatosis Type 1 in CSF or via tissue sampling.

Many other forms of cancer are known and can be found by detecting various types of genetic lesions in cells (e.g., in rare cells) using the methods of the invention. Cancers that can be detected by detecting appropriate lesions include cancers of the lymph, blood, stomach, gut, colon, testicles, pancreas, bladder, cervix, uterus, skin, and essentially all others for which a known genetic lesion exists. For a review of the topic, *see,* The Molecular Basis of Human Cancer Coleman and Tsongalis (Eds) Humana Press; ISBN: 0896036340; 1st edition (August 2001).

### Detection of Infection

Similarly, nucleic acids from pathogenic or infectious organisms can be detected by the methods of the invention, e.g., for infectious fungi, e.g., *Aspergillus,* or *Candida* species; bacteria, particularly *E*. *coli,* which serves a model for pathogenic bacteria (and, of course certain strains of which are pathogenic), as well as medically important bacteria such as *Staphylococci* (e.g., *aureus*), or *Streptococci* (*e.g., pneumoniae*); protozoa such as sporozoa (e.g., *Plasmodia*)*, rhizopods* (e.g., *Entamoeba*) and flagellates (*Trypanosoma, Leishmania, Trichomonas, Giardia,* etc.); viruses such as (+) RNA viruses (examples include Poxviruses e.g., *vaccinia;* Picornaviruses, e.g. *polio;* Togaviruses, e.g., *rubella;* Flaviviruses, e.g., HCV; and Coronaviruses), (-) RNA viruses (e.g., Rhabdoviruses, e.g., VSV; Paramyxovimses, e.g., RSV; Orthomyxovimses, e.g., influenza; Bunyaviruses; and Arenaviruses), dsDNA viruses (Reoviruses, for example), RNA to DNA viruses, i.e., Retroviruses, e.g., HIV and HTLV, and certain DNA to RNA viruses such as Hepatitis B.

### Detection of Industrial Enzymes

A variety of nucleic acids encoding enzymes (e.g., industrial enzymes) can also be detected according to the methods herein, such as amidases, amino acid racemases, acylases, dehalogenases, dioxygenases, diarylpropane peroxidases, epimerases, epoxide hydrolases, esterases, isomerases, kinases, glucose isomerases, glycosidases, glycosyl transferases, haloperoxidases, monooxygenases (e.g., p450s), lipases, lignin peroxidases, nitrile hydratases, nitrilases, proteases, phosphatases, subtilisins, transaminase, and nucleases. Similarly, agriculturally related proteins such as insect resistance proteins (e.g., the Cry proteins), starch and lipid production enzymes, plant and insect toxins, toxin-resistance proteins, Mycotoxin detoxification proteins, plant growth enzymes (e.g., Ribulose 1,5-Bisphosphate Carboxylase/Oxygenase, "RUBISCO"), lipoxygenase (LOX), and Phosphoenolpyruvate (PEP) carboxylase can also be detected.

The examples described above are offered to illustrate, but not to limit the claimed invention. One of skill will recognize a variety of non-critical parameters that can be altered or substituted without departing from the scope of the claimed invention. It is understood that the examples and embodiments described herein are for illustrative purposes only and that various modifications or changes in light thereof will be suggested to persons skilled in the art and are to be included within the spirit and purview of this application and scope of the appended claims. While the foregoing invention has been described in some detail for purposes of clarity and understanding, it will be clear to one skilled in the art from a reading of this disclosure that various changes in form and detail can be made without departing from the true scope of the invention. All publications, patents, patent applications, and/or other documents cited in this application are incorporated by reference in their entirety for all purposes to the same extent as if each individual publication, patent, patent application, and/or other document were individually indicated to be incorporated by reference for all purposes.
The following numbered paragraphs (paras.) contain further statements of various aspects of the present invention:-
1. A device that generates single cell aqueous reaction volumes in a flow channel, the device comprising:
   a) the flow channel;
   b) a first source of an aqueous solution that comprises a plurality of cells, which source is fluidly coupled to the flow channel;
   c) a second source of a partitioning solution that is immiscible in the aqueous solution, which second source is fluidly coupled to the flow channel;
   d) a controller operably coupled to the first and second sources, which controller directs flow of the aqueous solution from the first source into the flow channel, and the partitioning solution from the second source into the flow channel; whereby the partitioning solution partitions cells in the aqueous solution into separate aqueous reaction volumes;
   e) an excitation light source capable of illuminating the aqueous reaction volumes in said flow channel at a desired wavelength;
   f) a spectrophotometric detector capable of detecting a light emission signal from a single aqueous reaction volume in said flow channel; and,
   g) an electronic module operably connected to the detector for collecting and storing said signals detected by the detector from a plurality of aqueous reaction volumes.
2. The device of para 1, the device further comprising a thermal control element operably coupled to the flow channel for regulating the temperature of the aqueous reaction volumes in said flow channel.
3. The device of para 1, the electronic module comprising an algorithm for displaying the plurality of signals collected from the detector.
4. The device of para 1, the device further comprising an optical sensor for detecting cells in the aqueous solution, the sensor operably coupled to the controller.
5. A method for detecting the presence or absence of a biomolecule associated with a plurality of cells, the method comprising:
   a) providing:
      i) said plurality of cells in an aqueous first solution, said first solution further comprising at least one reagent for detecting the presence or absence of the biomolecule, wherein the reagent is capable of generating or participating in generating an amplified signal corresponding to the presence or absence of the biomolecule;
      ii) a second solution that is immiscible with said first solution and capable of forming vesicles when admixed with the first solution;
   b) combining the first solution and the second solution under conditions that result in the formation of a plurality of reaction vesicles, wherein said reaction vesicles thus formed each comprise, (i) one cell from the plurality of cells, and (ii) said reagent for detecting the presence or absence of the biomolecule;
   c) subjecting said reaction vesicles to conditions suitable for detecting the biomolecule, wherein the reagent generates or participates in generating an amplified signal corresponding to the presence or absence of the biomolecule associated with the one cell in each reaction vesicle;
   d) detecting said amplified signal from the plurality of vesicles;
   e) correlating the signal with the presence or absence of the biomolecule associated with said cells, thereby detecting the presence or absence of the biomolecule associated with said cells and generating a detection result; and
   f) displaying the detection result for said plurality of cells.
6. The method of para 5, wherein said plurality of cells comprises at least 100 cells.
7. The method of para 5, wherein said plurality of cells comprises at least 1,000 cells.
8. The method of para 5, wherein said plurality of cells comprises at least 10,000 cells.
9. The method of para 5, wherein said plurality of cells comprises at least 100,000 cells.
10. The method of para 5, wherein said plurality of cells comprises at least 1,000,000 cells.
11. The method of para 5, wherein said second solution is a non-aqueous solution.
12. The method of para 5, wherein said second solution is a silicone oil.
13. The method of para 5, wherein said amplified signal of (a)(i) is a colorimetric signal, a fluorescence signal, a phosphorescence signal, an isotopic signal, a radioisotopic signal, or an enzymatic reaction product.
14. The method of para 5, wherein said detecting step comprises quantitating said amplified signal.
15. The method of para 5, wherein said biomolecule is a genomic nucleic acid of interest, said detecting step comprising amplifying at least one genomic nucleic acid to produce a genomic amplification product, and detecting a signal corresponding to said genomic amplification product.
16. The method of para 5, wherein said biomolecule is a nucleic acid gene expression product of interest, said detecting step comprising, (i) amplifying said gene expression product to produce an amplification product, and (ii) detecting a signal, wherein the signal corresponds to the amplification product.
17. The method of para 15 or 16, wherein said detecting comprises real-time PCR detection.
18. The method of para 16, wherein said amplifying is by RT-PCR.
19. The method of para 16, wherein said at least one reagent is a nucleic acid polymerase comprising a reverse transcriptase activity.
20. The method of para 15 or 16, wherein said amplifying comprises PCR amplifying more than one nucleic acid, thereby generating more than one amplification product, and said PCR is a multiplex PCR.
21. The method of para 15 or 16, wherein said reagent is a plurality of reagents, said plurality comprising at least one primer-pair specific for the nucleic acid of interest, a thermostable DNA-dependent DNA polymerase, free nucleotide triphosphates, and wherein said subjecting said vesicle to conditions suitable for detecting the biomolecule comprises subjecting the vesicle to thermal cycling.
22. The method of para 21, wherein said each member of the primer-pair further comprises a universal priming sequence at the 5' end of the primer, and said reagents further comprise at least one universal primer complimentary to the universal priming sequence.
23. The method of para 21, wherein said reagents further comprise a solid phase component, wherein said solid phase component comprises a nucleic acid molecule at least partially complementary to said amplification product.
24. The method of para 23, wherein said solid phase component is a bead.
25. The method of para 5, wherein said biomolecule is a polypeptide of interest.
26. The method of para 25, wherein said polypeptide of interest is a cell-surface polypeptide.
27. The method of para 25, wherein said reagent is a moiety capable of specific binding to the polypeptide of interest, and wherein said subjecting said vesicles to conditions suitable for detecting the polypeptide of interest comprises subjecting the vesicles to conditions that allow specific interaction of the polypeptide of interest with said moiety.
28. The method of para 27, wherein said moiety is a second polypeptide.
29. The method of para 27, wherein said second polypeptide is an antibody, a fragment of an antibody, or derived from an antibody..
30. The method of para 27, wherein said polypeptide of interest is a cell surface receptor, and said moiety is a ligand for said receptor.
31. The method of para 27, wherein said moiety is attached to a solid phase component.
32. The method of para 31, wherein said solid phase component is a bead.
33. A method for detecting the presence or absence of a biomolecule associated with a plurality of cells, the method comprising:
   a) providing:
      i) said plurality of cells in an aqueous first solution, said first solution further comprising at least one reagent for detecting the presence or absence of the biomolecule, wherein the reagent is capable of generating or participating in generating an amplified signal corresponding to the presence or absence of the biomolecule;
      ii) a second solution that is immiscible with said first solution;
   b) channeling said aqueous solution through a liquid flow system, thereby generating a channeled liquid flow, said liquid flow system further comprising a means for delivering said second solution into the channeled liquid flow at intervals, thereby generating a plurality of partitioned aqueous reaction volumes in the channeled liquid flow, said partitioned aqueous reaction volumes separated from each other by partitions comprising the injected second solution, wherein each partitioned aqueous reaction volume thus formed comprises (i) said at least one cell from the plurality of cells, and (ii) said reagent for detecting the presence or absence of the biomolecule;
   c) subjecting the partitioned aqueous reaction volumes to conditions suitable for detecting the biomolecule, wherein the reagent generates or participates in generating an amplified signal corresponding to the presence or absence of the biomolecule associated with the one cell in each partitioned aqueous reaction volume, thereby generating a signal corresponding to the presence or absence of the biomolecule in the partitioned aqueous reaction volume;
   d) detecting said amplified signal form the plurality of partitioned aqueous reaction vol umes;
   e) correlating the signal with the presence or absence of the biomolecule associated with said cells, thereby detecting the presence or absence of the biomolecule associated with said cells and generating a detection result; and
   f) displaying the detection result for said plurality of cells.
34. The method of para 33, wherein said plurality of cells comprises at least 100 cells.
35. The method of para 33, wherein said plurality of cells comprises at least 1,000 cells.
36. The method of para 33, wherein said plurality of cells comprises at least 10,000 cells.
37. The method of para 33, wherein said plurality of cells comprises at least 100,000 cells.
38. The method of para 33, wherein said plurality of cells comprises at least 1,000,000 cells.
39. The method of para 33, wherein said second solution is a non-aqueous solution.
40. The method of para 33, wherein said second solution is a silicone oil.
41. The method of para 33, wherein said amplified signal of (a)(i) is a colorimetric signal, a fluorescence signal, a phosphorescence signal, an isotopic signal, a radioisotopic signal, or an enzymatic reaction product.
42. The method of para 33, wherein said detecting step comprises quantitating said amplified signal.
43. The method of para 33, wherein said biomolecule is a genomic nucleic acid of interest, said detecting step comprising amplifying at least one genomic nucleic acid to produce a genomic amplification product, and detecting a signal corresponding to said genomic amplification product.
44. The method of para 33, wherein said biomolecule is a nucleic acid gene expression product of interest, said detecting step comprising, (i) amplifying said gene expression product to produce an amplification product, and (ii) detecting a signal, wherein the signal corresponds to the amplification product.
45. The method of para 43 or 44, wherein the detecting comprises real-time PCR detection.
46. The method of para 44, wherein said amplifying is by RT-PCR.
47. The method of para 44, wherein said at least one reagent is a nucleic acid polymerase comprising a reverse transcriptase activity.
48. The method of para 43 or 44, wherein said amplifying comprises PCR amplifying more than one nucleic acid, thereby generating more than one amplification product, and said PCR is a multiplex PCR.
49. The method of para 43 or 44, wherein said reagent is a plurality of reagents, said plurality comprising at least one primer-pair specific for the nucleic acid of interest, a thermostable DNA-dependent DNA polymerase, free nucleotide triphosphates, and wherein said subjecting said vesicle to conditions suitable for detecting the biomolecule comprises subjecting the vesicle to thermal cycling.
50. The method of para 49, wherein said each member of the primer-pair further comprises a universal priming sequence at the 5' end of the primer, and said reagents further comprise at least one universal primer complimentary to the universal priming sequence.
51. The method of para 49, wherein said reagents further comprise a solid phase component, wherein said solid phase component comprises a nucleic acid molecule at least partially complementary to said amplification product.
52. The method of para 51, wherein said solid phase component is a bead.
53. The method of para 33, wherein said biomolecule is a polypeptide of interest.
54. The method of para 53, wherein said polypeptide of interest is a cell-surface polypeptide.
55. The method of para 53, wherein said reagent is a moiety capable of specific binding to the polypeptide of interest, and wherein said subjecting said partitioned aqueous reaction volumes to conditions suitable for detecting the polypeptide of interest comprises subjecting the partitioned aqueous reaction volumes to conditions that allow specific interaction of the polypeptide of interest with said moiety.
56. The method of para 55, wherein said moiety is a second polypeptide.
57. The method of para 55, wherein said second polypeptide is an antibody, a fragment of an antibody, or derived from an antibody.
58. The method of para 55, wherein said polypeptide of interest is a cell surface receptor, and said moiety is a ligand for said receptor.
59. The method of para 55, wherein said moiety is attached to a solid phase component.
60. The method of para 59, wherein said solid phase component is a bead.
61. A composition comprising:
   a) a plurality of aqueous reaction core solutions, each comprising:
      (i) a cell, and
      (ii) at least one reagent for detecting the presence or absence of a biomolecule associated with said cell, wherein the reagent is capable of generating or participating in generating an amplified signal corresponding to the presence or absence of the biomolecule; and
   b) an immiscible liquid shell that partitions the aqueous reaction core solution.
62. The composition of para 61, wherein said shell surrounds the reaction core solutions, thereby providing a plurality of vesicles.
63. The composition of para 61, wherein said shell partitions one aqueous core solution from an adjacent aqueous core solution, thereby partitioning two or more aqueous cores within the partitioned fluid.
64. The composition of para 61, wherein said partitioning solution is a silicon oil.
65. The composition of para 61, wherein said biomolecule is a genomic nucleic acid or a gene expression product.
66. The composition of para 65, wherein said at least one reagent is a plurality of reagents, said plurality comprising at least one primer-pair specific for the genomic nucleic acid or the gene expression product, , a thermostable DNA-dependent DNA polymerase, and free nucleotide triphosphates.
67. The composition of para 65, wherein said at least one reagent is a nucleic acid polymerase comprising a reverse transcriptase activity.
68. The composition of para 66, wherein said each member of the primer-pair further comprises a universal priming sequence at the 5' end of the primer, and said reagents further comprise at least one universal primer complimentary to the universal priming sequence.
69. The composition of para 61, wherein said at least one reagent comprises a solid phase component.
70. The composition of para 69, wherein said solid phase component is selected from particles, beads, strands, precipitates, gels, sol-gels, sheets, tubing, spheres, containers, channels, capillaries, pads, slices, films, plates, dipsticks and slides.
71. The composition of para 69, wherein said solid phase component is a bead.
72. The composition of para 61, wherein said biomolecule is a polypeptide of interest.
73. The composition of para 72, wherein said polypeptide of interest is a cell-surface polypeptide.
74. The composition of para 72, wherein said at least one reagent comprises a moiety capable of specific binding to the polypeptide of interest.
75. The composition of para 74, wherein said moiety is a second polypeptide.
76. The composition of para 74, wherein said second polypeptide is an antibody, a fragment of an antibody, or derived from an antibody..
77. The composition of para 74, wherein said polypeptide of interest is a cell surface receptor, and said moiety is a ligand for said receptor.
78. The composition of para 74, wherein said moiety is attached to a solid phase component.
79. The composition of para 78, wherein said solid phase component is a bead.

## Claims

1. A device that generates single cell aqueous reaction volumes in a flow channel, the device comprising:
a) the flow channel;
b) a first source of an aqueous solution that comprises a plurality of cells, which source is fluidly coupled to the flow channel;
c) a second source of a partitioning solution that is immiscible in the aqueous solution, which second source is fluidly coupled to the flow channel;
d) a controller operably coupled to the first and second sources, which controller directs flow of the aqueous solution from the first source into the flow channel, and the partitioning solution from the second source into the flow channel; whereby the partitioning solution partitions cells in the aqueous solution into separate aqueous reaction volumes;
e) an excitation light source capable of illuminating the aqueous reaction volumes in said flow channel at a desired wavelength;
f) a spectrophotometric detector capable of detecting a light emission signal from a single aqueous reaction volume in said flow channel; and,
g) an electronic module operably connected to the detector for collecting and storing said signals detected by the detector from a plurality of aqueous reaction volumes.

2. The device of claim 1, the device further comprising a thermal control element operably coupled to the flow channel for regulating the temperature of the aqueous reaction volumes in said flow channel.

3. The device of claim 1, the electronic module comprising an algorithm for displaying the plurality of signals collected from the detector.

4. The device of claim 1, the device further comprising an optical sensor for detecting cells in the aqueous solution, the sensor operably coupled to the controller.

5. A method for detecting the presence or absence of a biomolecule associated with a plurality of cells, the method comprising:
a) providing:
i) said plurality of cells in an aqueous first solution, said first solution further comprising at least one reagent for detecting the presence or absence of the biomolecule, wherein the reagent is capable of generating or participating in generating an amplified signal corresponding to the presence or absence of the biomolecule;
ii) a second solution that is immiscible with said first solution;
b) producing a plurality of reaction vesicles or a plurality of partitioned aqueous reaction volumes, wherein said reaction vesicles or partitioned aqueous reaction volumes each comprise, (i) one cell from the plurality of cells, and (ii) said reagent for detecting the presence or absence of the biomolecule;
c) subjecting said reaction vesicles or partitioned aqueous reaction volumes to conditions suitable for detecting the biomolecule, wherein the reagent generates or participates in generating an amplified signal corresponding to the presence or absence of the biomolecule associated with the one cell in each reaction vesicle or partitioned aqueous reaction volume;
d) detecting said amplified signal from the plurality of vesicles or partitioned aqueous reaction volumes;
e) correlating the signal with the presence or absence of the biomolecule associated with said cells, thereby detecting the presence or absence of the biomolecule associated with said cells and generating a detection result; and
f) displaying the detection result for said plurality of cells.

6. The method of claim 5, wherein producing the plurality of aqueous reaction volumes comprises channeling said aqueous solution through a liquid flow system, thereby generating a channeled liquid flow, said liquid flow system further comprising a means for delivering said second solution into the channeled liquid flow at intervals, thereby generating a plurality of partitioned aqueous reaction volumes in the channeled liquid flow, said partitioned aqueous reaction volumes separated from each other by partitions comprising the second solution.

7. The method of claim 5, wherein producing the plurality of reaction vesicles comprises combining the first solution and the second solution.

8. The method of claim 5, wherein said plurality of cells comprises at least 100, at least 1,000, at least 10,000, at least 100,000, or at least 1,000,000 cells.

9. The method of claim 5, wherein said second solution is a non-aqueous solution or silicone oil.

10. The method of claim 5, wherein said amplified signal of (a)(i) is a colorimetric signal, a fluorescence signal, a phosphorescence signal, an isotopic signal, a radioisotopic signal, or an enzymatic reaction product.

11. The method of claim 5, wherein said biomolecule is a genomic nucleic acid of interest or a nucleic acid gene expression product, said detecting step comprising amplifying said genomic nucleic acid of interest or said nucleic acid gene expression product to produce an amplification product, and detecting a signal corresponding to said amplification product.

12. The method of claim 11, wherein:
(i) said detecting comprises real-time PCR detection; or
(ii) said amplifying is by RT-PCR; or
(iii) said at least one reagent is a nucleic acid polymerase comprising a reverse transcriptase activity; or
(iv) said amplifying comprises PCR amplifying more than one nucleic acid, thereby generating more than one amplification product, and said PCR is a multiplex PCR; or
(v) said reagent is a plurality of reagents, said plurality comprising at least one primer-pair specific for the nucleic acid of interest, a thermostable DNA-dependent DNA polymerase, free nucleotide triphosphates, and wherein said subjecting said vesicles or said partitioned aqueous reaction volumes to conditions suitable for detecting the biomolecule comprises subjecting the vesicles or the partitioned aqueous reaction volumes to thermal cycling.

13. The method of claim 12(v), wherein said each member of the primer-pair further comprises a universal priming sequence at the 5' end of the primer, and said reagents further comprise at least one universal primer complimentary to the universal priming sequence.

14. The method of claim 12(v), wherein said reagents further comprise a solid phase component or a bead, wherein said solid phase component or bead comprises a nucleic acid molecule at least partially complementary to said amplification product.

15. The method of claim 5, wherein said biomolecule is a polypeptide, a cell surface polypeptide, or an antibody.
